# EUROPEAN PATENT APPLICATION

(11) **EP 3 919 483 A1**
(43) Date of publication of application: **08.12.2021**
(21) Application number: 20748476.7
(22) Date of filing: 19.01.2020
(51) Int. Cl.: C07D 401/04, A61K 31/497, A61P 35/00

(54) **BENZOPYRIDONE HETEROCYCLIC COMPOUND AND USE THEREOF**

(30) Priority: 29.01.2019 CN 201910087070
(71) Applicant: Brightgene Bio-medical Technology Co., Ltd., Suzhou, Jiangsu 215123 (CN)
(72) Inventor: FANG, Huaxiang, Wuhan Hubei 430073 (CN); LI, Fangfang, Wuhan Hubei 430073 (CN); XU, Yong, Wuhan Hubei 430073 (CN); HUANG, Lu, Wuhan Hubei 430073 (CN); TAO, Jinfeng, Wuhan Hubei 430073 (CN)
(74) Representative: Grünecker Patent- und Rechtsanwälte PartG mbB
(86) International application number: PCT/CN2020/073028
(87) International publication number: WO 2020/156285

(57) **Abstract**

A compound represented by formula I, or a pharmaceutically acceptable salt thereof, or a stereoisomer thereof, or a tautomer thereof, or a hydrate thereof, or a solvate thereof, or a metabolite thereof, or a prodrug thereof. R₁-R₅ and group A are as defined in the description. The compound is used for the preparation of drugs for treating diseases caused by KRAS G12C mutation and for treating and/or preventing cancers.

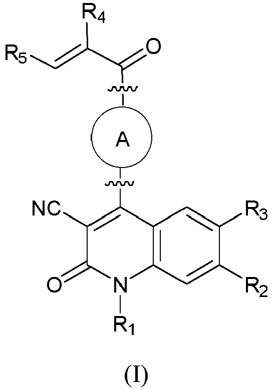

## Description

### TECHNICAL FIELD

The present disclosure belongs to the technical field of biology and medicine, and relates to a benzopyridone heterocyclic compound and the use thereof.

### BACKGROUND

In the field of cancer research, KRAS is one of the most well-known oncogenes. Mutations in the oncogene RAS frequently occur in human tumors, accounting for about one-third of all mutations in human malignant tumors. The RAS family includes HRAS, NRAS and KRAS. Mutations in KRAS, the main subtype of the RAS protein family, account for 86% of all RAS protein mutations, and are more common in pancreatic cancer, colorectal cancer, and lung cancer. In respect of non-small cell lung cancer (NSCLC), 15% to 30% of the patients carry KRAS gene mutations (among these NSCLC patients, patients suffering from lung adenocarcinoma accounts for 30% to 50%), and such proportion is higher than the percentage of those having mutations in EGFR, ALK, or the like. In colon cancer patients, the probability of abnormal KRAS gene mutations is 30% to 35%. And in respect of pancreatic cancer, KRAS gene mutations are present in more than 90% of the patients. The KRAS signaling pathway is an important anti-tumor pathway, and targeting KRAS signaling is becoming an important field for the discovery of anti-tumor drugs. However, due to the lack of good small molecule-binding cavities on the surface of KRAS protein, the development of KRAS-based small molecule inhibitors has always been one of the difficulties in the medical field. Currently, no KRAS inhibitor has been launched in the market in the world. Therefore, development of novel small molecule inhibitors of KRAS has huge clinical value and broad market prospects.

### SUMMARY

The technical problem to be solved by the present disclosure is to provide a benzopyridone heterocyclic compound with a novel structure and the use thereof. The compound according to the present disclosure has KRAS G12C inhibitory activity and provides a new commercial choice of KRAS G12C inhibitors.

The present disclosure solves the technical problem by the following technical solutions. According to the first aspect of the present disclosure, the present disclosure provides a compound represented by formula I, or a pharmaceutically acceptable salt thereof, or a stereoisomer thereof, or a tautomer thereof, or a hydrate thereof, or a solvate thereof, or a metabolite thereof, or a prodrug thereof, 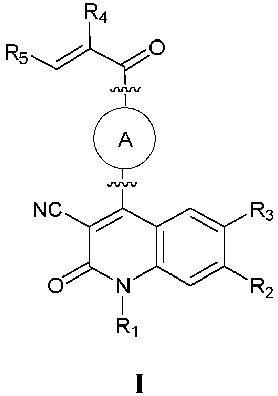 wherein,
R₁ is independently selected from an aryl optionally substituted with a plurality of R₆, or a heteroaryl optionally substituted with a plurality of R₆; and when R₁ is substituted with a plurality of R₆, each R₆ may be the same as or different from each other;
R₂ is independently selected from an aryl optionally substituted with a plurality of R₇, or a heteroaryl optionally substituted with a plurality of R₇; and when R₂ is substituted with a plurality of R₇, each R₇ may be the same as or different from each other;
R₃ is selected from H, halogen, cyano, amide group, hydroxy, amino, C₁-C₃ alkyl, C₁-C₃ heteroalkyl, C₁-C₃ haloalkyl, or C₃-C₈ heterocycloalkyl; said C₁-C₃ alkyl, C₁-C₃ heteroalkyl, C₁-C₃ haloalkyl, or C₃-C₈ heterocycloalkyl is optionally substituted with 0 to 3 R₇, and when said C₁-C₃ alkyl, C₁-C₃ heteroalkyl, C₁-C₃ haloalkyl, or C₃-C₈ heterocycloalkyl is substituted with a plurality of R₇, each R₇ may be the same as or different from each other;
R₄ and R₅ are each independently selected from H, halogen, C₁-C₈ alkyl optionally substituted with 0 to 3 R₇, or C₁-C₈ heteroalkyl optionally substituted with 0 to 3 R₇; 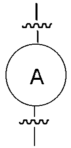 is selected from C₄-C₈ monoheterocycloalkyl optionally substituted with 0 to 3 R₈, C₆-C₁₂ bridged heterocycloalkyl optionally substituted with 0 to 3 R₈, or C₆-C₁₂ spiroheterocycloalkyl optionally substituted with 0 to 3 R₈;
R₆ is selected from halogen, OH, CN, NH₂, C₁-C₈ alkyl, C₁-C₈ heteroalkyl, C₁-C₃ haloalkyl, C₁-C₃ haloalkoxy, C₃-C₈ cycloalkyl, or C₃-C₈ heterocycloalkyl; wherein C₁-C₈ alkyl, C₁-C₈ heteroalkyl, C₁-C₃ haloalkyl, C₁-C₃ haloalkoxy, C₃-C₈ cycloalkyl, or C₃-C₈ heterocycloalkyl may be substituted with a plurality of the following groups: F, Cl, Br, I, OH, CN, NH₂, CH₃, CH₃CH₂, CH₃O, CF₃, CHF₂, CH₂F, cyclopropyl, isopropyl, N(CH₃)₂, and NH(CH₃)₂;
R₇ is selected from halogen, OH, CONH₂, CN, NH₂, C₁-C₈ alkyl, C₁-C₈ heteroalkyl, C₁-C₃ haloalkyl, C₁-C₃ haloalkoxy, C₃-C₈ cycloalkyl, or C₃-C₈ heterocycloalkyl, wherein C₁-C₈ alkyl, C₁-C₈ heteroalkyl, C₁-C₃ haloalkyl, C₁-C₃ haloalkoxy, C₃-C₈ cycloalkyl, or C₃-C₈ heterocycloalkyl may be substituted with a plurality of the following groups: F, Cl, Br, I, OH, CN, NH₂, CH₃, CH₃CH₂, CH₃O, CF₃, CHF₂, CH₂F, cyclopropyl, isopropyl, N(CH₃)₂, and NH(CH₃)₂; and
R₈ is selected from H, halogen, CN, OH, C₁-C₃ alkyl, halogen-substituted C₁-C₃ alkyl, or cyano-substituted C₁-C₃ alkyl.

According to the Examples in the present disclosure, it is preferred that said R₁ and R₂ are each independently selected from cyclopropyl, cyclobutyl, cyclopentyl, cyclohexyl, pyrrolyl, piperazinyl, piperidinyl, phenyl, pyridyl, pyrimidinyl, pyrazolyl, thiazolyl, oxazolyl, imidazolyl, or indazolyl.

According to the Examples in the present disclosure, it is preferred that said R₃ is selected from hydrogen, chlorine, fluorine, amino, cyano, hydroxy, isopropyl, cyclopropyl, methyl, difluoromethyl, trifluoromethyl, methoxy, trifluoromethoxy, -OCH₂CH₃, -OCH₂CHF₂, or -OCH₂CF₃.

According to the Examples in the present disclosure, it is preferred that said R₄ and R₅ are each independently selected from hydrogen, chlorine, fluorine, methyl, or -CH₂N(CH₃)₂.

According to the Examples in the present disclosure, it is preferred that said R₆ is selected from hydrogen, chlorine, fluorine, bromine, amino, cyano, hydroxy, methyl, ethylmethyl, isopropyl, cyclopropyl, difluoromethyl, trifluoromethyl, methoxy, trifluoromethoxy, -OCH₂CH₃, -OCH₂CHF₂, and -OCH₂CF₃.

According to the Examples in the present disclosure, it is preferred that said R₇ is selected from hydrogen, chlorine, fluorine, bromine, amino, carboxamido, cyano, hydroxy, methyl, ethylmethyl, isopropyl, cyclopropyl, difluoromethyl, trifluoromethyl, methoxy, trifluoromethoxy, -OCH₂CH₃, -OCH₂CHF₂, and -OCH₂CF₃.

According to the Examples in the present disclosure, it is preferred that said R₈ is selected from hydrogen, methyl, -CH₂OH or -CH₂CN.

According to the Examples in the present disclosure, it is preferred that said 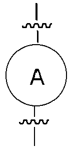 is selected from

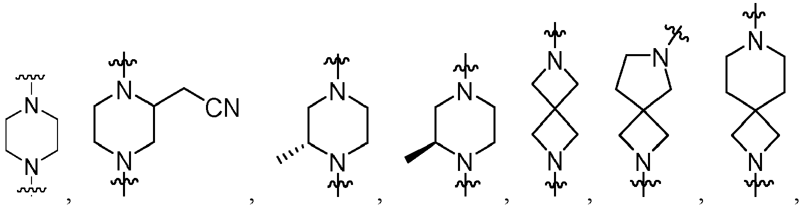

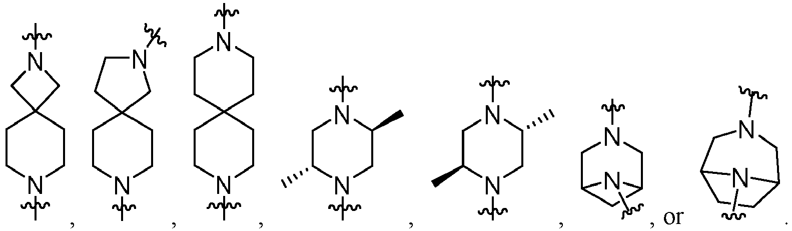

In the present disclosure, "hetero" as mentioned in "C₁-C₈ heteroalkyl", "C₃-C₈ heterocycloalkyl", "heteroaryl", "monoheterocycloalkyl", "C₆-C₁₂ bridged heterocycloalkyl", and "C₆-C₁₂ spiroheterocycloalkyl" means heteroatoms or heteroatom radicals, each of which are independently selected from -O-, -S-, -CN, -NH-, =O, -O-N=, -C(=O)O-, -C(=O)-, -S(=O)-, -S(=O)₂-, -C(=O)NH-, -S(=O)₂NH-, or -NHC(=O)NH-; and in any one of the above cases, the number of the heteroatoms or heteroatom radicals is independently selected from 1, 2 or 3.

In some embodiments of the present disclosure, the compound, or a pharmaceutically acceptable salt thereof, or a stereoisomer thereof, or a tautomer thereof, or a hydrate thereof, or a solvate thereof, or a metabolite thereof, or a prodrug thereof is selected from 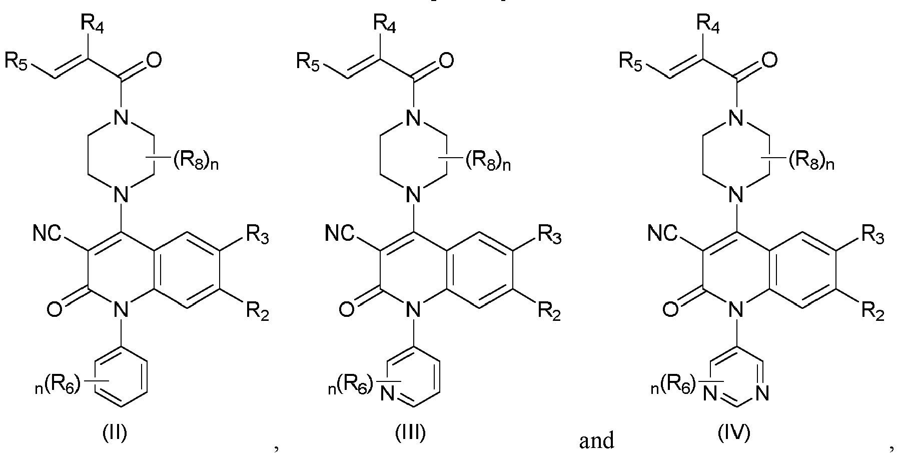 wherein R₂, R₃, R₄, R₅, R₆, R₇ and R₈ are as defined in claim 1 or 2, and n is 0, 1, 2, 3 or 4.

In some embodiments of the present disclosure, it is more preferred that the compound, or a pharmaceutically acceptable salt thereof, or a stereoisomer thereof, or a tautomer thereof, or a hydrate thereof, or a solvate thereof, or a metabolite thereof, or a prodrug thereof is selected from 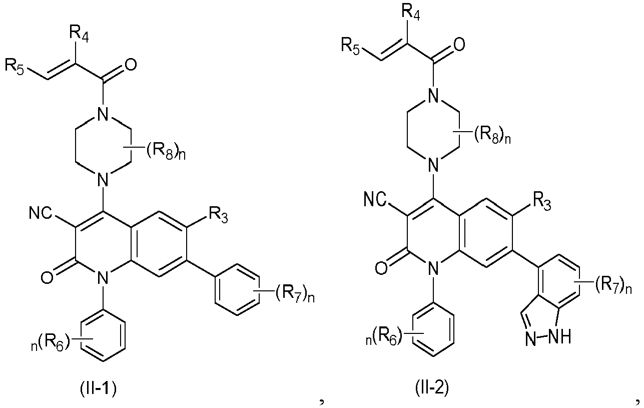 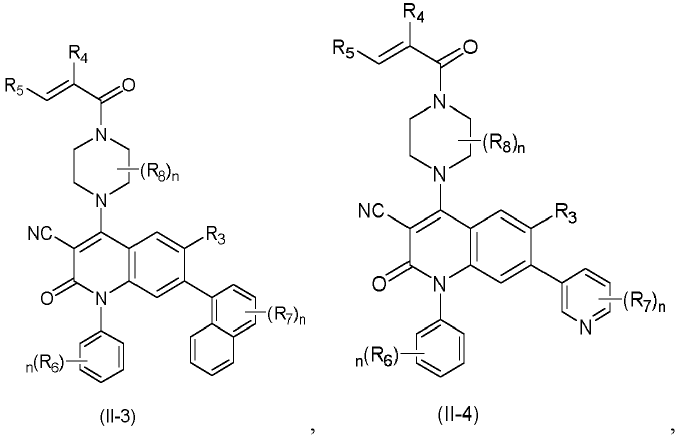 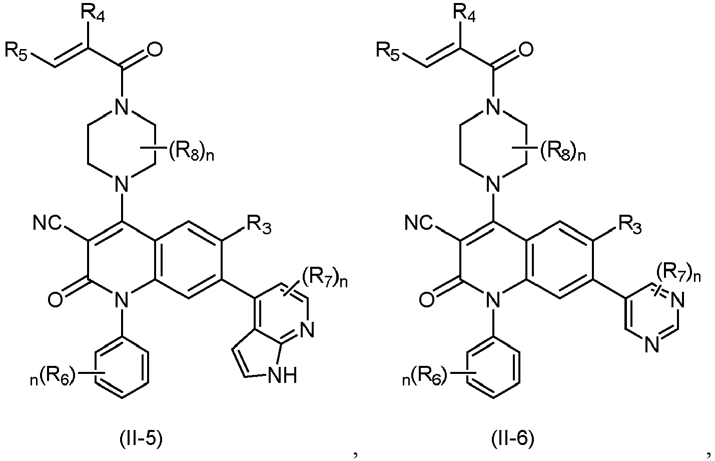 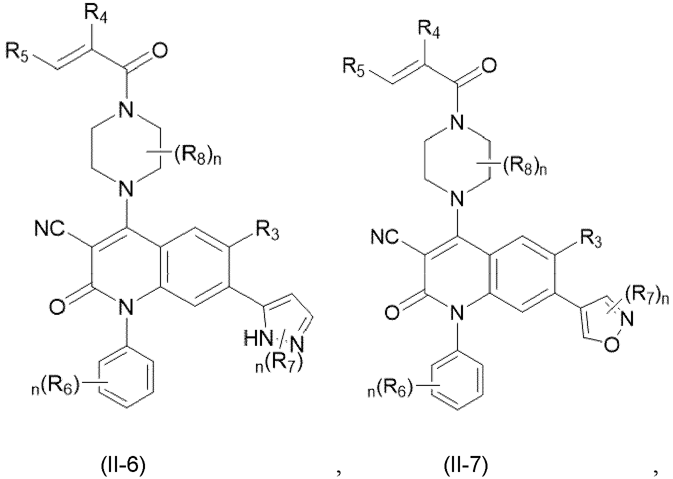 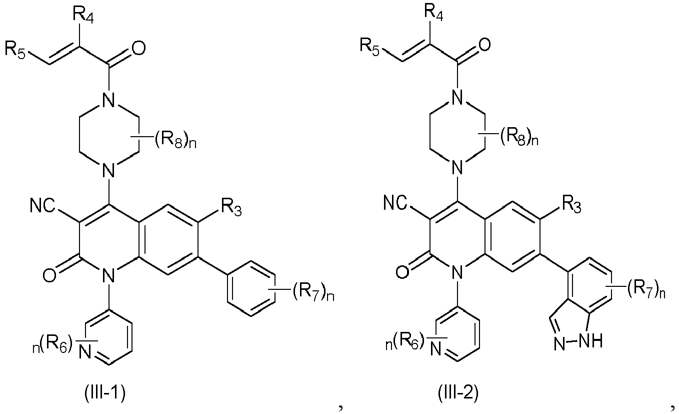 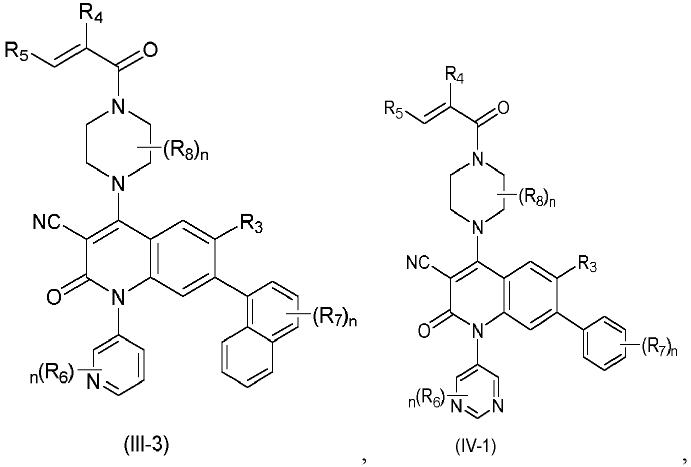 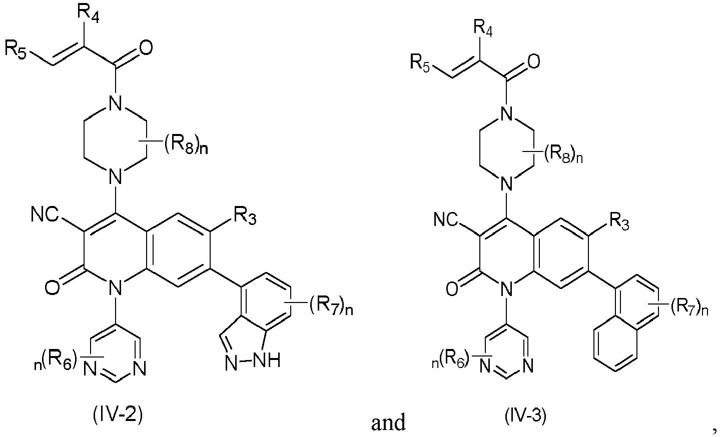 wherein,
R₃ is selected from H, F, Cl, OH, CF₃, CH₃, cyclopropyl, OCF₃, CHF₂ or OCH₃;
R₄ and R₅ are independently selected from H, F or CH₃;
R₆ is selected from H, F, Cl, Br, methyl, ethyl, isopropyl, methoxy, cyclopropyl or -CH₂N(CH₃)₂;
R₇ is selected from H, F, Cl, Br, NH₂, OH, OCH₃, CN, CF₃, CONH₂, methyl, ethyl, isopropyl, cyclopropyl or CHF₂;
R₈ is selected from H or methyl; and
n is 0, 1, 2, 3 or 4.

In the present disclosure, those skilled in the art can select the groups described in the general formula of the compound and the substituents thereof to provide stable compounds, or pharmaceutically acceptable salts thereof, or stereoisomers thereof, or tautomers thereof, or hydrates thereof, or solvates thereof, or metabolites thereof, or prodrugs thereof, including but not limited to the compounds described in the Examples of the present disclosure.

In some embodiments of the present disclosure, the present disclosure provides use of the above-mentioned compounds, or pharmaceutically acceptable salts thereof, or stereoisomers thereof, or tautomers thereof, or hydrates thereof, or solvates thereof, or metabolites thereof, or prodrugs thereof, or pharmaceutical compositions thereof in the preparation of drugs for treating and/or preventing cancer. The compounds described in the present disclosure can be used to treat and/or prevent cancers, wherein the cancers that can be treated and/or prevented include, but are not limited to, pancreatic cancer, colorectal cancer, and lung cancer.

In the present disclosure, there are also some embodiments that are derived from arbitrary combination of the above variables.

### Technical effects

In the present disclosure, a series of new compounds are synthesized; and related enzyme activity assays and cell viability assays show that the compounds according to the present disclosure have excellent efficacy on cells, and the IC₅₀ values thereof on *in-vitro* cell proliferation reach a scale of nM. The compounds can be well applied in the treatment of a variety of tumors. The compounds of the present disclosure have excellent inhibitory effect on both human non-small cell lung cancer cells NCI-H358 and Mia PaCa2 cells with KRAS G12C mutation, and have good selectivity. The compounds of the present disclosure show excellent anti-tumor activity in *in-vivo* efficacy experiments in animals. The compounds according to the present disclosure can be used as drugs to be prepared into KRAS G12C inhibitors for preventing and/or treating diseases caused by KRAS G12C mutation, and can be used to prepare drugs for treating and/or preventing cancers, wherein the cancers to be treated and/or prevented include, but are not limited to, pancreatic cancer, colorectal cancer, and lung cancer.

### Terms and Definition

Unless stated to the contrary, the following terms used in the specification and claims have the following meanings.

"Alkyl" refers to a saturated aliphatic hydrocarbyl, including linear and branched groups having 1 to 20 carbon atoms, such as linear and branched groups having 1 to 18 carbon atoms, 1 to 12 carbon atoms, 1 to 8 carbon atoms, 1 to 6 carbon atoms, or 1 to 4 carbon atoms. In the present disclosure, "alkyl" may be a monovalent, divalent, or trivalent group. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, and various branched isomers thereof, etc. Non-limiting examples also include methylene, methylidyne, ethylidene, ethylidyne, propylidene, propylidyne, butylidene, butylidyne, and various branched isomers thereof. Alkyl may be optionally substituted or unsubstituted.

"Cycloalkyl" refers to a monocyclic or polycyclic hydrocarbyl substituent which is saturated or partially unsaturated. "Cycloalkyl" comprises 3 to 12 ring atoms, which may be, for example, 3 to 12, 3 to 10, or 3 to 6 ring atoms, or may be a 3-membered ring, a 4-membered ring, a 5-membered ring, or a 6-membered ring. Non-limiting examples of monocyclic groups include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc. The cyclic group may be optionally substituted or unsubstituted.

"Heterocycloalkyl" refers to a monocyclic or polycyclic hydrocarbyl substituent which is saturated or partially unsaturated. "Heterocycloalkyl" comprises 3 to 20 ring atoms, which may be, for example, 3 to 16, 3 to 12, 3 to 10, or 3 to 6 ring atoms, wherein one or more ring atoms are selected from heteroatoms such as nitrogen, oxygen, and S(O)ₘ (where m is 0, 1, or 2), but a cyclic moiety of -O-O-, -O-S-, or -S-S- is not included, and the remaining ring atoms are carbon atoms. "Heterocycloalkyl" preferably comprises 3 to 12 ring atoms, of which 1 to 4 ring atoms are heteroatoms; more preferably, the heterocycloalkyl ring comprises 3 to 10 ring atoms; and most preferably, the heterocycloalkyl ring is a 5-membered ring or a 6-membered ring, of which 1 to 4 ring atoms are heteroatoms, more preferably 1 to 3 ring atoms are heteroatoms, and most preferably 1 to 2 ring atoms are heteroatoms. Non-limiting examples of monocyclic heterocyclyls include pyrrolidinyl, piperidinyl, piperazinyl, morpholinyl, thiomorpholinyl, homopiperazinyl, and the like. Polycyclic heterocyclyls include spiroheterocyclyls, fused heterocyclyls, or bridged heterocyclyls.

"Spiroheterocyclyl" refers to a 5- to 18-membered polycyclic group which has two or more cyclic structures with monocyclic rings sharing one atom with each other, wherein the ring(s) contain(s) one or more double bonds, but no ring has an aromatic system with fully conjugated electrons; and wherein one or more ring atoms are selected from heteroatoms such as nitrogen, oxygen, and S(O)ₚ (where p is selected from 0, 1, or 2), and the remaining ring atoms are carbon atoms. It is preferably a 6- to 14-membered polycyclic group, and more preferably a 7- to 10-membered polycyclic group. According to the number of spiro atoms shared among the rings, spiroheterocyclyls are classified into monospiroheterocyclyls, bispiroheterocyclyls or polyspiroheterocyclyls, and a monospiroheterocyclyl or a bispiroheterocyclyl is preferred. More preferably, the spiroheterocyclyl is a 3-membered/6-membered monospiroheterocyclyl, a 4-membered/4-membered monospiroheterocyclyl, a 4-membered/5-membered monospiroheterocyclyl, a 4-membered/6-membered monospiroheterocyclyl, a 5-membered/5-membered monospiroheterocyclyl, or a 5-membered/6-membered monospiroheterocyclyl, wherein "a-membered/b-membered monospiroheterocyclyl" refers to a spiroheterocyclyl in which an a-membered monocyclic ring and a b-membered monocyclic ring share one atom with each other. Non-limiting examples of "spiroheterocyclyl" include but are not limited to diazaspiro[3.3]heptane.

"Bridged heterocyclyl" refers to a 5- to 14-membered or 5- to 18-membered polycyclic group containing two or more cyclic structures that share two atoms not directly attached to each other, wherein one or more rings may contain one or more double bonds, but no ring has an aromatic system with fully conjugated π electrons; and wherein one or more ring atoms are selected from heteroatoms such as nitrogen, oxygen or sulfur, and the remaining ring atoms are carbon atoms. It is preferably a 6- to 14-membered polycyclic group, and more preferably a 7-to 10-membered polycyclic group. According to the number of constituent rings, bridged heterocyclyls can be classified into bicyclic, tricyclic, tetracyclic or polycyclic bridged heterocyclyls. A bicyclic, tricyclic, or tetracyclic bridged heterocyclyl is preferred, and a bicyclic or tricyclic bridged heterocyclyl is more preferred. Non-limiting examples of "fused heterocyclyl" include but are not limited to diazabicyclo[3.1.1]heptane.

"Haloalkyl" or "haloalkoxy" means an alkyl or alkoxy group substituted with one or more halogen atoms which are the same or different. Examples of preferred alkyl or alkoxy include but are not limited to trifluoromethyl, trifluoroethyl and trifluoromethoxy.

"Aryl" means a monocyclic, bicyclic, or tricyclic carbocyclic ring system containing 6 to 14 ring atoms, wherein at least one ring system is aromatic, each ring system contains a ring composed of 3 to 7 atoms, and there is one or more connection points connected to the remaining part of the molecule. Examples include but are not limited to phenyl, naphthyl, anthracene and the like. Preferably, said aryl is a carbocyclic ring system having 6 to 10 or 6 to 7 ring atoms.

"Heteroaryl" means a monocyclic, bicyclic, or tricyclic system containing 5 to 14 ring atoms, wherein at least one ring system is aromatic, at least one ring system contains one or more heteroatoms selected from nitrogen, oxygen and sulfur, each ring system contains a ring composed of 5 to 7 atoms, and there are one or more connection points connected to the remaining part of the molecule. The term "heteroaryl" may be used interchangeably with the term "heteroaromatic ring" or the term "heteroaromatic compound". Examples include but are not limited to furanyl, imidazolyl, 2-pyridinyl, 3-pyridinyl, thiazolyl, purinyl, and quinolyl. Preferably, said heteroaryl is a ring system having 5 to 10 ring atoms.

"Halogen" refers to fluorine, chlorine, bromine, and iodine, and preferably fluorine, chlorine and bromine.

"Optional" or "optionally" means that the event or environment described subsequently may, but not necessarily, occur and such description includes the occasion where the event or environment occurs or does not occur. For example, "a heterocyclic group optionally substituted with alkyl" means that alkyl may but not have to be present, and such description includes the case where the heterocyclic group is substituted with alkyl and the case where the heterocyclic group is not substituted with alkyl.

"Substituted" means that one or more, preferably up to 5, and more preferably 1 to 3 hydrogen atoms in a group are each independently substituted with a corresponding number of substituents.

"Pharmaceutically acceptable salt" refers to a salt of a compound of the present disclosure, which is prepared from a compound having particular substituent(s) as found by the present disclosure and a relatively non-toxic acid or base. When a compound of the present disclosure contains a relatively acidic functional group, a base addition salt may be obtained by contacting the neutral form of such compound with a sufficient amount of a base in a pure solution or a suitable inert solvent. Pharmaceutically acceptable base addition salts include salts of sodium, potassium, calcium, ammonium, organic amine or magnesium or similar salts. When a compound of the present disclosure contains a relatively basic functional group, an acid addition salt may be obtained by contacting the neutral form of such compound with a sufficient amount of an acid in a pure solution or a suitable inert solvent. Examples of pharmaceutically acceptable acid addition salts include an inorganic acid salt, wherein said inorganic acid includes, for example, hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphorous acid, and the like; an organic acid salt, wherein said organic acid includes, for example, acetic acid, propionic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid, and methanesulfonic acid, and the similar acids; a salt of an amino acid (such as arginine and the like); and a salt of an organic acid such as glucuronic acid and the like (refer to Berge et al., "Pharmaceutical Salts", Journal of Pharmaceutical Science 66: 1-19 (1977)). Certain specific compounds of the present disclosure contain basic and acidic functional groups and thus may be converted to any base addition salt or acid addition salt. Preferably, through bringing the salt into contact with a base or an acid in a conventional manner and then separating the parent compound, the neutral form of the compound is thereby regenerated. The difference between the parent form of the compound and its various salt forms lies in certain physical properties, such as different solubility in a polar solvent.

"Pharmaceutical composition" means a mixture containing one or more compounds represented by formula I as described herein, or a pharmaceutically acceptable salt thereof, or a stereoisomer thereof, or a tautomer thereof, or a hydrate thereof, or a solvate thereof, or a metabolite thereof, or a prodrug thereof, other chemical components, and other components such as a pharmaceutically acceptable excipient. The purpose of a pharmaceutical composition is to promote the administration to an organism, facilitate the absorption of an active ingredient and thus exert biological activity.

The pharmaceutically acceptable salts of the present disclosure may be synthesized from the parent compounds containing acid radicals or basic radicals by conventional chemical methods. Generally, such salts are prepared by the following method: reacting these compounds in the form of free acid or base with a stoichiometric amount of an appropriate base or acid in water or an organic solvent or a mixture of both.

In addition to a salt form, the compounds provided by the present disclosure also exist in a form of prodrug. The prodrugs of the compounds described herein are apt to undergo chemical changes under physiological conditions to be converted into the compounds of the present disclosure. In addition, the prodrugs can be converted to the compounds of the present disclosure by chemical or biochemical methods in an *in-vivo* environment.

Certain compounds of the present disclosure may exist in unsolvated or solvated forms, including hydrated forms. Generally speaking, the solvated form is equivalent to the unsolvated form, and both are included within the scope of the present disclosure.

The compounds of the present disclosure may have specific geometric isomeric forms or stereoisomeric forms. The present disclosure contemplates all such compounds, including cis- and trans-isomers, (-)- and (+)-enantiomers, (R)- and (*S*)-enantiomers, diastereomers, (*D*)- and (*L*)-enantiomers, as well as the racemic mixtures and other mixtures thereof, such as enantiomerically or diastereomerically enriched mixtures, and all these mixtures fall within the scope of the present disclosure. Additional asymmetric carbon atom(s) may be present in the substituent(s) such as alkyl. All these isomers and the mixtures thereof are included within the scope of the present disclosure.

Unless otherwise specified, the term "enantiomer" or "optical isomer" refers to either of a pair of stereoisomers that are the mirror images of each other.

Unless otherwise specified, the term "cis-trans isomer" or "geometric isomer" refers to an isomer resulting from the fact that the double bond or the single bond between ring-forming carbon atoms cannot rotate freely.

Unless otherwise specified, the term "diastereomer" refers to either of a pair of stereoisomers, of which the molecule has two or more chiral centers and which has a non-mirror-image relationship with the other stereoisomer of the molecule.

Unless otherwise specified, "(*D*)" or "(+)" means right-handed, "(*L*)" or "(-)" means left-handed, and "(*DL*)" or "(±)" means racemic.

Unless otherwise specified, the wedge-shaped solid-line bond ( 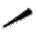 ) and the wedge-shaped dashed-line bond ( 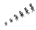 ) are used to represent the absolute configuration of a stereogenic center, and the straight solid-line bond ( 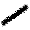 ) and the straight dashed-line bond ( 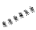 ) are used to represent the relative configuration of a stereogenic center. The wavy line ( 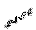 ) is used to represent a wedge-shaped solid-line bond ( 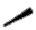 ) or a wedge-shaped dashed-line bond ( 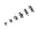 ), or the wavy line ( 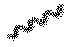 ) is used to represent a straight solid-line bond ( 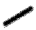 ) and a straight dashed-line bond ( 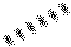 ).

The compounds of the present disclosure may have specific tautomeric forms. Unless otherwise specified, the term "tautomer" or "tautomeric form" refers to each of the isomers in which different isomeric forms of a functional group are in dynamic equilibrium and may rapidly convert to each other at room temperature. If tautomers possibly exist (for example, exist in a solution), a chemical equilibrium between the tautomers may be achieved. For example, proton tautomers (also referred to as prototropic tautomers) involve interconversions via proton migration, such as keto-enol isomerization and imine-enamine isomerization. Valence tautomers involve interconversions via recombination of some bonding electrons. Among them, a specific example of keto-enol tautomerization is the interconversion between the following two tautomers: pentane-2,4-dione and 4-hydroxypent-3-en-2-one.

Unless otherwise specified, the terms "enriched in one isomer", "isomerically enriched", "enriched in one enantiomer" or "enantiomerically enriched" mean that the content of one of the isomers or enantiomers is less than 100%, and the content of this isomer or enantiomer is greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise specified, the term "isomeric excess" or "enantiomeric excess" refers to the difference between the relative percentages of two isomers or two enantiomers. For example, if the content of one isomer or enantiomer is 90% and the content of the other isomer or enantiomer is 10%, then the isomeric or enantiomeric excess (ee value) is 80%.

The optically active (R)- and (*S*)-isomers and (*D*)- and (*L*)-isomers may be prepared by chiral synthesis, or with chiral reagents, or by other conventional techniques. If an enantiomer of a certain compound of the present disclosure is desired, it may be prepared by asymmetric synthesis or derivatization that uses a chiral auxiliary, in which the resulting mixture of diastereomers is separated and the auxiliary group is cleaved to provide the desired pure enantiomer. Alternatively, when the molecule contains a basic functional group (such as amino) or an acidic functional group (such as carboxyl), a salt of the diastereomer is formed by the molecule and an appropriate optically active acid or base, then diastereomeric resolution is performed by conventional methods well known in the art, and the pure enantiomer is obtained by recovery. In addition, the separation of enantiomers and diastereomers is usually accomplished by using chromatography, which adopts a chiral stationary phase and is optionally combined with a chemical derivatization method (for example, the formation of carbamate from amine). The compounds of the present disclosure may contain an atomic isotope in an unnatural proportion at one or more atoms constituting such compounds. For example, the compounds may be labeled with a radioisotope, such as tritium (³H), iodine-125 (¹²⁵I) or C-14 (¹⁴C). For another example, hydrogen may be substituted with heavy hydrogen to form a deuterated drug. The bond formed by deuterium and carbon is stronger than the bond formed by ordinary hydrogen and carbon. Compared with an undeuterated drug, a deuterated drug has advantages such as reduced toxicity and side effects, increased drug stability, enhanced efficacy, and prolonged biological half-life of drugs. All variations of the isotopic composition of the compounds of the present disclosure are included within the scope of the present disclosure regardless of the radioactivity.

Additional aspects and advantages of the present disclosure will be set forth in part in the following description, and, in part, will become obvious from the following description, or may be learned by the practice of the present disclosure.

### DETAILED DESCRIPTION

Preparation of the compounds of the present disclosure, or pharmaceutically acceptable salts thereof, or stereoisomers thereof, or tautomers thereof, or hydrates thereof, or solvates thereof, or metabolites thereof, or prodrugs thereof can be accomplished by the exemplary methods described in the following Examples and operations in related publications used by a person skilled in the art, but these Examples are not intended to limit the scope of the present disclosure.

The structures of the compounds of the present disclosure are determined by nuclear magnetic resonance (NMR) or mass spectrometry (MS). NMR determination is carried out by using Bruker AVANCE-400 or Varian Oxford-300 Nuclear Magnetic Resonance Spectrometer, deuterated dimethyl sulfoxide (DMSO-d₆), deuterated chloroform (CDC1₃) or deuterated methanol (CD₃OD) is used as solvent, and tetramethylsilane (TMS) is used as the internal standard; and the chemical shift is provided in the unit of 10⁻⁶ (ppm).

MS determination is carried out by using Agilent SQD (ESI) Mass Spectrometer (manufacturer: Agilent, model: 6110) or Shimadzu SQD (ESI) mass spectrometer (manufacturer: Shimadzu, model: 2020).

HPLC determination is carried out by using Agilent 1200DAD High-pressure Liquid Chromatograph (Sunfirc C18, 150 × 4.6 mm, 5 µm, column) and Waters 2695-2996 High-pressure Liquid Chromatograph (Gimini C18, 150 × 4.6 mm, 5 µm column).

GF254 silica gel plates supplied by Qingdao Haiyang Chemical Co Ltd are used as the silica gel plates for the thin-layer chromatography. The specifications of the silica gel plates used in thin-layer chromatography (TLC) are 0.15 mm to 0.2 mm, and the specifications of the silica gel plates used for the separation and purification of products by thin-layer chromatography are 0.4 mm to 0.5 mm.

200-300 mesh silica gel supplied by Qingdao Haiyang Chemical Co Ltd is generally adopted as the carrier for column chromatography.

The known starting materials in the present disclosure may come from companies such as Accela ChemBio Inc. and Beijing Ouhe Technology Co. Ltd, etc., or may be synthesized according to methods known in the art.

Unless otherwise specified in the Examples, the reactions were all carried out under an argon atmosphere or a nitrogen atmosphere.

An argon atmosphere or a nitrogen atmosphere means that the reaction flask is connected to an argon or nitrogen balloon with a volume of about 1 L.

A hydrogen atmosphere means that the reaction flask is connected to a hydrogen balloon with a volume of about 1 L. A hydrogenation reaction is usually carried out by evacuating, filling the reaction flask with hydrogen, and repeating the operations three times.

Unless otherwise specified in the Examples, the reaction temperature is room temperature, and the temperature ranges from 20°C to 30°C.

Thin-layer chromatography (TLC) is adopted to monitor the process of reactions in the Examples. Systems used as developing solvents in the reactions include: A: dichloromethane and methanol system; B: petroleum ether and ethyl acetate system, wherein the volume ratio of the solvents is adjusted according to the polarity of compounds.

Unless otherwise specified in the Examples, using preparative HPLC (formic acid) method means that the compound is separated and obtained by chromatography in a formic acid system (phase A: H₂O + 0.225% formic acid, phase B: acetonitrile).

The eluent systems of the column chromatography used to purify the compound and the developing solvent systems of thin-layer chromatography include: A: dichloromethane and methanol system; B: petroleum ether and ethyl acetate system, wherein the volume ratio of the solvents is adjusted according to the polarity of compounds, or may be adjusted by adding a small amount of triethylamine and an acidic or basic reagent.

The present disclosure is described in detail below by Examples, but it does not imply any disadvantageous limitation on the present disclosure. The compounds of the present disclosure may be prepared by a variety of synthetic methods well known to a person skilled in the art, including the specific embodiments listed below, embodiments formed by combining said specific embodiments with other chemical synthesis methods, and equivalent alternatives well known to a person skilled in the art. Preferred embodiments include, but are not limited to, the Examples of the present disclosure. Various changes and improvements made to the specific embodiments of the present disclosure without departing from the spirit and scope of the present disclosure will be apparent to a person skilled in the art. The following synthetic schemes describe the steps for preparing the compounds disclosed in the present disclosure. Unless otherwise specified, each substituent has the definition as described in the present disclosure.

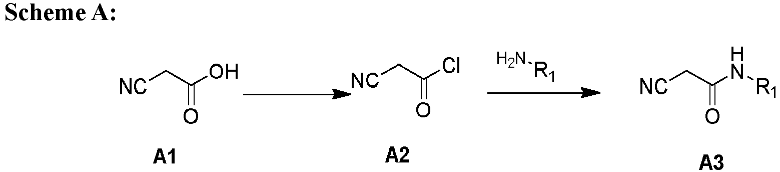

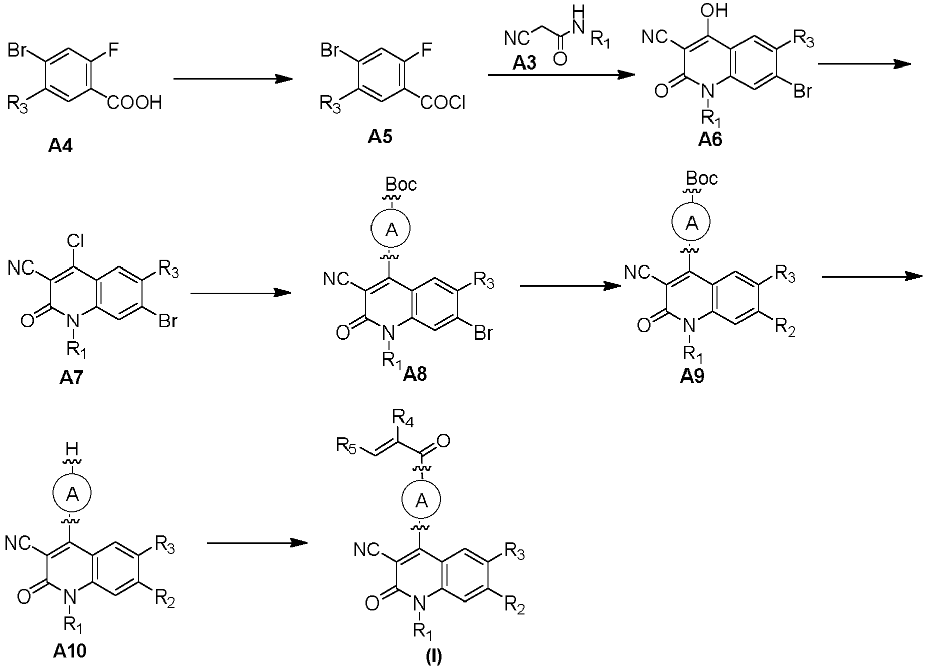

Compound A1 is reacted with thionyl chloride or oxalyl chloride to obtain A2, and then A2 is reacted with an amine-based compound to obtain A3. Compound A4 is reacted with thionyl chloride or oxalyl chloride to obtain A5. Compounds A5 and A3 undergo intramolecular ring closure under the action of a suitable strong base, such as sodium hydride or LiHMDS, to obtain A6. Compound A6 is reacted with a suitable chlorinating reagent (such as phosphorus oxychloride) to obtain A7. Compound A7 is reacted with a Boc-protected amine under the action of a suitable base (such as TEA or DIPEA) to obtain Compound A8. Compound A8 is subjected to a suzuki reaction with the corresponding boronic acid or boronic ester under a suitable condition with a palladium catalyst (such as Pd(dppf)₂Cl₂ dichloromethane complex) to obtain Compound A9. Compound A9 undergoes a deprotection reaction under an acidic condition to obtain A10. Compound A10 is reacted with a suitable acylation reagent (such as acryloyl chloride) in the presence of a suitable base (such as TEA or DIPEA) to obtain Compound (I).

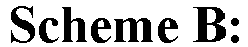

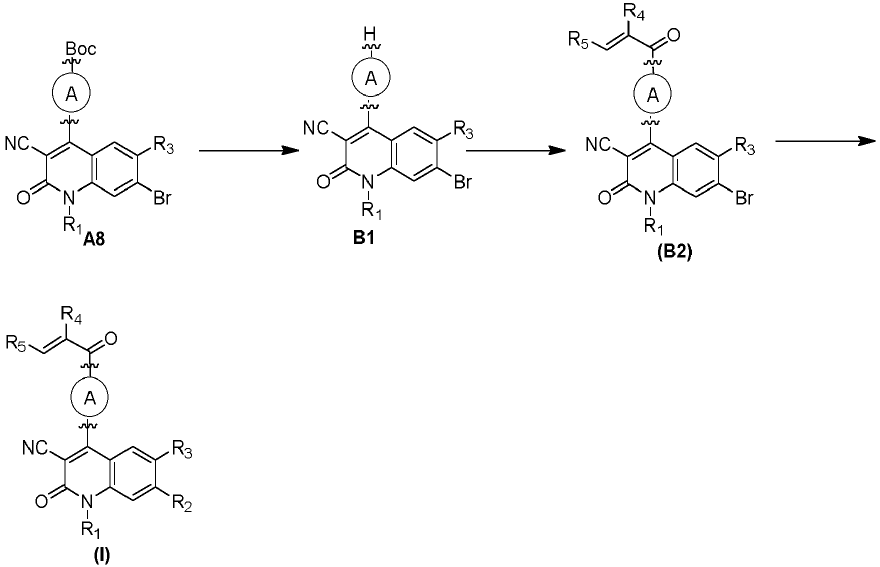

Compound A8 undergoes a deprotection reaction under an acidic condition to obtain B1. Compound A9 undergoes a deprotection reaction under an acidic condition to obtain A10. Compound A10 is reacted with a suitable acylation reagent (such as acryloyl chloride) in the presence of a suitable base (such as TEA or DIPEA) to obtain Compound B2. Compound B2 is subjected to a suzuki reaction with the corresponding boronic acid or boronic ester to obtain Compound (I).

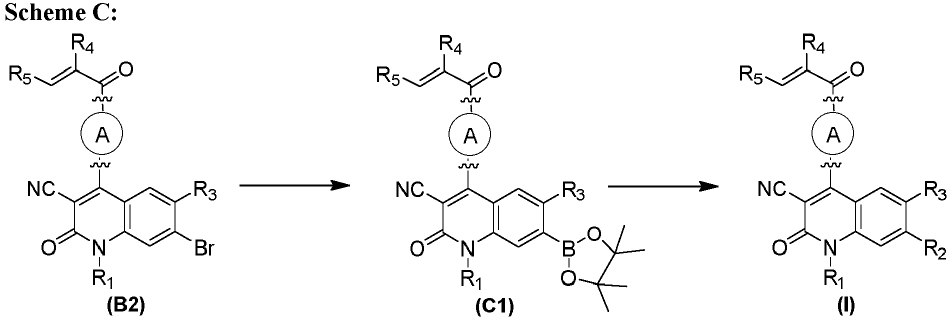

Compound B2 is reacted with bis(pinacolato)diboron under a suitable condition with a palladium catalyst (such as Pd(dppf)₂Cl₂ dichloromethane complex) to obtain intermediate C1 as a boronic ester. Compound C1 is reacted with the corresponding halide such as bromide or chloride under a suitable condition with a palladium catalyst (such as Pd(dppf)₂Cl₂ dichloromethane complex) to obtain Compound (I).

### Example 1: Preparation of Compound I-1

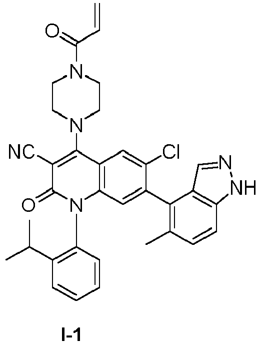

Synthesis Route (refer to Scheme A):

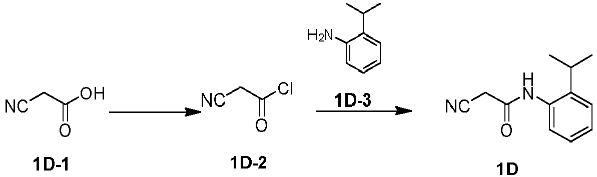

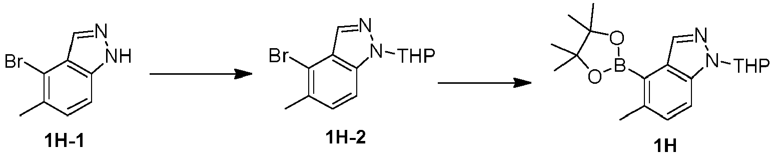

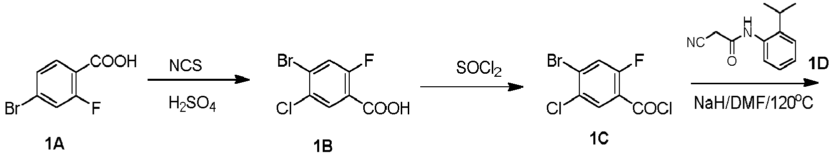

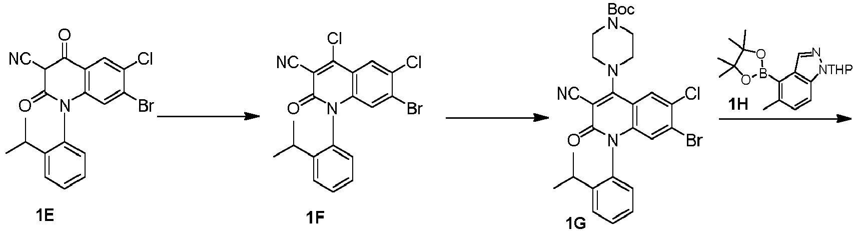

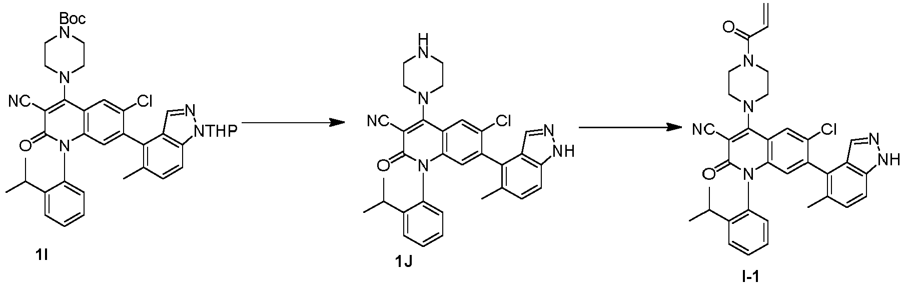

### Preparation Method:

### Step 1: Synthesizing Compound 1D-2

Oxalyl chloride (15.3 g, 120 mmol) and DMF (0.1 ml) were added to a mixture of 2-cyanoacetic acid 1D-1 (8.5 g, 100 mmol) in dichloromethane (DCM) (100mL), and then the resultant was stirred at room temperature for 3 hours. After TLC showed that the reaction was complete, the solvent was removed under reduced pressure to obtain Compound 1D-2 (11 g) as a white solid, which was used directly in the next step without further purification.

### Step 2: Synthesizing Compound 1D

Compound 1D-2 (11 g) obtained in the previous step was dissolved in DCM (100 mL), then 2-isopropylaniline ID-3 (14.8 g, 110 mmol) and triethylamine (20.2 g, 200 mmol) were added thereto, and stirring was then performed at room temperature for 3 hours. After TLC showed that the reaction was complete, the solvent was removed under reduced pressure, and the resulting residue was purified by silica gel column chromatography (petroleum ether/ethyl acetate = 10 : 1 (V : V volume ratio)) to obtain Compound 1D (17.8 g, white solid), yield: 88%.

MS m/z (ESI): 203[M+1].

### Step 3: Synthesizing Compound 1H-2

The compound 4-bromo-5-methyl-1H-indazole 1H-1 (3 g, 14.2 mmol) was added to DCM (30 mL). Then, 3,4-dihydro-2H-pyran (2.39 g, 28.4 mmol, 2.60 mL) and p-toluenesulfonic acid monohydrate (270 mg, 1.42 mmol) were successively added thereto, and the mixture was stirred at room temperature for 2 hours. After TLC showed the completion of the reaction, the reaction mixture was concentrated under vacuum, and the resulting residue was purified by silica gel column chromatography (petroleum ether/ethyl acetate = 10 : 1 (V : V volume ratio)) to obtain Compound 1H-2 (4 g, white solid), yield: 95.3%.

MS m/z (ESI): 297[M+1].

### Step 4: Synthesizing Compound 1H

Compound 1H-2 (550 mg, 1.85 mmol) was added to dioxane (10 ml). KOAc (364 mg, 3.7 mmol) and bis(pinacolato)diboron (705 mg, 2.8 mmol) were added thereto. After the reaction solution was purged with nitrogen gas three times, a dichloromethane complex of Pd(dppf)Cl₂ (159 mg, 0.19 mmol) was added successively. The resulting reaction solution was again purged with nitrogen gas three times and then stirred overnight at 100°C. After TLC showed that the reaction was complete, the reaction solution was concentrated under vacuum, and the resulting residue was purified by silica gel column chromatography (petroleum ether/ethyl acetate = 10 : 1 (V : V volume ratio)) to obtain Compound 1H (429 mg, light yellow transparent liquid), yield: 67.3%.

MS m/z (ESI): 345[M+1].

### Step 5: Synthesizing Compound 1B

Compound 1A (22 g, 100 mmol) was added to concentrated sulfuric acid (250 ml) at room temperature. The mixture was stirred and dissolved, and then the resulting solution was cooled to 0°C to 5°C. Thereafter, N-chlorosuccinimide (NCS) (13.3 g, 100 mmol) was added in portions. After the addition, the resulting solution was warmed up to room temperature and allowed to react overnight. TLC showed that the reaction was complete. Stirring was performed while the reaction solution was slowly added into a large amount of ice water (1 L), and a large amount of solid precipitated. Filtering was carried out and the solid was washed with water. The obtained solid was further purified by silica gel column chromatography (dichloromethane/methanol/formic acid = 100/1/0.1 (V : V : V volume ratio)) to obtain Compound 1B (11.4 g, light yellow solid), yield: 45%.

MS m/z(ESI): 253[M+1].

### Step 6: Synthesizing Compound 1C

Compound 1B (10 g, 39.5 mmol) was dissolved in dichloromethane (150 ml) at room temperature, then thionyl chloride (9.4 g, 79 mmol) was added thereto, and thereafter the mixture was warmed up to 45°C to react for 2 hours. TLC showed that the reaction was complete. The solvent was removed by rotary evaporation, so as to obtain Compound 1C (13 g), which was used directly in the next step without purification.

### Step 7: Synthesizing Compound 1E

Compound 1D (8 g, 40 mmol) was added to DMF (100 ml) at room temperature, the mixture was stirred and dissolved, and then the resulting solution was cooled to 0°C to 5°C in an ice-water bath. Thereafter, 60% sodium hydride (4 g, 100 nmol) was added in portions. After the addition, the reaction solution was warmed up to room temperature and allowed to continue the reaction for half an hour. Then, the DMF solution (50 ml) of Compound 1C (13 g) obtained in the previous step was added dropwise into a reaction flask. After the addition, stirring was performed at room temperature for 1 hour, and then the solution was warmed up to 120°C for reacting for 4 hours. TLC showed that the reaction was complete, and LCMS showed the target MS. After the reaction solution was cooled to room temperature, water (500 ml) was slowly added for dilution, and the pH was adjusted to 3 to 4 with hydrochloric acid (6 N), and extraction was conducted with dichloromethane (3×150 mL). The organic phases were combined and dried over anhydrous sodium sulfate; the desiccant was removed by filtration; and the solvent was removed under reduced pressure. The residue was purified by silica gel column chromatography (dichloromethane/methanol = 100/1 (V : V volume ratio)) to obtain Compound 1E (2.14 g, light yellow solid), yield: 13%.

MS m/z(ESI): 417[M+1].

### Step 8: Synthesizing Compound 1F

Compound 1E (2 g, 4.8 mmol) was added to toluene (30 ml) at room temperature, and then phosphorus oxychloride (1.5 g, 9.6 mmol) was added thereto. After the addition, the reaction solution was warmed up to 110°C and allowed to react overnight. After TLC showed that the reaction was complete, the reaction solution was cooled to room temperature. Stirring was performed while the reaction solution was poured into a large amount of ice water (100 ml) to quench the reaction. Then, a saturated sodium bicarbonate solution (100 ml) was added thereto, and extraction was conducted with ethyl acetate (3×50 mL). The organic phases were combined and dried over anhydrous sodium sulfate; the desiccant was removed by filtration; and the solvent was removed under reduced pressure. The residue was purified by silica gel column chromatography (petroleum ether/ethyl acetate = 10/1 (V : V volume ratio)) to obtain Compound 1F (1.4 g, light yellow solid), yield: 67%.

MS m/z(ESI): 435[M+1].

### Step 9: Synthesizing Compound 1G

Compound 1G (435 mg, 1 mmol) and N,N-diisopropylethylamine (DIPEA) (258 mg, 2 mmol) were added to DMF (5 ml) at room temperature. Then, N-Boc piperazine (372 mg, 2 mmol) was added thereto, the temperature was controlled to be 110°C, and the resultant was allowed to react overnight. TLC showed that the reaction was complete. The reaction solution was added to 20 ml of water and extraction was conducted with dichloromethane (3×10 ml). The resulting solution was successively washed with a saturated sodium carbonate solution (2×10 mL), water (2×10 mL) and brine (2×10 mL). The organic phase was dried over anhydrous sodium sulfate. The desiccant was removed by filtration. After the resultant was concentrated under reduced pressure, the residue was purified by silica gel column chromatography (dichloromethane/methanol = 100/1 (V : V volume ratio) to obtain Compound 1G (423 mg, light yellow solid), yield: 73%.

MS m/z(ESI): 585[M+1].

### Step 10: Synthesizing Compound 1I

Compound 1G (1.17 g, 2 mmol) was added to a solution of dioxane (10 mL) and water (2 mL) at room temperature. Then, potassium phosphate (848 mg, 4 mmol), Compound 1H (460 mg, 3 mmol) and a dichloromethane complex of Pd(dppf)Cl₂ (162 mg, 0.2 mmol) were added thereto. The reaction solution was allowed to react at 100°C for 12 hours under nitrogen protection. TLC showed that the reaction was complete. A saturated sodium bicarbonate solution (50 mL) was added and extraction was conducted with dichloromethane (2×20 mL). The organic phases were combined and then dried over anhydrous sodium sulfate; the desiccant was removed by filtration; and the solvent was removed under reduced pressure. The residue was purified by silica gel column chromatography (petroleum ether/ethyl acetate = 3/1 (V : V volume ratio)) to obtain Compound 1I (822 mg, light yellow solid), yield: 57%.

MS m/z(ESI): 721[M+1].

### Step 11: Synthesizing the hydrochloride of Compound 1J

Compound 1I (820 mg, 1.14 mmol) was dissolved in ethyl acetate (5 mL) at room temperature. Then, a solution of hydrochloric acid in ethyl acetate (4 N, 5 ml) was added thereto. The mixture was stirred at room temperature for 2 hours. The solution changed from clear to turbid, and solid precipitated. The reaction was monitored by TLC. After the reaction was complete, the reaction solution was cooled to 0°C, allowed to stand for 1 hour, and then filtered. The solid was washed with ethyl ether and then dried to obtain the hydrochloride of Compound 1J (599 mg, white solid), yield: 92%.

MS m/z(ESI): 537[M+1].

### Step 12: Synthesizing the compound represented by Formula I-1

The hydrochloride (599 mg) of Compound 1J obtained in the previous step was dissolved in dichloromethane (10 ml), and then the resultant was cooled to -10°C. Triethylamine (202 mg, 2 mmol) and acryloyl chloride (100 mg, 1.1 mmol) were added thereto successively. Then, after the mixture was naturally warmed up to room temperature and allowed to react for 1 hour, TLC showed the completion of the reaction. The reaction was quenched by adding MeOH (1 mL). The residue obtained after drying the reaction mixture by rotary evaporation was separated and purified by preparative HPLC (formic acid) to obtain the target compound represented by formula I-1 (33 mg, white solid).

MS m/z(ESI): 591[M+1].

¹H NMR (400 MHz, MeOD) 8.20 (s, 1H), 7.50-7.45 (m, 3H), 7.36-7.27 (m, 3H), 7.143 (d, J=8Hz, 1H), 6.92-6.99(m, 1H), 6.47(s,1H), 6.31 (dd, J=2.0, 16.8 Hz, 1H), 5.84 (dd, J=1.6, 10.4 Hz, 1H), 4.03(brs,4H), 3.90(brs,4H), 2.64-2.62 (m,1H), 2.08(s,3H), 1.17(d, J=7.2Hz,3H), 1.00(d, J=6.8Hz,3H).

### Example 2: Preparation of Compound I-2-1 and Compound 1-2-2

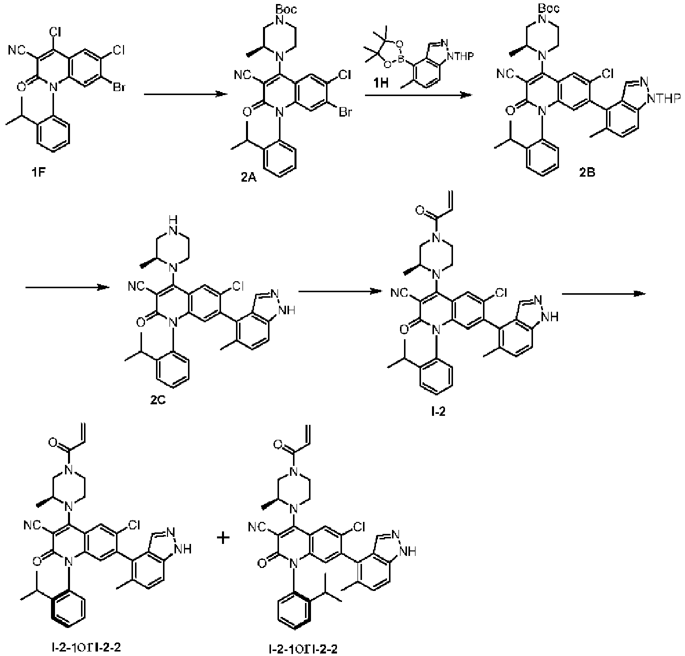

### Step 1: Synthesizing Compound 2A

Compound 1G (435 mg, 1 mmol) and N,N-diisopropylethylamine (DIPEA) (258 mg, 2 mmol) were added to DMF (5 ml) at room temperature. Then, (S)-4-N-tert-butoxycarbonyl-2-methylpiperazine (400 mg, 2 mmol) was added thereto. The temperature was controlled to be 110°C and the resultant was allowed to react overnight. TLC showed that the reaction was complete. The reaction solution was added to 20 ml of water and extraction was conducted with dichloromethane (3×10 ml). The resulting solution was successively washed with a saturated sodium carbonate solution (2×10 mL), water (2×10 mL) and brine (2×10 mL). The organic phase was dried over anhydrous sodium sulfate. The desiccant was removed by filtration. After the resultant was concentrated under reduced pressure, the residue was purified by silica gel column chromatography (dichloromethane/methanol = 100/1 (V : V volume ratio)) to obtain Compound 2A (480 mg, light yellow solid), yield: 80.2%.

MS m/z(ESI): 599[M+1].

### Step 2: Synthesizing Compound 2B

Compound 1G (1.2 g, 2 mmol) was added to a solution of dioxane (10 mL) and water (2 mL) at room temperature. Then, potassium phosphate (848 mg, 4 mmol), Compound 1H (460 mg, 3 mmol) and a dichloromethane complex of Pd(dppf)Cl₂ (162 mg, 0.2 mmol) were added thereto. The reaction solution was allowed to react at 100°C for 12 hours under nitrogen protection. TLC showed that the reaction was complete. A saturated sodium bicarbonate solution (50 mL) was added and extraction was conducted with dichloromethane (2×20 mL). The organic phases were combined and then dried over anhydrous sodium sulfate; the desiccant was removed by filtration; and the solvent was removed under reduced pressure. The residue was purified by silica gel column chromatography (petroleum ether/ethyl acetate = 3 : 1 (V : V volume ratio)) to obtain Compound 2B (926 mg, light yellow solid), yield: 63%.

MS m/z(ESI): 735[M+1].

### Step 3: Synthesizing the hydrochloride of Compound 2C

Compound 2B (837 mg, 1.14 mmol) was dissolved in ethyl acetate (5 mL) at room temperature. Then, a solution of hydrochloric acid in ethyl acetate (4 N, 5 ml) was added thereto. The mixture was stirred at room temperature for 2 hours. The solution changed from clear to turbid, and solid precipitated. The reaction was monitored by TLC. After the reaction was complete, the reaction solution was cooled to 0°C, allowed to stand for 1 hour, and then filtered. The solid was washed with ethyl ether and then dried to obtain the hydrochloride of Compound 2C (602 mg, white solid).

MS m/z(ESI): 551[M+1].

### Step 4: Synthesizing the compound represented by Formula 1-2

The hydrochloride (551 mg) of Compound 2C obtained in the previous step was dissolved in dichloromethane (10 ml), and then the resultant was cooled to -10°C. Triethylamine (202 mg, 2 mmol) and acryloyl chloride (100 mg, 1.1 mmol) were added theretosuccessively. Then, after the mixture was naturally warmed up to room temperature and allowed to react for 1 hour, TLC showed the completion of the reaction. The reaction was quenched by adding MeOH (1 mL). The residue obtained after drying the reaction mixture by rotary evaporation was separated and purified by preparative HPLC to obtain the target compound represented by Formula 1-2 (130 mg, light yellow solid). MS m/z(ESI):605[M+1].

### Step 5: Synthesizing the compounds represented by Formula 1-2-1 and Formula 1-2-2

Compound 1-2 was subjected to chiral resolution by SFC (column model: CHIRALPAK IC, 250 mm ^{∗} 30 mm, 5 µm; mobile phase A: n-hexane/dichloromethane (75/25, containing 10 mM methylamine); mobile phase B: methanol, detection wavelength: 254 nm) to obtain Compound 1-2-1 (t_{R} = 3.55 min) and Compound 1-2-2 (t_{R} = 4.49 min).

Compound represented by Formula 1-2-1:
MS m/z(ESI):605[M+1].

¹H NMR (400 MHz, MeOD) 8.23 (s, 1H), 7.52-7.43 (m, 3H), 7.38-7.27 (m, 3H), 7.21-7.12 (m, 1H), 6.95-6.83(m, 1H), 6.48(s,1H), 6.33 (d, J=16.4 Hz, 1H), 5.85 (dd, J=1.6, 10.4 Hz, 1H), 4.42-4.14(m,2H), 4.10-3.90(m,2H), 3.72-3.45 (m,3H), 2.70-2.56(m,1H), 2.09(s,3H), 1.40-1.33(m,3H), 1.22-1.15(m, 3H), 1.03-0.92(m, 3H).

Compound represented by Formula 1-2-2:
MS m/z(ESI):605[M+1].

¹H NMR (400 MHz, MeOD) 8.22 (s, 1H), 7.51-7.43 (m, 3H), 7.37-7.29 (m, 3H), 7.27-7.24(m, 1H), 6.95-6.83(m, 1H), 6.50(s,1H), 6.33 (d, J=16.4 Hz, 1H), 5.84 (dd, J=1.6, 10.4 Hz, 1H), 4.42 (brs,2H), 4.13-4.09(m,2H), 3.65-3.50 (m,3H), 2.72-2.51(m,1H), 2.09(s,3H), 1.38-1.35(m,3H), 1.20-1.15(m, 3H), 1.04-0.90(m,3H).

### Example 3: Preparation of Compound 1-3-1 and Compound 1-3-2

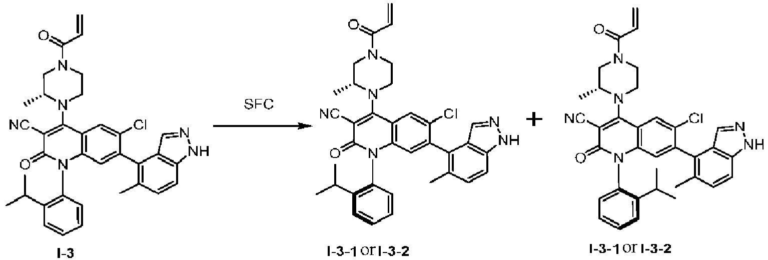

Step 1: Compound 1-3 was synthesized by referring to the synthesis method of Compound 1-2 in Example 2.

Step 2: Compound 1-3 was subjected to chiral resolution by SFC (column model: CHIRALPAK IC, 250 mm ^{∗} 30 mm, 5 µm; mobile phase A: n-hexane/dichloromethane (75/25, containing 10 mM methylamine); mobile phase B: methanol, detection wavelength: 254 nm) to obtain Compound 1-3-1 (t_{R} = 3.22 min) and Compound 1-3-2 (t_{R} = 4.25 min).

Compound represented by Formula 1-3-1:
MS m/z(ESI):605[M+1].

¹H NMR (400 MHz, MeOD) 8.23 (s, 1H), 7.52-7.24 (m, 7H), 6.90-6.89 (m, 1H), 6.51 (m, 1H), 6.36-6.30 (m, 1H), 5.87-5.84 (m, 1H), 4.49-4.08 (m, 5H), 3.55-3.48 (m, 2H), 2.55-2.52 (m, 1H), 2.10 (s, 3H), 1.37 (s, 3H), 1.17 (s, 3H), 1.00 (s, 3H).

Compound represented by Formula 1-3-2:
MS m/z(ESI):605[M+1].

¹H NMR (400 MHz, MeOD) 8.23 (s, 1H) , 7.47-7.21 (m, 7H), 6.85 (m, 1H), 6.50-6.33 (m, 2H), 5.88-5.85 (m, 1H), 4.46-3.54 (m, 7H), 2.57-2.56 (m, 1H), 2.08 (m, 3H), 1.37 (s, 3H), 1.17 (s, 3H), 1.00 (s, 3H).

### Example 4: Preparation of Compound I-4-1 and Compound I-4-2

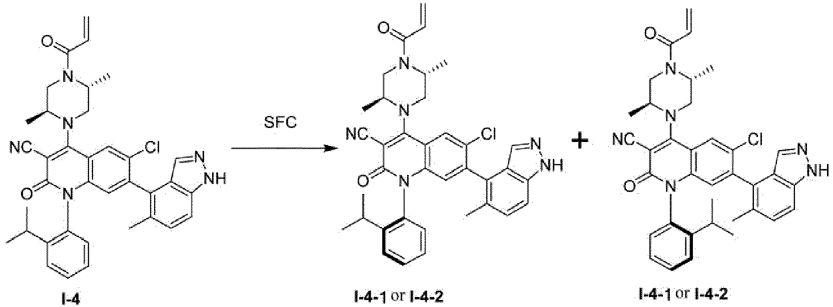

Step 1: Compound 1-4 was synthesized by referring to the synthesis method of Compound 1-2 in Example 2.

Step 2: Compound 1-4 was subjected to chiral resolution by SFC (column model: CHIRALPAK IC, 250 mm ^{∗} 30 mm, 5 µm; mobile phase A: n-hexane/dichloromethane (75/25, containing 10 mM methylamine); mobile phase B: methanol, detection wavelength: 254 nm) to obtain Compound 1-4-1 (t_{R} = 3.31 min) and Compound 1-4-2 (t_{R} = 4.31 min).

Compound represented by Formula 1-4-1:
MS m/z(ESI):619[M+1].

¹H NMR (400 MHz, MeOD) 8.20 (m, 1H) , 7.51 (m, 3H), 7.47-7.25 (m, 5H), 6.50 (s, 1H), 6.32 (m, 1H), 5.85 (m, 1H), 4.60-4.40 (m, 5H), 4.52 (m, 2H), 2.51-2.40 (m, 1H), 2.08 (s, 3H), 1.53-1.47 (m, 8H), 1.26-1.21 (m, 3H), 1.03-0.96 (m, 3H).

Compound represented by Formula 1-4-2:
MS m/z(ESI):619[M+1].

¹H NMR (400 MHz, MeOD) 8.17-8.15 (m, 1H) , 7.55-7.31 (m, 7H), 6.90 (m, 1H), 6.54-6.51 (m, 1H), 6.36-6.33 (m, 1H), 5.85 (m, 1H), 4.90 (m, 3H), 4.59-4.45 (m, 3H), 3.55 (m, 2H), 2.71 (m, 1H), 2.13-2.12 (m, 3H), 1.52-1.50 (m, 7H), 1.22-1.21 (m, 3H), 1.05 (m, 3H).

### Example 5: Preparation of Compound I-5-1 and Compound I-5-2

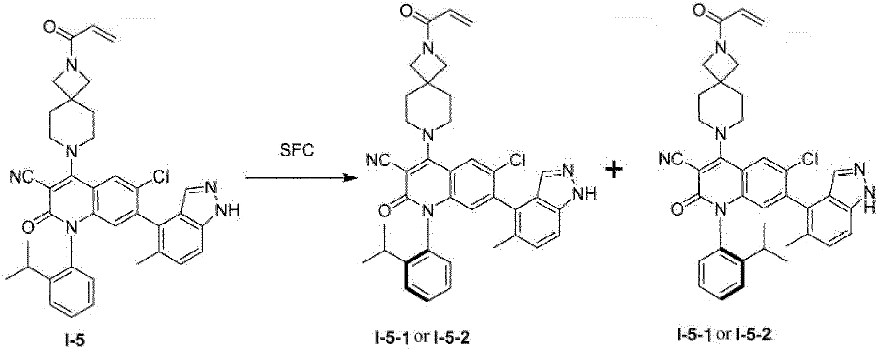

### Step 1:

Compound 1-5 was synthesized by referring to the synthesis method of Compound 1-2 in Example 2.

### Step 2:

Compound 1-5 was subjected to chiral resolution by SFC (column model: CHIRALPAK IC, 250 mm ^{∗} 30 mm, 5 µm; mobile phase A: n-hexane/dichloromethane (75/25, containing 10 mM methylamine); mobile phase B: methanol, detection wavelength: 254 nm) to obtain Compound 1-5-1 (t_{R} = 3.43 min) and Compound 1-5-2 (t_{R} = 4.51 min).

Compound represented by Formula 1-5-1:
MS m/z(ESI):631[M+1].

¹H NMR (400 MHz, MeOD) 8.15 (m, 1H) , 7.45-7.26 (m, 7H), 6.44-6.26 (m, 3H), 5.76-5.75 (m, 1H), 5.48-5.47 (m, 1H), 4.20-4.18 (m, 2H), 2.63-2.58 (m, 1H), 2.18-2.15 (m, 10H), 1.16-1.14 (m, 3H), 1.01-0.99 (m, 3H).

Compound represented by Formula 1-5-2:
MS m/z(ESI):631[M+1].

¹H NMR (400 MHz, MeOD) 8.16 (m, 1H) , 7.42-7.24 (m, 7H), 6.43-6.25 (m, 3H), 5.75-5.74 (m, 1H), 5.49-5.47(m, 1H), 4.22-4.20 (m, 2H), 2.64-2.59(m, 1H), 2.18-2.15 (m, 10H), 1.17-1.15 (m, 3H), 1.01-0.98(m, 3H).

### Example 6: Preparation of Compound I-6-1 and Compound I-6-2

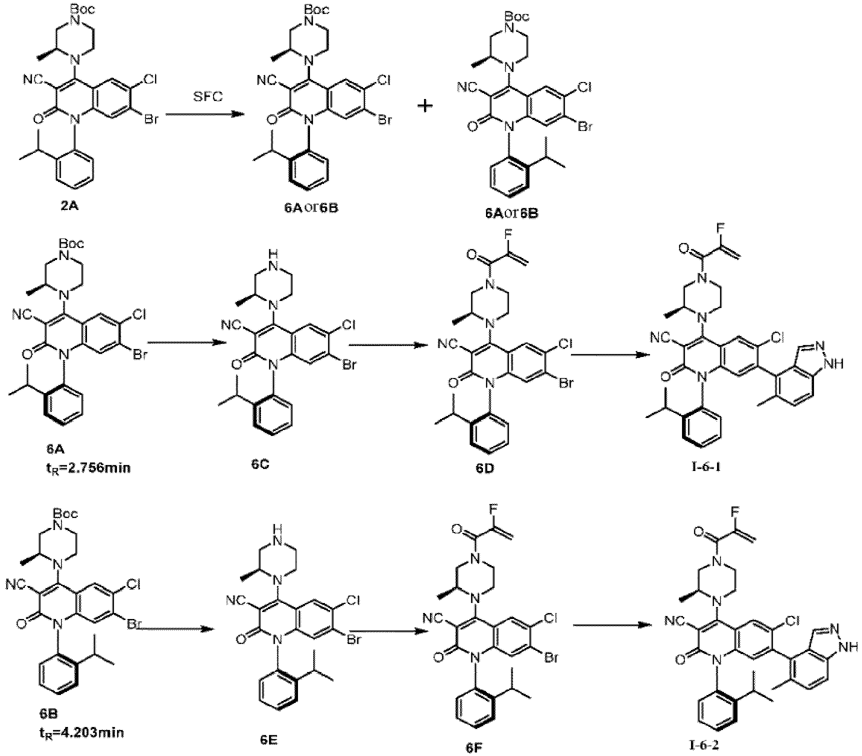

### Step 1: Synthesizing Compounds 6A and 6B

Compound 2A was subjected to chiral resolution by SFC (column model: CHIRALPAK IC, 250 mm ^{∗} 30 mm, 5 µm; mobile phase A: n-hexane/dichloromethane (75/25, containing 10 mM methylamine); mobile phase B: methanol, detection wavelength: 254 nm) to obtain Compound 6A (t_{R} = 2.756 min) and Compound 6B (t_{R} = 4.203 min).

### Step 2: Synthesizing Compound 6C

Compound 6A (681 mg, 1.14 mmol) was dissolved in ethyl acetate (5 mL) at room temperature. Then, a solution of hydrochloric acid in ethyl acetate (4 N, 5 ml) was added thereto. The mixture was stirred at room temperature for 2 hours. The solution changed from clear to turbid, and solid precipitated. The reaction was monitored by TLC. After the reaction was complete, the reaction solution was cooled to 0°C, allowed to stand for 1 hour, and then filtered. The solid was washed with ethyl ether and then dried to obtain the hydrochloride of Compound 6C (450 mg, white solid).

MS m/z(ESI): 499[M+1].

### Step 3: Synthesizing Compound 6D

The hydrochloride (400 mg) of Compound 6C obtained in the previous step was dissolved in DMF (10 ml). At room temperature, triethylamine (202 mg, 2 mmol), HATU (760 mg, 2 mmol) and 2-fluoroacrylic acid (100 mg, 1.1 mmol) were added thereto successively. Then, after the mixture was naturally warmed up to room temperature and allowed to react for 1 hour, TLC showed the completion of the reaction. The reaction solution was added to 20 ml of water and extraction was conducted with dichloromethane (3×10 ml). The resulting solution was successively washed with a saturated sodium carbonate solution (2×10 mL), water (2×10 mL) and brine (2×10 mL). The organic phase was dried over anhydrous sodium sulfate. The desiccant was removed by filtration. After the resultant was concentrated under reduced pressure, the residue was purified by silica gel column chromatography (dichloromethane/methanol = 100/1 (V : V volume ratio) to obtain Compound 6D (280 mg, light yellow solid).

MS m/z(ESI): 571[M+1].

### Step 4: Synthesizing Compound 1-6-1

Compound 6D (200 mg, 0.35 mmol) was added to dioxane (2 ml) and water (0.5 ml). K₃PO₄ (212 mg, 1.00 mmol) and 5-methyl-1H-indazol-4-yl-4-boronic acid (176 mg, 1 mmol) were added thereto. After the reaction solution was purged with nitrogen gas three times, a dichloromethane complex of Pd(dppf)Cl₂ (25 mg, 0.03 mmol) was added. The resulting reaction solution was again purged with nitrogen gas three times and then stirred overnight at 100°C. After TLC showed that the reaction was complete, the reaction solution was concentrated under vacuum, and the resulting residue was purified by preparative silica gel plate (prepar-TLC) (developing solvent system: petroleum ether/ethyl acetate = 1 : 2 (V : V volume ratio)) to obtain Compound 1-6-1 (29 mg, light yellow solid).

MS m/z(ESI):623[M+1].

¹H NMR (400 MHz, CDCl₃) 8.01 (d, J =4.4 MHz,, 1H), 7.45-7.39 (m, 4H), 7.33-7.29 (m, 2H), 7.14-7.09 (m, 1H), 6.62 (s, 1H), 5.48-5.22 (m, 2H), 4.32-3.43 (m, 7H), 2.55-2.50 (m, 1H), 2.12 (s, 3H), 2.09 (s, 3H), 1.42 (d, J =6.4 MHz, 3H), 1.18 (d, J =6.8 MHz, 3H), 1.01 (d, J =6.8 MHz, 3H).

### Step 5: Synthesizing Compound 6E

Compound 6B (681 mg, 1.14 mmol) was dissolved in ethyl acetate (5 mL) at room temperature. Then, a solution of hydrochloric acid in ethyl acetate (4 N, 5 ml) was added thereto. The mixture was stirred at room temperature for 2 hours. The solution changed from clear to turbid, and solid precipitated. The reaction was monitored by TLC. After the reaction was complete, the reaction solution was cooled to 0°C, allowed to stand for 1 hour, and then filtered. The solid was washed with ethyl ether and then dried to obtain the hydrochloride of Compound 6E (446 mg, white solid).

MS m/z(ESI): 499[M+1].

### Step 6: Synthesizing Compound 6F

The hydrochloride (400 mg) of Compound 6E obtained in the previous step was dissolved in DMF (10 ml). At room temperature, triethylamine (202 mg, 2 mmol), HATU (760 mg, 2 mmol) and 2-fluoroacrylic acid (100 mg, 1.1 mmol) were added thereto successively. Then, after the mixture was naturally warmed up to room temperature and allowed to react for 1 hour, TLC showed the completion of the reaction. The reaction solution was added to 20 ml of water and extraction was conducted with dichloromethane (3×10 ml). The resulting solution was successively washed with a saturated sodium carbonate solution (2×10 mL), water (2×10 mL) and brine (2×10 mL). The organic phase was dried over anhydrous sodium sulfate. The desiccant was removed by filtration. After the resultant was concentrated under reduced pressure, the residue was purified by silica gel column chromatography (dichloromethane/methanol = 100/1 (V : V volume ratio) to obtain Compound 6F (272 mg, light yellow solid).

MS m/z(ESI): 571[M+1].

### Step 7: Synthesizing Compound 1-6-2

Compound 6F (200 mg, 0.35 mmol) was added to dioxane (2 ml) and water (0.5 ml). K₃PO₄ (212 mg, 1.00 mmol) and 5-methyl-1H-indazol-4-yl-4-boronic acid (176 mg, 1 mmol) were added thereto. After the reaction solution was purged with nitrogen gas three times, a dichloromethane complex of Pd(dppf)Cl₂ (25 mg, 0.03 mmol) was added. The resulting reaction solution was again purged with nitrogen gas three times and then stirred overnight at 100°C. After TLC showed that the reaction was complete, the reaction solution was concentrated under vacuum, and the resulting residue was purified by preparative silica gel plate (prepar-TLC) (developing solvent system: petroleum ether/ethyl acetate = 1 : 2 (V : V volume ratio)) to obtain Compound 1-6-2 (25 mg, light yellow solid).

MS m/z(ESI):623[M+1].

¹H NMR (400 MHz, CDCl₃) 8.03 (s,, 1H), 7.47-7.29 (m, 6H), 7.12-7.11 (m, 1H), 6.61 (s, 1H), 5.48-5.21 (m, 2H), 4.33-3.43 (m, 7H), 2.51-2.46 (m, 1H), 2.10 (s, 3H), 2.09 (s, 3H), 1.42 (d, J =6.4 MHz, 3H), 1.18 (d, J =6.8 MHz, 3H), 0.98 (d, J =6.8 MHz, 3H).

### Example 7: Preparation of Compound I-7-1 and Compound I-7-2

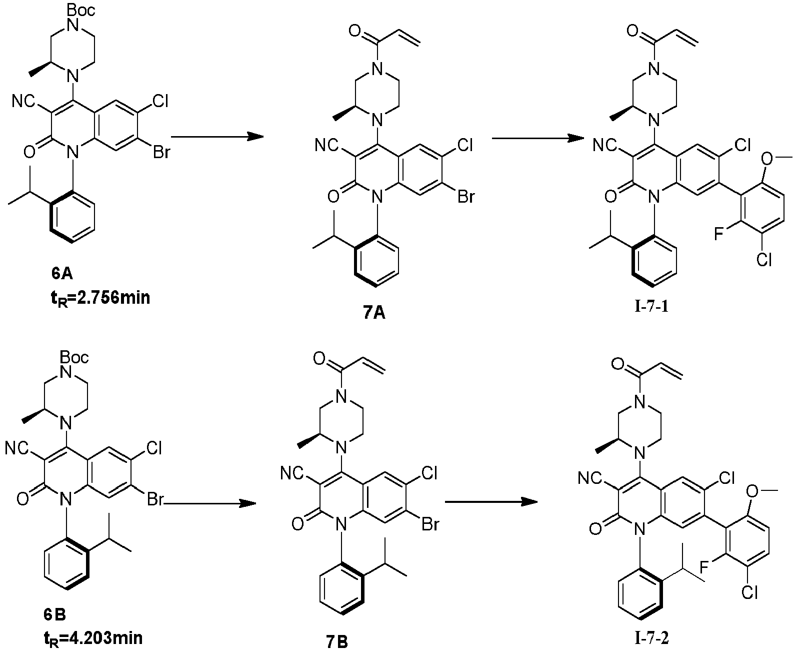

### Step 1: Synthesizing Compound 7A

Compound 6A (681 mg, 1.14 mmol) was dissolved in DCM (5 mL) at room temperature. Then, TFA (2 ml) was added thereto. The mixture was stirred at room temperature for 2 hours. The reaction was monitored by TLC. After the reaction was complete, the reaction solution was dried by rotary evaporation. The resulting yellow liquid compound was redissolved in DCM (5 mL), and then the resultant was cooled to -10°C. Triethylamine (303 mg, 3 mmol) and acryloyl chloride (150 mg, 1.5 mmol) were added thereto successively. Then, after the mixture was naturally warmed up to room temperature and allowed to react for 1 hour, TLC showed the completion of the reaction. The reaction was quenched by adding MeOH (1 mL). After the reaction mixture was concentrated under reduced pressure, the residue was purified by silica gel column chromatography (dichloromethane/methanol=100/1 (V : V volume ratio)) to obtain Compound 7A (272 mg, light yellow solid).

MS m/z(ESI): 553[M+1].

¹HNMR (400 MHz, CDCl₃) 7.92 (s, 1H), 7.57-7.56 (m, 2H), 7.44-7.40 (m, 1H), 7.11-7.09 (m, 1H), 6.88 (s, 1H), 6.64-6.60 (m, 1H), 6.42-6.37 (m, 1H), 5.83-5.80 (m, 1H), 4.29-4.00 (m, 4H), 3.75-3.56 (m, 3H), 2.43-2.36 (m, 1H), 1.32 (d, J=6.0 MHz, 3H), 1.18 (d, J=6.8 MHz, 3H), 1.04 (d, J=6.8 MHz, 3H).

### Step 2: Synthesizing Compound 1-7-1

Compound 7A (100 mg, 0.18 mmol) was added to dioxane (2 ml) and water (0.5 ml). K₃PO₄ (106 mg, 0.5 mmol) and 3-chloro-2-fluoro-6-methoxyphenylboronic acid (102 mg, 0.5 mmol) were added thereto. After the reaction solution was purged with nitrogen gas three times, a dichloromethane complex of Pd(dppf)Cl₂ (25 mg, 0.03 mmol) was added thereto. The resulting reaction solution was again purged with nitrogen gas three times and then stirred overnight at 100°C. After TLC showed that the reaction was complete, the reaction solution was concentrated under vacuum, and the resulting residue was purified by preparative silica gel plate (prepar-TLC) (developing solvent system: petroleum ether/ethyl acetate = 1 : 2 (V : V volume ratio)) to obtain Compound 1-7-1 (21 mg, light yellow solid).

MS m/z(ESI): 633[M+1].

¹H NMR (400 MHz, CDCl₃) 7.98 (s, 1H), 7.53-7.48 (m, 3H), 7.39-7.33 (m, 1H), 7.13-7.11 (m, 1H), 6.70-6.38 (m, 4H), 5.83-5.80 (m, 1H), 4.42-3.92 (m, 5H), 3.74 (s, 3H), 3.56-3.38 (m, 2H), 2.51-2.44 (m, 1H), 1.36 (d, J =6.0 MHz, 3H), 1.17 (d, J =6.8 MHz, 3H), 1.04 (d, J =6.8 MHz, 3H).

### Step 3: Synthesizing Compound 7B

Compound 6B (681 mg, 1.14 mmol) was dissolved in DCM (5 mL) at room temperature. Then, TFA (2 ml) was added thereto. The mixture was stirred at room temperature for 2 hours. The reaction was monitored by TLC. After the reaction was complete, the reaction solution was dried by rotary evaporation. The resulting yellow liquid compound was redissolved in DCM (5 mL), and then the resultant was cooled to -10°C. Triethylamine (303 mg, 3 mmol) and acryloyl chloride (150 mg, 1.5 mmol) were added thereto successively. Then, after the mixture was naturally warmed up to room temperature and allowed to react for 1 hour, TLC showed the completion of the reaction. The reaction was quenched by adding MeOH (1 mL). After the reaction mixture was concentrated under reduced pressure, the residue was purified by silica gel column chromatography (dichloromethane/methanol = 100/1 (V : V volume ratio)) to obtain Compound 7B (252 mg, light yellow solid).

MS m/z(ESI): 553[M+1].

¹HNMR (400 MHz, CDCl₃) 7.93 (s, 1H), 7.58-7.57 (m, 2H), 7.44-7.41 (m, 1H), 7.12-7.09 (m, 1H), 6.87 (s, 1H), 6.65-6.60 (m, 1H), 6.43-6.37 (m, 1H), 5.84-5.81 (m, 1H), 4.29-4.00 (m, 4H), 3.76-3.57 (m, 3H), 2.44-2.36 (m, 1H), 1.31 (d, J=6.0 MHz, 3H), 1.19 (d, J=6.8 MHz, 3H), 1.05 (d, J=6.8 MHz, 3H).

### Step 2: Synthesizing Compound 1-7-2

Compound 7B (100 mg, 0.18 mmol) was added to dioxane (2 ml) and water (0.5 ml). K₃PO₄ (106 mg, 0.5 mmol) and 3-chloro-2-fluoro-6-methoxyphenylboronic acid (102 mg, 0.5 mmol) were added thereto. After the reaction solution was purged with nitrogen gas three times, a dichloromethane complex of Pd(dppf)Cl₂ (25 mg, 0.03 mmol) was added thereto. The resulting reaction solution was again purged with nitrogen gas three times and then stirred overnight at 100°C. After TLC showed that the reaction was complete, the reaction solution was concentrated under vacuum, and the resulting residue was purified by preparative silica gel plate (prepar-TLC) (developing solvent system: petroleum ether/ethyl acetate = 1 : 2 (V : V volume ratio)) to obtain Compound 1-7-2 (23 mg, light yellow solid).

MS m/z(ESI): 633[M+1].

¹H NMR (400 MHz, CDCl₃) 7.98 (s, 1H), 7.52-7.48 (m, 2H), 7.39-7.35 (m, 2H), 7.13-7.11 (m, 1H), 6.69-6.60 (m, 2H), 6.54 (s, 1H), 6.42-6.38 (m, 1H), 5.83-5.80 (m, 1H), 4.30-3.99 (m, 4H), 3.67 (s, 3H), 3.74-3.37 (m, 3H), 2.53-2.46 (m, 1H), 1.37 (d, J =6.4 MHz, 3H), 1.19 (d, J =6.8 MHz, 3H), 1.00 (d, J =6.8 MHz, 3H).

### Example 8: Preparation of Compound I-8-1 and Compound I-8-2

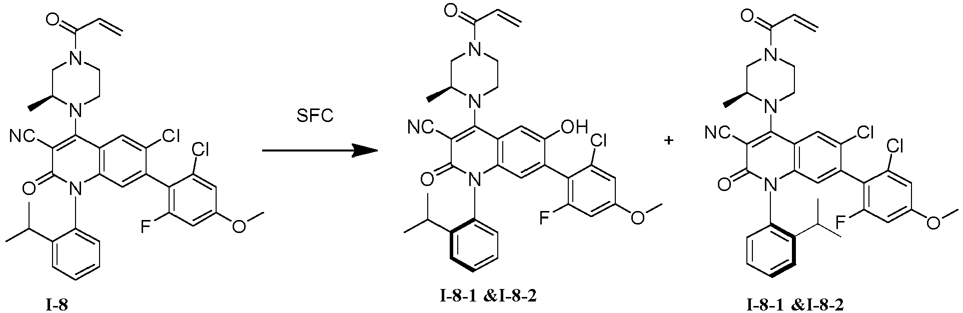

Synthesis of Compound 1-8-1:
Compound 6A (t_{R} = 2.745 min) and 2-chloro-6-fluoro-4-methoxyphenylboronic acid were used as the starting materials to obtain Compound 1-8-1 (refer to the synthesis of Compound 1-7-1).

MS m/z(ESI): 633[M+1].

¹H NMR (400 MHz, CDCl₃) 7.96 (s, 1H), 7.52-7.47 (m, 2H), 7.37-7.32 (m, 1H), 7.12-7.09 (m, 1H), 6.80-6.60 (m, 3H), 6.51-6.38 (m, 2H), 5.83-5.79 (m, 1H), 4.39-3.99 (m, 4H), 3.77 (s, 3H), 3.64-3.35 (m, 3H), 2.50-2.43 (m, 1H), 1.36 (d, J =6.0 MHz, 3H), 1.17 (d, J =6.8 MHz, 3H), 1.02 (d, J =6.8 MHz, 3H).

Synthesis of Compound 1-8-2:
Compound 6B (t_{R} = 4.203 min) and 2-chloro-6-fluoro-4-methoxyphenylboronic acid were used as the starting materials to obtain Compound 1-8-2 (refer to the synthesis of Compound 1-7-2).

MS m/z(ESI): 633[M+1].

¹H NMR (400 MHz, CDCl₃)7.97 (s, 1H), 7.52-7.48 (m, 2H), 7.38-7.34 (m, 1H), 7.12-7.10 (m, 1H), 6.80-6.63 (m, 3H), 6.52-6.38 (m, 2H), 5.83-5.80 (m, 1H), 4.42-3.75 (m, 5H), 3.64 (s, 3H), 3.56-3.36 (m, 2H), 2.52-2.47 (m, 1H), 1.36 (d, J =6.4 MHz, 3H), 1.19 (d, J =6.8 MHz, 3H), 1.01 (d, J =6.8 MHz, 3H).

Compound 6B (t_{R} = 4.203 min) and commercially available boronic acid or boronic acid ester were used as the starting materials to obtain Example Compounds 1-9 to 1-77 in Table 1 (refer to the synthesis of Compound 1-7-2).

**Table 1**

| **Examples** | **Structures** | **LCMS [M+H]⁺** | **NMR** |
|---|---|---|---|
| 1-9 | 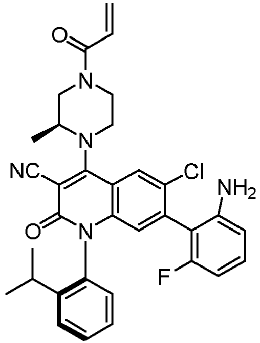 | 584.2 | HNMR: (400MHz, CDC13) 8.03 (s, 1H), 7.52-7.48 (m, 3H), 7.37-7.33 (m, 1H), 7.16-7.07 (m, 2H), 6.63-6.38 (m, 4H), 5.83-5.34 (m, 1H), 4.39-3.37 (m, 9H), 2.46-2.41 (m, 1H), 1.36 (d, J =6.0 MHz, 3H), 1.16 (d, J =6.4 MHz, 3H), 1.02 (d, J =6.8 MHz, 3H). |
| I-10 | 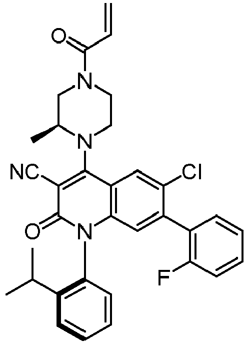 | 569.3 | HNMR: (400MHz, CDC13) 7.98 (s, 1H), 7.52-7.47 (m, 2H), 7.40-7.33 (m, 2H), 7.19-7.08 (m, 4H), 6.73-6.59 (m, 2H), 6.43-6.38 (m, 1H), 5.83-5.80 (m, 1H), 4.28-4.01 (m, 4H), 3.57-3.39 (m, 2H), 2.50-2.47 (m, 1H), 1.36 (d, J =6.4 MHz, 3H), 1.32-1.30 (m, 1H), 1.19 (d, J =6.8 MHz, 3H), 1.05 (d, J =6.8 MHz, 3H). |
| I-11 | 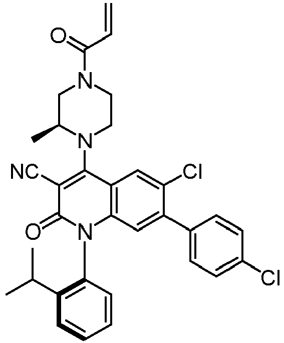 | 585.2 | HNMR: (400MHz, CDC13) 7.96 (s, 1H), 7.54-7.48 (m, 2H), 7.39-7.34 (m, 3H), 7.24-7.17 (m, 2H), 7.17-7.15 (m, 2H), 7.11-7.09 (M, 2H), 6.72-6.57 (m, 1H), 6.53 (s, 1H), 6.43-6.38 (m, 1H), 5.83-5.80 (m, 1H), 4.38-4.11 (m, 4H), 3.61-3.39 (m, 2H), 2.51-2.45 (m, 1H), 1.35 (d, J =6.0 MHz, 3H), 1.32-1.29 (m, 1H), 1.19 (d, J =6.8 MHz, 3H), 1.00 (d, J =6.8 MHz, 3H). |
| I-12 | 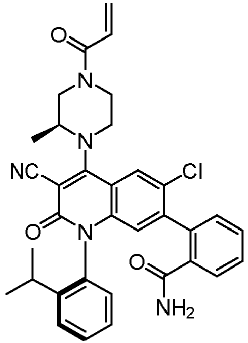 | 594.2 | HNMR: (400MHz, CDC13) 7.91 (s, 1H), 7.65-7.63 (m, 2H), 7.49-7.44 (m, 4H), 7.35-7.31 (m, 1H), 7.13-7.06 (m, 2H), 6.57-6.54 (m, 2H), 6.42-6.37 (m, 1H), 5.82-5.80 (m, 4H), 4.27-3.99 (m, 4H), 3.58-3.36 (m, 2H), 2.49 (m, 1H), 1.41-1.35 (m, 4H), 1.18 (m, 3H), 1.05 (m, 3H). |
| I-13 | 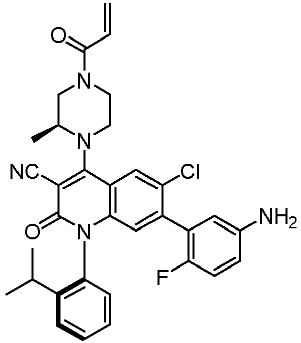 | 584.1 | HNMR: (400MHz, CDC13) 7.96 (s, 1H), 7.52-7.47 (m, 2H), 7.38-7.34 (m, 1H), 7.10 (d, J =7.6 MHz, 1H), 6.89 (t, J =8.8 MHz, 2H), 6.67-6.63 (m, 2H), 6.58 (s, 1H), 6.42-6.34 (m, 2H), 5.82-5.80 (m, 1H), 4.26-4.12 (m, 4H), 3.59-3.54 (m, 3H), 3.41-3.38 (m, 1H), 2.49-2.45 (m, 1H), 1.35 (d, J =6.0 MHz, 3H), 1.32-1.25 (m, 1H), 1.18 (d, J =6.8 MHz, 3H), 1.03 (d, J =6.8 MHz, 3H). |
| I-14 | 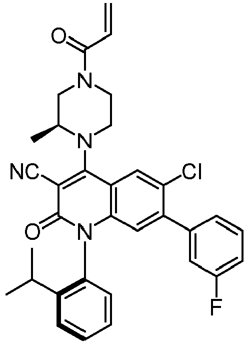 | 569.2 | HNMR: (400MHz, CDC13) 7.97 (s, 1H), 7.52-7.50 (m, 2H), 7.39-7.32 (m, 2H), 7.12-7.08 (m, 2H), 6.99-6.93 (m, 2H), 6.64 (brs, 1H), 6.56 (s, 1H), 6.43-6.38 (m, 1H), 5.83-5.80 (m, 1H), 4.38-4.01 (m, 4H), 3.58-3.38 (m, 2H), 2.49-2.51-2.44 (m, 1H), 1.36 (d, J =6.0 MHz, 3H), 1.26-1.25 (m, 1H), 1.19 (d, J =6.8 MHz, 3H), 1.05 (d, J =6.8 MHz, 3H). |
| I-15 | 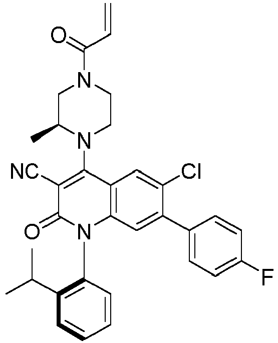 | 569.2 | HNMR: (400MHz, CDC13) 7.96 (s, 1H), 7.52-7.48 (m, 2H), 7.38-7.34 (m, 1H), 7.24-7.17 (m, 2H), 7.17-7.05 (m, 3H), 6.64-6.63 (m, 1H), 6.54 (s, 1H), 6.43-6.38 (m, 1H), 5.83-5.80 (m, 1H), 4.38-4.08 (m, 4H), 3.60-3.39 (m, 2H), 2.52-2.45 (m, 1H), 1.36 (d, J =6.4 MHz, 3H), 1.30-1.26 (m, 1H), 1.20 (d, J =6.8 MHz, 3H), 1.02 (d, J =6.8 MHz, 3H). |
| I-16 | 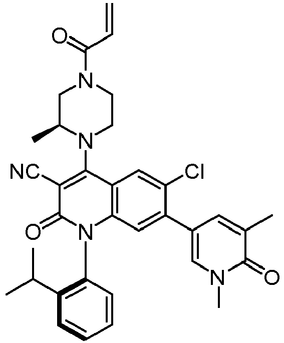 | 596.3 | HNMR: (400MHz, CDC13) 7.93 (s, 1H), 7.55-7.54 (m, 2H), 7.42-7.38 (m, 1H), 7.12-7.07 (m, 3H), 6.63 (m, 1H), 6.48 (s, 1H), 6.42-6.38 (m, 2H), 5.83-5.81 (m, 1H), 4.38-4.01 (m, 4H), 3.52 (s, 3H), 3.41-3.83 (m, 1H), 2.51-2.43 (m, 1H), 2.13 (s, 3H), 1.35-1.30 (m, 4H), 1.20 (d, J =6.8 MHz, 3H), 1.05 (d, J =6.8 MHz, 3H). |
| I-17 | 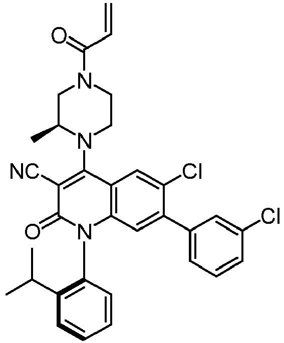 | 585.2 | HNMR: (400MHz, CDC13) 7.96 (s, 1H), 7.53-7.49 (m, 2H), 7.39-7.30 (m, 3H), 7.22-7.21 (m, 1H), 7.09 (t, J =8.4 MHz, 2H), 6.65-6.61 (m, 1H), 6.54 (s, 1H), 6.43-6.38 (m, 1H), 5.83-5.80 (m, 1H), 4.38-3.99 (m, 4H), 3.57-3.39 (m, 2H), 2.50-2.51-2.45 (m, 1H), 1.36 (d, J =6.0 MHz, 3H), 1.32-1.30 (m, 1H), 1.19 (d, J =6.8 MHz, 3H), 1.05 (d, J =6.8 MHz, 3H). |
| I-18 | 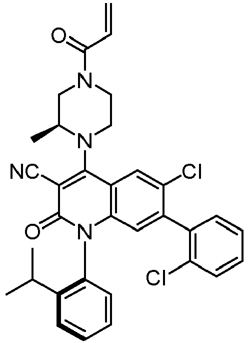 | 585.2 | HNMR: (400MHz, CDC13) 7.97-7.96 (m, 1H), 7.50-7.42 (m, 3H), 7.38-7.28 (m, 3H), 7.15-7.02 (m, 2H), 6.71-6.63 (m, 1H), 6.58 (s, 1H), 6.43-6.38 (m, 1H), 5.83-5.80 (m, 1H), 4.28-4.00 (m, 4H), 3.57-3.38 (m, 3H), 2.50-2.46 (m, 1H), 1.37 (d, J =6.0 MHz, 3H), 1.20-1.16 (m, 3H), 1.06-1.03 (m, 3H). |
| I-19 | 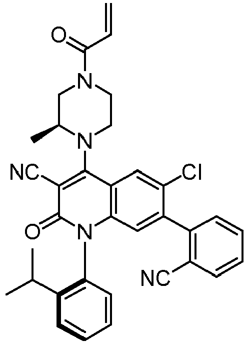 | 576.1 | HNMR: (400MHz, CDC13) 8.01 (brs, 1H), 7.83-7.81 (m, 1H), 7.74-7.72 (m, 1H), 7.65-7.61 (m, 1H), 7.51-7.36 (m, 3H), 7.24-7.12 (m, 2H), 6.66-6.62 (m, 1H), 6.58 (s, 1H), 6.43-6.38 (m, 1H), 5.83-5.80 (m, 1H), 4.28-3.38 (m, 7H), 2.51-2.46 (m, 1H), 1.37 (d, J =6.4 MHz, 3H), 1.20-1.18 (m, 3H), 1.04-1.03 (m, 3H). |
| I-20 | 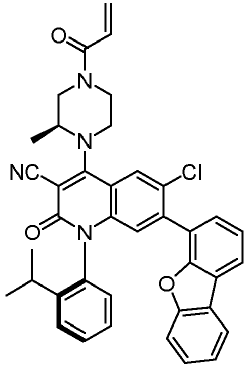 | 641.2 | HNMR: (400MHz, CDC13) 8.06 (brs, 1H), 8.00-7.95 (m, 2H), 7.49-7.28 (m, 8H), 7.13-7.11 (m, 1H), 6.82 (s, 1H), 6.65 (m, 1H), 6.44-6.39 (m, 1H), 5.84-5.81 (m, 1H), 4.37-4.11 (m, 4H), 3.60-3.42 (m, 3H), 2.62-2.55 (m, 1H), 1.39 (d, J =6.0 MHz, 3H), 1.22 (d, J =6.4 MHz, 3H), 1.15 (d, J =6.4 MHz, 3H). |
| I-21 | 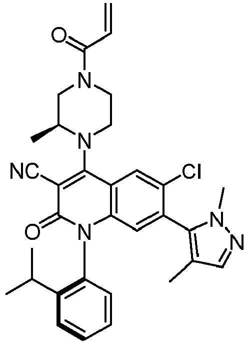 | 569.3 | HNMR: (400MHz, CDC13) 7.97 (brs, 1H), 7.53-7.52 (m, 2H), 7.40-7.36 (m, 1H), 7.11-7.09 (m, 1H), 6.66-6.57 (m, 1H), 6.43-6.38 (m, 1H), 5.93 (s, 1H), 5.84-5.81 (m, 1H), 4.40-3.97 (m, 4H), 3.56 (s, 1H), 3.45-3.37 (m, 3H), 2.46-2.42 (m, 1H), 2.25 (s, 3H), 1.36 (d, J =6.0 MHz, 3H), 1.19 (d, J =6.4 MHz, 3H), 1.01 (d, J =6.4 MHz, 3H). |
| I-22 | 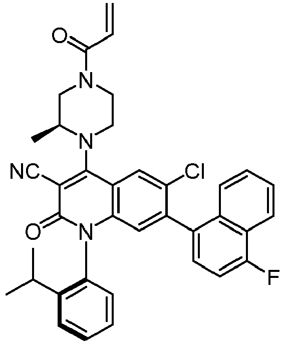 | 619.1 | HNMR: (400MHz, CDC13) 8.17-8.14 (m, 1H), 8.03-8.02 (m, 1H), 7.59-7.29 (m, 6H), 7.16-7.04 (m, 3H), 6.66-6.62 (m, 2H), 6.43-6.39 (m, 1H), 5.83-5.81 (m, 1H), 4.34-4.01 (m, 4H), 3.59-3.41 (m, 3H), 2.52-2.49 (m, 1H), 1.40 (d, J =6.0 MHz, 3H), 1.21-1.14 (m, 3H), 1.11-0.86 (m, 3H). |
| I-23 | 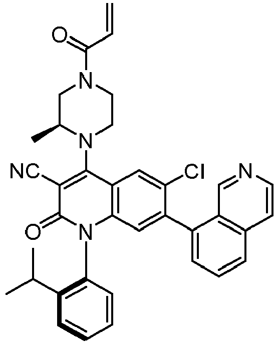 | 602.3 | HNMR: (400MHz, CDC13) 8.81-8.70 (m, 1H), 8.58-8.54 (m, 1H), 8.06-8.04 (m, 1H), 7.89-7.87 (m, 1H), 7.72-7.67 (m, 2H), 7.49-7.40 (m, 2H), 7.24-7.21 (m, 3H), 6.67-6.66 (m, 2H), 6.43-6.39 (m, 1H), 5.84-5.81 (m, 1H), 4.36-3.77 (m, 4H), 3.49-3.44 (m, 3H), 2.52-2.49 (m, 1H), 1.41 (d, J =6.0 MHz, 3H), 1.22-0.86 (m, 6H). |
| I-24 | 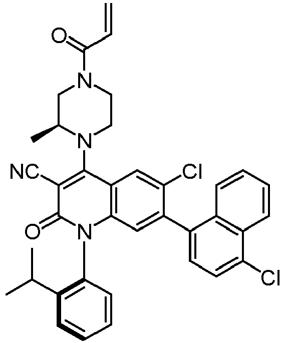 | 635.2 | HNMR: (400MHz, CDC13) 8.35-8.32 (m, 1H), 8.02 (m, 1H), 7.65-7.39 (m, 6H), 7.26-7.03 (m, 3H), 6.66-6.62 (m, 2H), 6.42-6.38 (m, 1H), 5.86-5.83 (m, 1H), 4.35-4.04 (m, 4H), 3.45-3.41 (m, 3H), 2.52-2.49 (m, 1H), 1.41-1.40 (m, 3H), 1.23-0.86 (m, 6H). |
| I-25 | 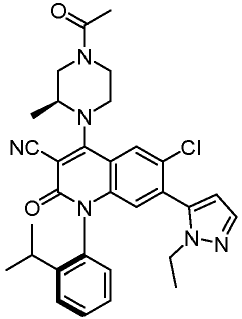 | 557.2 | HNMR: (400MHz, CDC13) 7.98 (m, 1H), 7.52-7.51 (m, 3H), 7.40-7.36 (m, 1H), 7.11-7.09 (m, 1H), 6.72-6.63 (m, 1H), 6.59 (s, 1H), 6.43-6.39 (m, 1H), 6.17-6.12 (m, 1H), 5.84-5.81 (m, 1H), 4.40-3.85 (m, 6H), 3.50-3.38 (m, 3H), 2.47-2.44 (m, 1H), 1.37-1.36 (m, 3H), 1.25-1.16 (m, 6H), 1.01 (d, J =7.2 MHz, 3H). |
| I-26 | 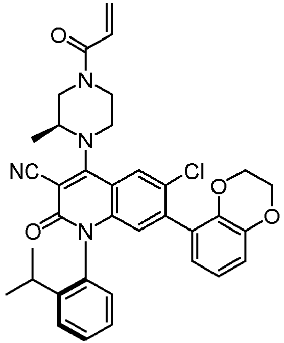 | 609.1 | HNMR: (400MHz, CDC13) 7.94 (m, 1H), 7.49-7.46 (m, 2H), 7.36-7.33 (m, 1H), 7.11-7.10 (m, 1H), 6.92-6.81 (m, 2H), 6.63-6.53 (m, 3H), 6.42-6.37 (m, 1H), 5.82-5.80 (m, 1H), 4.37-4.09 (m, 8H), 3.50-3.38 (m, 3H), 2.51-2.47 (m, 1H), 1.35-1.34 (m, 3H), 1.18 (d, J =6.4 MHz, 3H), 1.01 (d, J =6.4 MHz, 3H). |
| I-27 | 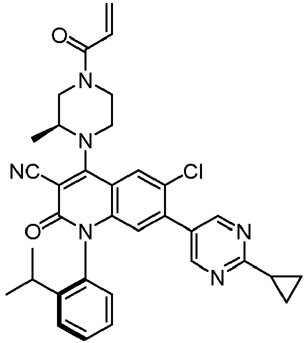 | 593.3 | HNMR: (400MHz, CDC13) 8.48 (s, 2H), 7.99 (m, 1H), 7.49-7.34 (m, 2H), 7.10-7.08 (m, 2H), 6.70-6.54 (m, 2H), 6.43-6.38 (m, 1H), 5.83-5.81 (m, 1H), 4.40-4.00 (m, 5H), 3.49-3.39 (m, 2H), 2.49-2.45 (m, 1H), 2.29-2.22 (m, 1H), 1.36-1.35 (m, 3H), 1.21-1.11 (m, 7H), 1.04 (d, J =6.8 MHz, 3H). |
| I-28 | 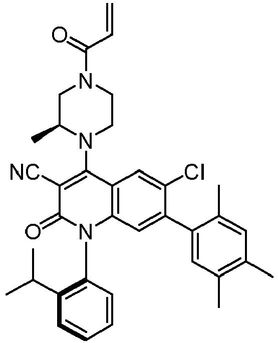 | 593.2 | HNMR: (400MHz, CDC13) 7.94-7.91 (m, 1H), 7.45-7.40 (m, 2H), 7.34-7.25 (m, 1H), 7.19-7.05 (m, 1H), 7.00-6.97 (m, 1H), 6.72-6.53 (m, 3H), 6.42-6.36 (m, 1H), 5.82-5.79 (m, 1H), 4.37-4.00 (m, 4H), 3.72-3.36 (m, 3H), 2.49-2.43 (m, 1H), 2.24-2.18 (m, 6H), 2.04-1.89 (m, 3H), 1.45-1.40 (m, 3H), 1.17 (m, 3H), 1.09-0.96 (m, 3H). |
| I-29 | 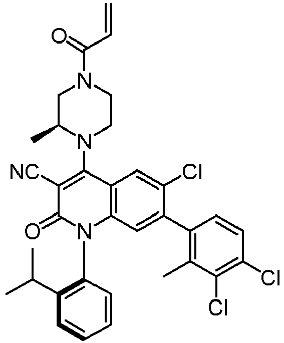 | 633.3 | HNMR: (400MHz, CDC13) 7.96 (m, 1H), 7.52-7.48 (m, 2H), 7.37-7.30 (m, 2H), 7.12-7.09 (m, 1H), 6.83-6.74 (m, 1H), 6.65-6.63 (m, 1H), 6.45-6.38 (m, 2H), 5.83-5.81 (m, 1H), 4.40-4.01 (m, 5H), 3.58-3.38 (m, 2H), 2.48-2.43 (m, 1H), 2.20-2.02 (m, 3H), 1.37 (d, J =6.4 MHz, 3H), 1.20-1.17 (m, 3H), 1.05-0.95 (m, 3H). |
| I-30 | 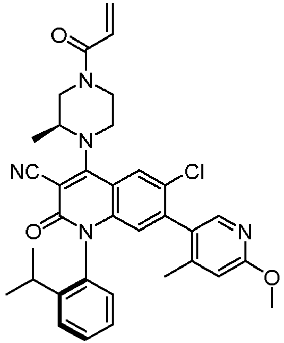 | 596.2 | HNMR: (400MHz, CDC13) 7.96 (m, 1H), 7.73-7.69 (m, 1H), 7.50-7.48 (m, 2H), 7.36-7.35 (m, 1H), 7.11 (m, 1H), 6.62 (m, 2H), 6.49-6.38 (m, 2H), 5.83-5.80 (m, 1H), 4.37-4.02 (m, 4H), 3.92 (s, 3H), 3.71-3.38 (m, 3H), 2.48-2.43 (m, 1H), 2.06-1.98 (m, 3H), 1.37 (d, J =6.0 MHz, 3H), 1.18 (d, J =6.4 MHz, 3H), 1.08-0.96 (m, 3H). |
| I-31 | 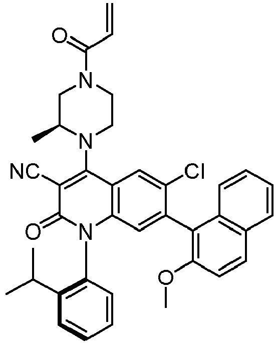 | 631.3 | HNMR: (400MHz, CDC13) 8.05 (s, 1H), 7.92-7.90 (m, 1H), 7.82-7.80 (m, 1H), 7.44-7.29 (m, 5H), 7.26-7.24 (m, 1H), 7.11-7.09 (m, 2H), 6.65-6.61 (m, 2H), 6.43-6.38 (m, 2H), 5.83-5.80 (m, 1H), 4.37-4.02 (m, 4H), 3.78 (s, 3H), 3.71-3.40 (m, 3H), 2.54-2.51 (m, 1H), 1.41 (d, J =6.0 MHz, 3H), 1.18 (d, J =6.8 MHz, 3H), 1.03 (d, J =6.8 MHz, 3H). |
| I-32 | 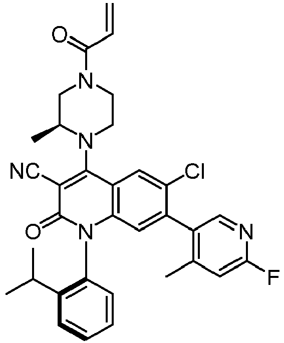 | 584.2 | HNMR: (400MHz, CDC13) 7.98 (s, 1H), 7.81-7.75 (m, 1H), 7.51-7.48 (m, 2H), 7.37-7.34 (m, 1H), 7.14-7.09 (m, 1H), 6.82 (s, 1H), 6.64-6.61 (m, 1H), 6.48-6.38 (m, 2H), 5.83-5.81 (m, 1H), 4.37-4.00 (m, 4H), 3.58-3.39 (m, 3H), 2.49-2.43 (m, 1H), 2.15-2.05 (m, 3H), 1.37 (d, J =6.4 MHz, 3H), 1.18 (d, J =6.8 MHz, 3H), 1.07-0.94 (m, 3H). |
| I-34 | 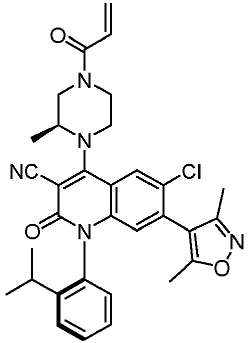 | 570.3 | HNMR: (400MHz, CDC13) 7.95 (s, 1H), 7.54-7.37 (m, 3H), 7.10-7.07 (m, 1H), 6.70-6.55 (m, 2H), 6.43-6.38 (m, 1H), 5.83-5.80 (m, 1H), 4.38-4.23 (m, 4H), 3.58-3.36 (m, 3H), 2.49-2.45 (m, 1H), 1.72-1.71 (m, 3H), 1.59-1.58 (m, 3H), 1.37-1.36 (m, 3H), 1.20-1.17 (m, 3H), 1.01-0.98 (m, 3H). |
| I-35 | 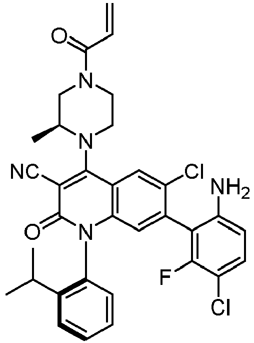 | 618.2 | HNMR: (400MHz, CDC13) 8.04 (s, 1H), 7.52-7.48 (m, 2H), 7.41-7.37 (m, 1H), 7.19-7.11 (m, 2H), 6.69-6.60 (m, 2H), 6.46-6.38 (m, 2H), 5.83-5.80 (m, 1H), 4.29-4.07 (m, 4H), 3.45-3.38 (m, 3H), 2.47-2.43 (m, 1H), 1.37 (d, J =6.4 MHz, 3H), 1.18 (d, J =6.8 MHz, 3H), 1.01 (d, J =6.8 MHz, 3H). |
| I-36 | 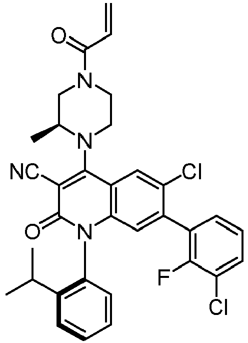 | 603.2 | HNMR: (400MHz, CDC13) 7.99 (s, 1H), 7.54-7.34 (m, 4H), 7.17-7.09 (m, 2H), 7.00-6.97 (m, 1H), 6.66-6.60 (m, 2H), 6.43-6.38 (m, 1H), 5.83-5.80 (m, 1H), 4.40-3.99 (m, 4H), 3.77-3.39 (m, 3H), 2.51-2.44 (m, 1H), 1.36 (d, J =6.4 MHz, 3H), 1.19 (d, J =6.8 MHz, 3H), 1.04 (d, J =6.8 MHz, 3H). |
| I-37 | 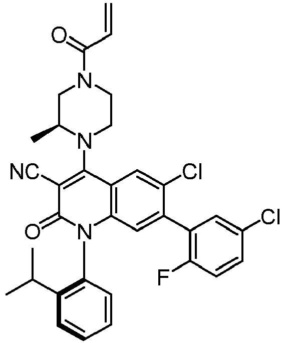 | 603.2 | HNMR: (400MHz, CDC13) 7.98-7.95 (m, 1H), 7.54-7.50 (m, 2H), 7.37-7.33 (m, 2H), 7.12-7.04 (m, 3H), 6.68-6.56 (m, 2H), 6.43-6.38 (m, 1H), 5.83-5.79 (m, 1H), 4.40-3.96 (m, 4H), 3.80-3.39 (m, 3H), 2.50-2.45 (m, 1H), 1.36 (d, J =6.0 MHz, 3H), 1.18 (d, J =6.4 MHz, 3H), 1.04 (d, J =6.4 MHz, 3H). |
| I-38 | 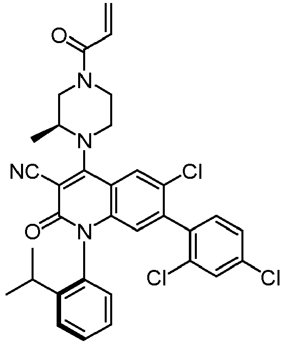 | 619.2 | HNMR: (400MHz, CDC13) 7.97 (s, 1H), 7.55-7.49 (m, 3H), 7.38-7.35 (m, 1H), 7.14-6.96 (m, 2H), 6.66-6.59 (m, 1H), 6.49-6.38 (m, 2H), 5.83-5.80 (m, 1H), 4.39-4.00 (m, 4H), 3.59-3.38 (m, 3H), 2.48-2.43 (m, 1H), 1.37 (d, J =6.0 MHz, 3H), 1.19-1.16 (m, 3H), 1.03-1.00 (m, 3H). |
| I-39 | 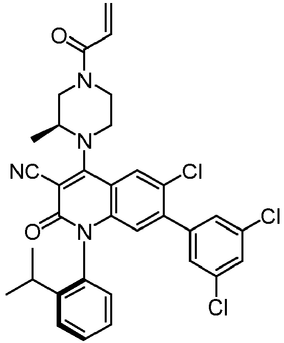 | 619.2 | HNMR: (400MHz, CDC13) 7.96 (s, 1H), 7.61-7.50 (m, 2H), 7.41-7.36 (m, 2H), 7.22-7.08 (m, 3H), 6.66-6.61 (m, 1H), 6.51-6.39 (m, 2H), 5.83-5.81 (m, 1H), 4.39-4.00 (m, 4H), 3.76-3.39 (m, 3H), 2.50-2.43 (m, 1H), 1.35 (d, J =6.0 MHz, 3H), 1.19 (d, J =6.8 MHz, 3H), 1.05 (d, J =6.8 MHz, 3H). |
| I-40 | 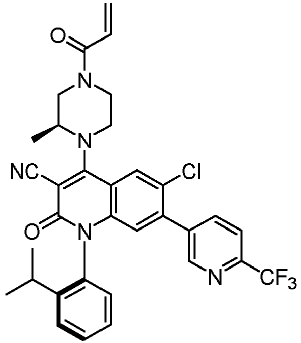 | 620.1 | HNMR: (400MHz, CDC13) 8.56 (s, 1H), 8.02 (s, 1H), 7.85-7.82 (m, 1H), 7.75-7.73 (m, 1H), 7.55-7.50 (m, 2H), 7.39-7.35 (m, 1H), 7.11-7.09 (m, 1H), 6.70-6.58 (m, 2H), 6.43-6.39 (m, 1H), 5.84-5.81 (m, 1H), 4.41-3.96 (m, 4H), 3.81-3.40 (m, 3H), 2.51-2.45 (m, 1H), 1.36 (d, J =6.4 MHz, 3H), 1.20 (d, J =6.8 MHz, 3H), 1.04 (d, J =6.8 MHz, 3H). |
| I-41 | 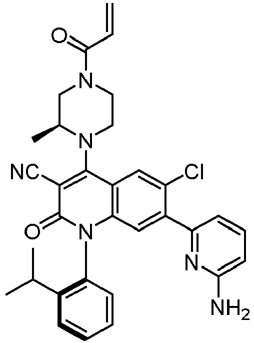 | 567.3 | HNMR: (400MHz, CDC13) 7.95-7.89 (m, 2H), 7.53-7.44 (m, 3H), 7.38-7.34 (m, 1H), 7.11-7.09 (m, 1H), 6.66-6.59 (m, 1H), 6.53-6.38 (m, 3H), 5.83-5.80 (m, 1H), 4.64 (brs, 2H), 4.38-3.95 (m, 4H), 3.75-3.38 (m, 3H), 2.52-2.45 (m, 1H), 1.34 (d, J =6.4 MHz, 3H), 1.19 (d, J =6.8 MHz, 3H), 1.04 (d, J =6.8 MHz, 3H). |
| I-42 | 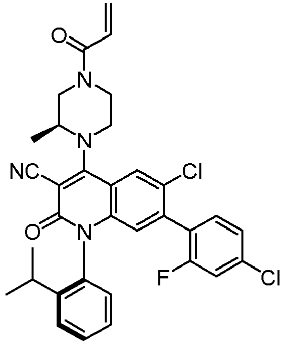 | 603.2 | HNMR: (400MHz, CDC13) 7.97-7.96 (m, 1H), 7.80-7.76 (m, 1H), 7.55-7.47 (m, 2H), 7.38-7.34 (m, 1H), 7.14-7.06 (m, 3H), 6.66-6.59 (m, 1H), 6.54 (s, 1H), 6.43-6.38 (m, 1H), 5.83-5.80 (m, 1H), 4.40-3.98 (m, 4H), 3.61-3.38 (m, 3H), 2.50-2.43 (m, 1H), 1.36 (d, J =6.4 MHz, 3H), 1.18 (d, J =6.8 MHz, 3H), 1.03 (d, J =6.8 MHz, 3H). |
| I-43 | 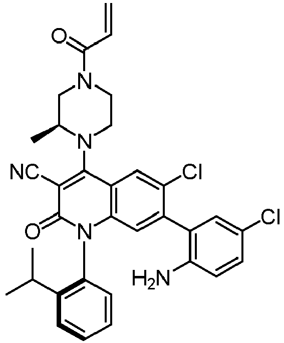 | 600.1 | HNMR: (400MHz, CDC13) 7.99 (m, 1H), 7.52-7.49 (m, 2H), 7.37-7.36 (m, 1H), 7.14-7.08 (m, 2H), 6.85-6.77 (m, 1H), 6.67-6.59 (m, 3H), 6.43-6.38 (m, 1H), 5.83-5.80 (m, 1H), 4.26-3.98 (m, 4H), 3.58-3.39 (m, 3H), 2.46-2.43 (m, 1H), 1.36 (d, J =6.0 MHz, 3H), 1.20-1.67 (m, 3H), 1.10-0.98 (m, 3H). |
| I-44 | 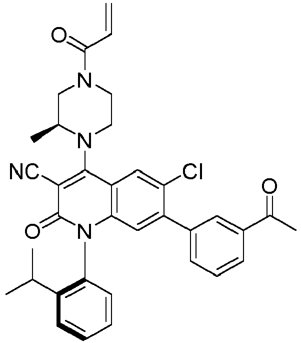 | 593.2 | HNMR: (400MHz, CDC13) 7.98-7.96 (m, 2H), 7.83 (m, 1H), 7.53-7.47 (m, 3H), 7.45-7.34 (m, 2H), 7.12-7.10 (m, 1H), 6.67-6.58 (m, 2H), 6.43-6.39 (m, 1H), 5.83-5.81 (m, 1H), 4.39-4.02 (m, 4H), 3.73-3.40 (m, 3H), 2.57 (s, 3H), 2.54-2.49 (m, 1H), 1.36 (d, J =6.0 MHz, 3H), 1.20 (d, J =6.8 MHz, 3H), 1.08 (d, J =6.8 MHz, 3H). |
| I-45 | 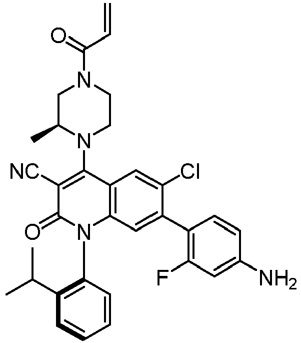 | 584.2 | HNMR: (400MHz, CDC13) 7.94 (s, 1H), 7.52-7.47 (m, 2H), 7.37-7.33 (m, 1H), 7.11-7.09 (m, 1H), 6.89-6.85 (m, 1H), 6.67-6.61 (m, 2H), 6.44-6.36 (m, 3H), 5.82-5.80 (m, 1H), 4.37-3.96 (m, 4H), 3.88 (s, 2H), 3.75-3.53 (m, 3H), 2.52-2.45 (m, 1H), 1.34 (d, J =6.4 MHz, 3H), 1.18 (d, J =6.8 MHz, 3H), 1.04 (d, J =6.8 MHz, 3H). |
| I-46 | 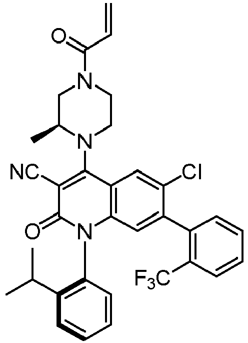 | 619.2 | HNMR: (400MHz, CDC13) 7.96-7.95 (m, 1H), 7.72-7.71 (m, 1H), 7.56-7.45 (m, 4H), 7.36-7.29 (m, 1H), 7.14-7.08 (m, 2H), 6.68-6.54 (m, 2H), 6.42-6.38 (m, 1H), 5.83-5.80 (m, 1H), 4.29-4.10 (m, 4H), 3.65-3.39 (m, 3H), 2.50-2.46 (m, 1H), 1.38 (d, J =6.4 MHz, 3H), 1.20-1.17 (m, 3H), 1.04-0.95 (m, 3H). |
| I-47 | 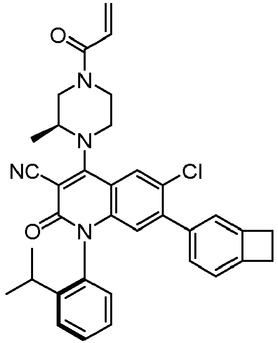 | 577.2 | HNMR: (400MHz, CDC13) 7.95 (s, 1H), 7.52-7.46 (m, 2H), 7.36-7.32 (m, 1H), 7.11-7.01 (m, 3H), 6.92 (m, 1H), 6.63-6.57 (m, 2H), 6.42-6.38 (m, 1H), 5.83-5.80 (m, 1H), 4.38-3.17 (m, 12H), 2.51-2.48 (m, 1H), 1.361.32 (m, 3H), 1.20-1.13 (m, 3H), 1.07-1.05 (m, 3H). |
| I-48 | 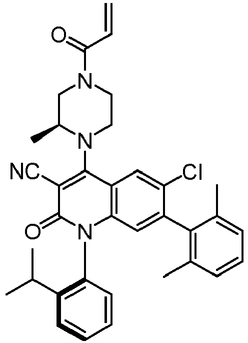 | 579.1 | HNMR: (400MHz, CDC13) 7.99 (s, 1H), 7.55-7.54 (m, 2H), 7.37-7.31 (m, 1H), 7.21-7.18 (m, 1H), 7.11-7.07 (m, 3H), 6.65-6.57 (m, 1H), 6.50 (s, 1H), 6.42-6.38 (m, 1H), 5.83-5.80 (m, 1H), 4.37-3.39 (m, 7H), 2.52-2.47 (m, 1H), 1.91 (s, 6H), 1.38 (d, J =6.0 MHz, 3H), 1.18 (d, J =6.8 MHz, 3H), 1.00 (d, J =6.8 MHz, 3H). |
| I-49 | 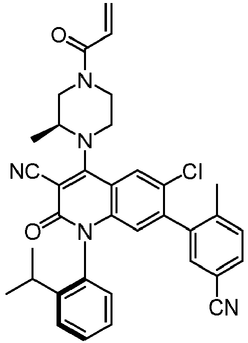 | 590.2 | HNMR: (400MHz, CDC13) 7.98 (s, 1H), 7.59-7.33 (m, 6H), 7.18-7.09 (m, 1H), 6.61-6.43 (m, 1H), 6.45-6.39 (m, 2H), 5.83-5.81 (m, 1H), 4.40-4.02 (m, 4H), 3.71-3.39 (m, 3H), 2.48-2.45 (m, 1H), 2.13-2.05 (m, 3H), 1.37 (d, J =5.6 MHz, 3H), 1.19 (t, J =6.4 MHz, 3H), 1.07-0.95 (m, 3H). |
| I-50 | 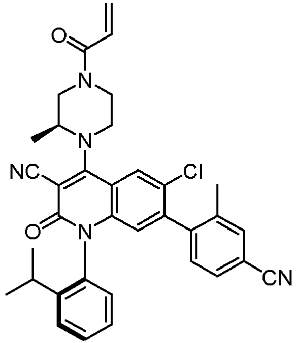 | 590.2 | HNMR: (400MHz, CDC13) 7.98 (s, 1H), 7.53-7.49 (m, 4H), 7.37-7.36 (m, 1H), 7.16-7.00 (m, 2H), 6.56-6.64 (m, 1H), 6.44-6.39 (m, 2H), 5.84-5.81 (m, 1H), 4.44-4.02 (m, 4H), 3.80-3.39 (m, 3H), 2.48-2.45 (m, 1H), 2.11-2.04 (m, 3H), 1.38 (m, 3H), 1.20-1.19 (m, 3H), 1.05-0.88 (m, 3H). |
| I-51 | 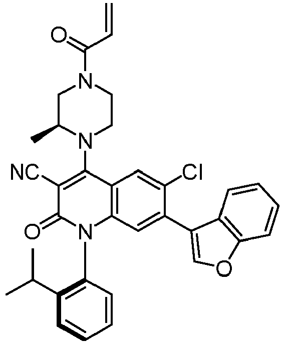 | 591.2 | HNMR: (400MHz, CDC13) 8.02 (s, 1H), 7.88 (s, 1H), 7.54-7.48 (m, 3H), 7.41-7.30 (m, 2H), 7.23-7.16 (m, 3H), 6.85 (s, 1H), 6.65 (m, 1H), 6.43-6.39 (m, 1H), 5.83-5.81 (m, 1H), 4.37-4.02 (m, 4H), 3.79-3.41 (m, 3H), 2.56-2.49 (m, 1H), 1.37 (d, J =5.6 MHz, 3H), 1.21 (d, J =6.4 MHz, 3H), 1.02 (d, J =6.4 MHz, 3H). |
| I-52 | 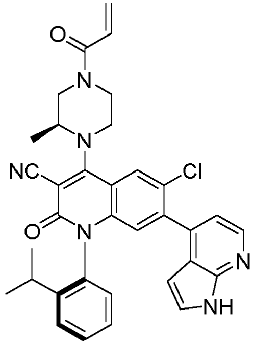 | 591.3 | HNMR: (400MHz, CDC13) 9.38 (s, 1H), 8.32 (d, J =4.8 MHz, 1H), 8.03 (s, 1H), 7.49-7.42 (m, 2H), 7.34-7.31 (m, 2H), 7.12-7.10 (m, 1H), 6.91 (d, J =4.8 MHz, 1H), 6.70 (s, 1H), 6.70-6.64 (m, 1H), 6.43-6.39 (m, 1H), 6.15 (brs, 1H), 5.84-5.81 (m, 1H), 4.39-4.03 (m, 4H), 3.71-3.42 (m, 3H), 2.54-2.49 (m, 1H), 1.38 (d, J =6.0 MHz, 3H), 1.19 (d, J =6.4 MHz, 3H), 1.04 (d, J =6.4 MHz, 3H). |
| I-53 | 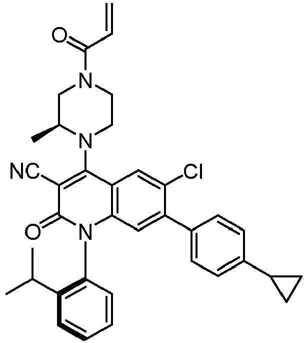 | 591.2 | HNMR: (400MHz, CDC13) 7.94 (s, 1H), 7.52-7.46 (m, 2H), 7.36-7.32 (m, 1H), 7.14-7.06 (m, 5H), 6.66-6.60 (m, 1H), 6.57 (s, 1H), 6.42-6.38 (m, 1H), 6.15 (brs, 1H), 5.82-5.80 (m, 1H), 4.37-4.01 (m, 4H), 3.76-3.38 (m, 3H), 2.53-2.46 (m, 1H), 1.93-1.86 (m, 1H), 1.35 (d, J =5.6 MHz, 3H), 1.19 (d, J =6.8 MHz, 3H), 1.05 (d, J =6.8 MHz, 3H), 1.02-0.98 (m, 2H), 0.73-0.69 (m, 2H). |
| I-54 | 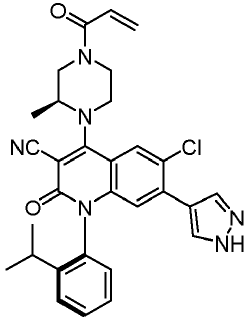 | 541.3 | HNMR: (400MHz, CDC13) 7.93 (s, 1H), 7.79-7.77 (m, 1H), 7.56-7.55 (m, 2H), 7.42-7.35 (m, 2H), 7.14-7.12 (m, 1H), 6.68 (m, 2H), 6.43-6.38 (m, 1H), 5.83-5.80 (m, 1H), 4.25-4.12 (m, 4H), 3.76-3.38 (m, 3H), 2.53-2.46 (m, 1H), 1.33-1.31 (m, 3H), 1.19 (d, J =6.8 MHz, 3H), 1.02 (d, J =6.8 MHz, 3H). |
| I-55 | 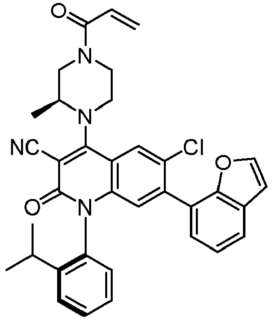 | 591.2 | HNMR: (400MHz, CDC13) 8.03 (s, 1H), 7.68-7.63 (m, 1H), 7.54-7.42 (m, 4H), 7.33-7.29 (m, 1H), 7.12-7.09 (m, 2H), 6.81-6.77 (m, 2H), 6.67-6.62 (m, 1H), 6.43-6.38 (m, 1H), 5.83-5.80 (m, 1H), 4.37-4.01 (m, 4H), 3.59-3.40 (m, 3H), 2.58-2.51 (m, 1H), 1.37 (d, J =6.0 MHz, 3H), 1.20 (d, J =6.8 MHz, 3H), 1.11 (d, J =6.8 MHz, 3H). |
| I-56 | 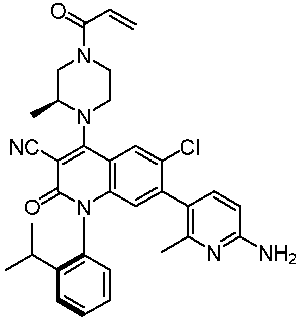 | 581.3 | HNMR: (400MHz, CDC13) 7.96 (s, 1H), 7.84 (s, 1H), 7.51-7.35 (m, 4H), 7.12-7.10 (m, 1H), 6.66-6.58 (m, 1H), 6.51-6.38 (m, 2H), 5.83-5.80 (m, 1H), 5.07 (s, 2H), 4.40-3.36 (m, 7H), 2.47-2.43 (m, 1H), 2.10-2.03 (m, 3H), 1.36 (d, J =5.6 MHz, 3H), 1.18 (d, J =6.8 MHz, 3H), 1.00 (m, 3H). |
| I-57 | 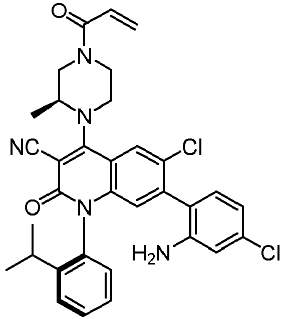 | 600.2 | HNMR: (400MHz, CDC13) 7.98 (m, 1H), 7.50-7.46 (m, 2H), 7.36-7.34 (m, 1H), 7.10-7.08 (m, 1H), 6.80-6.57 (m, 1H), 6.43-6.38 (m, 1H), 5.83-5.80 (m, 1H), 4.37-3.99 (m, 4H), 3.71-3.37 (m, 5H), 2.45-2.42 (m, 1H), 1.35 (d, J =6.0 MHz, 3H), 1.18 (d, J =6.8 MHz, 3H), 1.08-0.99 (m, 3H). |
| I-58 | 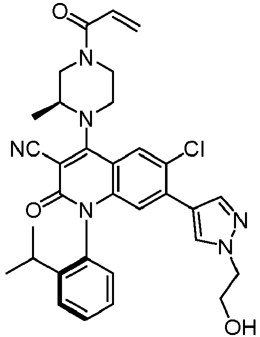 | 585.2 | HNMR: (400MHz, CDC13) 7.92-7.91 (m, 1H), 7.83 (s, 1H), 7.59-7.55 (m, 2H), 7.44-7.38 (m, 2H), 7.14-7.12 (m, 1H), 6.66 (m, 2H), 6.42-6.38 (m, 1H), 5.83-5.80 (m, 1H), 4.38-3.99 (m, 9H), 3.71-3.37 (m, 2H), 2.71 (t, J =6.0 MHz, 1H), 2.50-2.46 (m, 1H), 1.34 (d, J =6.0 MHz, 3H), 1.19 (d, J =6.8 MHz, 3H), 1.02 (d, J =6.8 MHz, 3H). |
| I-59 | 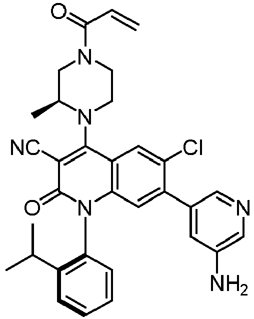 | 567.2 | HNMR: (400MHz, CDC13) 8.08 (m, 1H), 7.97 (s, 1H), 7.81 (m, 1H), 7.56-7.52 (m, 2H), 7.38-7.31 (m, 1H), 7.11-7.09 (m, 1H), 6.88 (s, 1H), 6.70-6.63 (m, 2H), 6.43-6.38 (m, 1H), 5.83-5.80 (m, 1H), 4.42-3.99 (m, 4H), 3.83-3.39 (m, 5H), 2.51-2.44 (m, 1H), 1.36-1.35 (m, 3H), 1.19 (d, J =6.8 MHz, 3H), 1.04 (d, J =6.8 MHz, 3H). |
| I-60 | 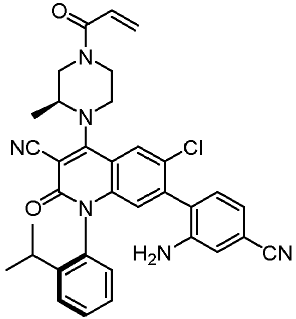 | 591.3 | HNMR: (400MHz, CDC13) 8.08-7.89 (m, 2H), 7.51-7.38 (m, 2H), 7.13-6.96 (m, 4H), 6.55 (m, 2H), 6.43-6.38 (m, 1H), 5.84-5.81 (m, 1H), 4.43-4.14 (m, 6H), 3.62-3.38 (m, 3H), 2.51-2.45 (m, 1H), 1.41-1.37 (m, 3H), 1.16-1.14 (m, 3H), 1.06-0.99 (m, 3H). |
| I-61 | 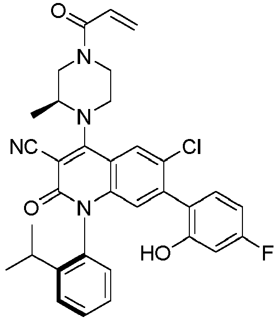 | 585.2 | HNMR: (400MHz, CDC13) 7.95 (m, 1H), 7.51-7.35 (m, 2H), 7.11-7.05 (m, 2H), 6.91-6.89 (m, 1H), 6.70-6.54 (m, 3H), 6.44-6.38 (m, 1H), 5.83-5.81 (m, 1H), 5.66 (m, 1H), 4.37-4.01 (m, 4H), 3.72-3.40 (m, 3H), 2.49-2.45 (m, 1H), 1.36-1.32 (m, 3H), 1.20-1.14 (m, 3H), 1.05-1.03 (m, 3H). |
| I-62 | 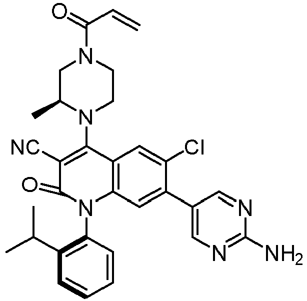 | 568.5 | HNMR: (400MHz, CDC13) 8.24 (s, 2H), 7.97 (s, 1H), 7.53-7.50 (m, 2H), 7.39-7.35 (m, 1H), 7.11-7.09 (m, 1H), 6.66-6.63 (m, 1H), 6.51 (s, 1H), 6.43-6.38 (m, 1H), 5.83-5.80 (m, 1H), 5.18 (s, 2H), 4.25-3.99 (m, 4H), 3.72-3.39 (m, 3H), 2.49-2.45 (m, 1H), 1.35 (d, J =6.4 MHz, 3H), 1.19 (d, J =6.8 MHz, 3H), 1.03 (d, J =6.8 MHz, 3H). |
| I-63 | 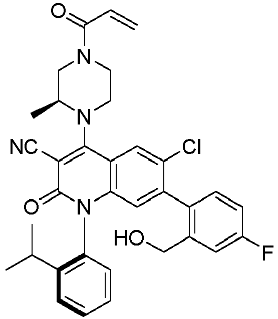 | 599.2 | HNMR: (400MHz, CDC13) 7.98-7.95 (m, 1H), 7.52-7.33 (m, 4H), 7.10-7.08 (m, 1H), 7.02-6.89 (m, 2H), 6.64-6.62 (m, 1H), 6.50 (s, 1H), 6.43-6.38 (m, 1H), 5.83-5.80 (m, 1H), 4.49-3.98 (m, 6H), 3.71-3.38 (m, 3H), 2.49-2.43 (m, 1H), 1.36 (d, J =6.4 MHz, 3H), 1.18 (d, J =6.8 MHz, 3H), 1.05-0.98 (m, 3H). |
| I-64 | 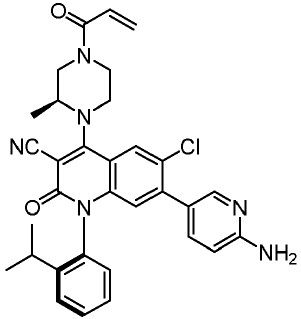 | 567.1 | HNMR: (400MHz, CDC13) 7.94 (s, 1H), 7.90-7.89 (m, 1H), 7.53-7.50 (m, 2H), 7.47-7.44 (m, 1H), 7.41-7.37 (m, 1H), 7.11-7.08 (m, 1H), 6.68-6.62 (m, 1H), 6.54-6.49 (m, 2H), 6.43-6.38 (m, 1H), 5.83-5.80 (m, 1H), 4.58 (s, 2H), 4.37-3.37 (m, 7H), 2.50-2.48 (m, 1H), 1.34 (d, J =6.4 MHz, 3H), 1.19 (d, J =6.8 MHz, 3H), 1.04 (d, J =6.8 MHz, 3H). |
| I-65 | 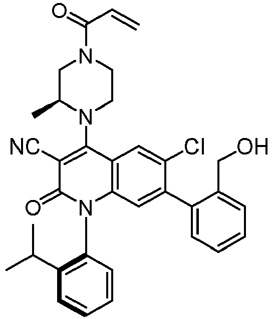 | 581.2 | HNMR: (400MHz, CDC13) 7.97-7.95(m, 1H), 7.55-7.53 (m, 1H), 7.47-7.40 (m, 3H), 7.35-7.30 (m, 2H), 7.11-7.09 (m, 1H), 7.02-6.95 (m, 1H), 6.65 (m, 2H), 6.43-6.38 (m, 1H), 5.83-5.80 (m, 1H), 4.50-4.02 (m, 6H), 3.71-3.39 (m, 3H), 2.50-2.45 (m, 1H), 1.37 (d, J =6.4 MHz, 3H), 1.18 (d, J =6.8 MHz, 3H), 1.06-0.99 (m, 3H). |
| I-66 | 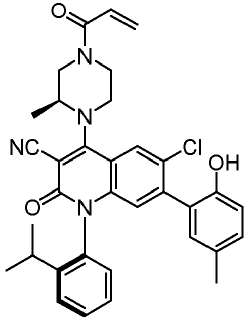 | 581.2 | HNMR: (400MHz, CDC13) 7.97-7.96(m, 1H), 7.68-7.66 (m, 3H), 7.10-7.06 (m, 2H), 6.79-6.76 (m, 2H), 6.69-6.63 (m, 2H), 6.43-6.38 (m, 1H), 5.83-5.81 (m, 1H), 4.36-3.98 (m, 6H), 3.75-3.38 (m, 3H), 2.50-2.47 (m, 1H), 2.30-2.25 (m, 3H), 1.35 (m, 3H), 1.19-1.17 (m, 3H), 1.07-1.02 (m, 3H). |
| I-67 | 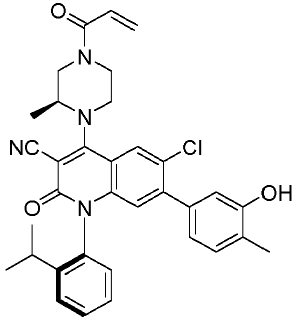 | 581.3 | HNMR: (400MHz, CDC13) 7.94 (s, 1H), 7.69-7.65 (m, 2H), 7.35-7.31 (m, 1H), 7.12-7.07 (m, 2H), 6.74-6.58 (m, 4H), 6.42-6.37 (m, 1H), 5.82-5.80 (m, 1H), 5.63 (s, 1H), 4.37-4.00 (m, 4H), 3.77-3.38 (m, 3H), 2.51-2.44 (m, 1H), 2.25 (s, 3H), 1.34 (m, 3H), 1.18 (d, J =6.8 MHz, 3H), 1.04 (d, J =6.8 MHz, 3H). |
| I-68 | 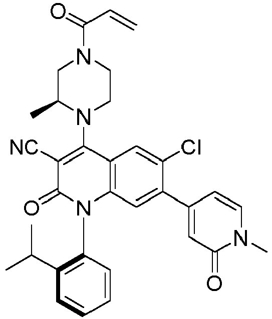 | 582.3 | HNMR: (400MHz, CDC13) 7.95 (s, 1H), 7.61-7.52 (m, 2H), 7.42-7.35 (m, 2H), 7.10-7.08 (m, 1H), 6.64-6.58 (m, 1H), 6.52 (s, 1H), 6.42-6.35 (m, 2H), 6.09-6.06 (m, 1H), 5.83-5.80 (m, 1H), 4.37-4.00 (m, 4H), 3.70-3.37 (m, 6H), 2.45-2.40 (m, 1H), 1.35 (d, J =6.0 MHz, 3H), 1.18 (d, J =6.8 MHz, 3H), 1.01 (d, J =6.8 MHz, 3H). |
| I-69 | 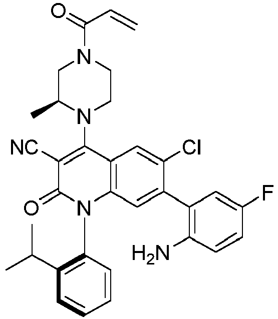 | 584.2 | HNMR: (400MHz, CDC13) 7.96 (s, 1H), 7.55-7.47 (m, 2H), 7.36-7.30 (m, 1H), 7.02-7.00 (m, 1H), 6.91-6.85 (m, 1H), 6.80-6.77 (m, 1H), 6.64-6.58 (m, 3H), 6.43-6.38 (m, 2H), 5.81-5.79 (m, 1H), 4.31-3.92 (m, 4H), 3.70-3.37 (m, 3H), 2.55-2.49 (m, 1H), 1.37 (m, 3H), 1.22 (d, J =6.8 MHz, 3H), 1.09(d, J =6.8 MHz, 3H). |
| I-70 | 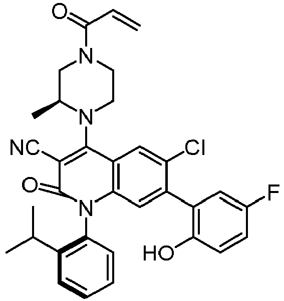 | 585.3 | HNMR: (400MHz, CDC13) 7.95 (s, 1H), 7.51-7.45 (m, 2H), 7.34-7.30 (m, 1H), 7.09-7.07 (m, 1H), 6.95-6.90 (m, 1H), 6.82-6.79 (m, 1H), 6.67-6.59 (m, 3H), 6.42-6.38 (m, 2H), 5.83-5.81 (m, 1H), 4.34-3.99 (m, 4H), 3.70-3.37 (m, 3H), 2.48-2.45 (m, 1H), 1.33 (m, 3H), 1.17 (d, J =6.8 MHz, 3H), 1.04 (d, J =6.8 MHz, 3H). |
| I-71 | 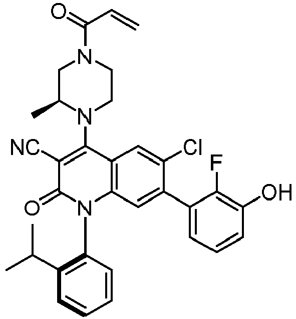 | 585.2 | HNMR: (400MHz, CDC13) 7.97 (s, 1H), 7.52-7.42 (m, 2H), 7.37-7.33 (m, 1H), 7.12-6.99 (m, 3H), 6.64-6.59 (m, 2H), 6.42-6.38 (m, 1H), 5.97 (s, 1H), 5.83-5.80 (m, 1H), 4.37-4.01 (m, 4H), 3.79-3.38 (m, 3H), 2.51-2.44 (m, 1H), 1.36-1.35 (m, 3H), 1.18 (d, J =6.8 MHz, 3H), 1.04 (d, J =6.8 MHz, 3H). |
| I-72 | 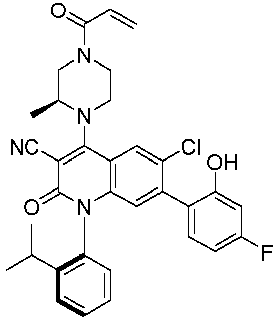 | 585.1 | HNMR: (400MHz, CDCl3) 7.94 (s, 1H), 7.52-7.43 (m, 3H), 7.08-7.06 (m, 1H), 6.89-6.85 (m, 1H), 6.63-6.58 (m, 4H), 6.43-6.39 (m, 1H), 5.83 (m, 1H), 4.37-4.00 (m, 4H), 3.70-3.37 (m, 3H), 2.49-2.45 (m, 1H), 1.33 (m, 3H), 1.17 (d, J =6.8 MHz, 3H), 1.04 (d, J =6.8 MHz, 3H). |
| I-73 | 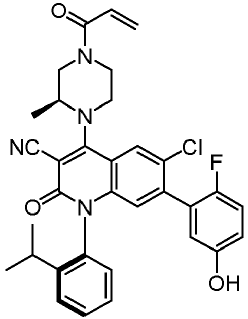 | 585.2 | HNMR: (400MHz, CDCl3) 7.96 (s, 1H), 7.51-7.46 (m, 2H), 7.36-7.32 (m, 1H), 7.10-7.08 (m, 1H), 6.96-6.91 (m, 1H), 6.82-6.78 (m, 1H), 6.64-6.55 (m, 2H), 6.42-6.37 (m, 1H), 6.12 (s, 1H), 5.83-5.81 (m, 1H), 4.38-4.00 (m, 4H), 3.78-3.38 (m, 3H), 2.49-2.42 (m, 1H), 1.34 (m, 3H), 1.17 (d, J =6.8 MHz, 3H), 1.03 (d, J =6.8 MHz, 3H). |
| I-74 | 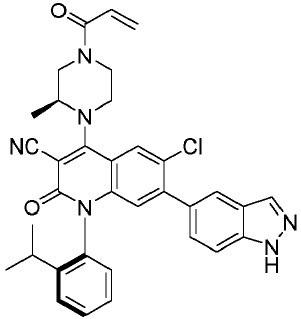 | 591.3 | HNMR (400 MHz, CDCl3) 8.43 (s, 1H), 7.60-7.53 (m, 3H), 7.48-7.37 (m, 3H), 7.30-7.22 (m, 1H), 6.95-6.83(m, 1H), 6.48(s,1H), 6.33 (d, J=16.4 Hz, 1H), 5.85 (dd, J=1.6, 10.4 Hz, 1H), 4.42-4.14(m,2H), 4.10-3.90(m,2H), 3.72-3.45 (m,3H),2.72-2.58(m,1H), 1.42-1.35(m,3H), 1.24-1.17(m, 3H), 1.11-0.98(m, 3H). |
| I-75 | 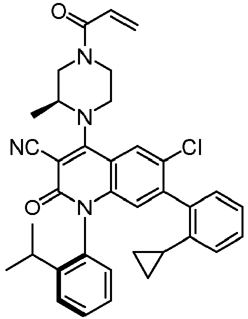 | 591.3 | HNMR: (400MHz, CDCl3) 7.96 (s, 1H), 7.48-7.45 (m, 2H), 7.34-7.32 (m, 1H), 7.18-7.09 (m, 2H), 6.96-6.85 (m, 1H), 6.64-6.62 (m, 2H), 6.42-6.38 (m, 1H), 6.12 (s, 1H), 5.83-5.80 (m, 1H), 4.37-4.02 (m, 4H), 3.71-3.39 (m, 3H), 2.51-2.46 (m, 1H), 1.38-1.36 (m, 3H), 1.29-1.24 (m, 1H), 1.20-1.17 (m, 3H), 1.05-0.95 (m, 3H), 0.88-0.62 (m, 4H). |
| I-76 | 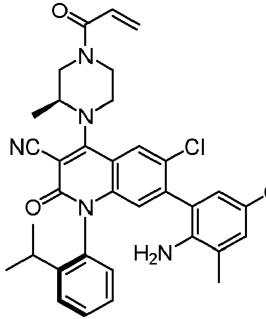 | 614.3 | HNMR: (400MHz, CDCl3) 8.00-7.99 (m, 1H), 7.53-7.32 (m, 2H), 7.12-7.06 (m, 2H), 6.73-6.59 (m, 2H), 6.43-6.38 (m, 1H), 5.83-5.80 (m, 1H), 4.42-4.01 (m, 4H), 3.77-3.31 (m, 3H), 2.47-2.42 (m, 1H), 2.17-1.96 (m, 3H), 1.30-1.29 (m, 3H), 1.19-1.14 (m, 3H), 1.09-0.98 (m, 3H). |
| I-77 | 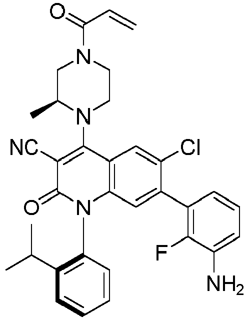 | 584.1 | HNMR: (400MHz, CDCl3) 7.96 (s, 1H), 7.52-7.44 (m, 2H), 7.37-7.33 (m, 1H), 7.11-7.09 (m, 1H), 6.95-6.91 (m, 1H), 6.82-6.79 (m, 1H), 6.64-6.59 (m, 2H), 6.43-6.38 (m, 2H), 5.82-5.80 (m, 1H), 4.39-4.03 (m, 5H), 3.80 (s, 2H), 3.58-3.38 (m, 2H), 2.51-2.44 (m, 1H), 1.35 (d, J =6.0 MHz, 3H), 1.18 (d, J =6.8 MHz, 3H), 1.04 (d, J =6.8 MHz, 3H). |

### Preparation of Compound 1-76

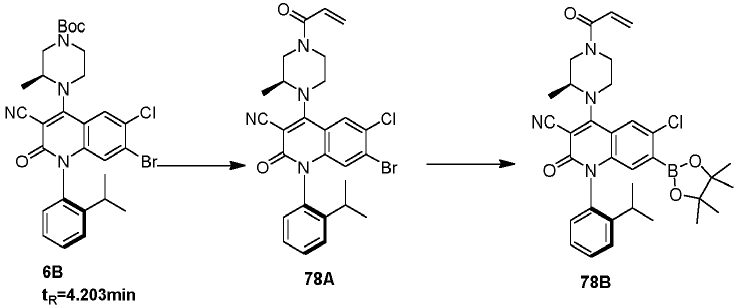

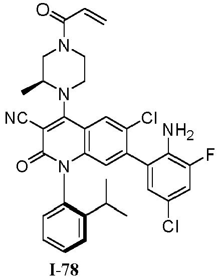

### Step 1: Synthesizing Compound 78A

Compound 6B (681 mg, 1.14 mmol) was dissolved in DCM (5 mL) at room temperature. Then, TFA (2 ml) was added thereto. The mixture was stirred at room temperature for 2 hours. The reaction was monitored by TLC. After the reaction was complete, the reaction solution was dried by rotary evaporation. The resulting yellow liquid compound was redissolved in DCM (5 mL), and then the resultant was cooled to -10°C. Triethylamine (303 mg, 3 mmol) and acryloyl chloride (150 mg, 1.5 mmol) were added thereto successively. Then, after the mixture was naturally warmed up to room temperature and allowed to react for 1 hour, TLC showed the completion of the reaction. The reaction was quenched by adding MeOH (1 mL). After the reaction mixture was concentrated under reduced pressure, the residue was purified by silica gel column chromatography (dichloromethane/methanol=100/1 (V : V volume ratio)) to obtain Compound 78A (252 mg, light yellow solid).

MS m/z(ESI): 553[M+1].

### Step 2: Synthesizing Compound 78B

Compound 78A (1.0 g, 1.85 mmol) was added to dioxane (10 ml). KOAc (364 mg, 3.7 mmol) and bis(pinacolato)diboron (705 mg, 2.8 mmol) were added thereto. The reaction solution was purged with nitrogen gas three times, and then a dichloromethane complex of Pd(dppf)Cl₂ (159 mg, 0.19 mmol) was added thereto successively. The resulting reaction solution was again purged with nitrogen gas three times and then stirred overnight at 100°C. After TLC showed that the reaction was complete, the reaction solution was concentrated under vacuum, and the resulting residue was purified by silica gel column chromatography (petroleum ether/ethyl acetate = 10 : 1 (V : V volume ratio)) to obtain Compound 78B (750 mg, light yellow solid).

MS m/z (ESI): 601[M+1].

### Step 3: Synthesizing Compound 1-78

Compound 78B (100 mg, 0.16 mmol) was added to dioxane (2 ml) and water (0.5 ml). K₃PO₄ (106 mg, 0.5 mmol) and 2-bromo-4-chloro-6-fluoroaniline (111 mg, 0.5 mmol) were added thereto. After the reaction solution was purged with nitrogen gas three times, a dichloromethane complex of Pd(dppf)Cl₂ (25 mg, 0.03 mmol) was added thereto. The resulting reaction solution was again purged with nitrogen gas three times and then stirred overnight at 100°C. After TLC showed that the reaction was complete, the reaction solution was concentrated under vacuum, and the resulting residue was purified by preparative silica gel plate (prepar-TLC) (developing solvent system: petroleum ether/ethyl acetate = 1 : 2 (V : V volume ratio)) to obtain Compound 1-78 (26 mg, light yellow solid).

MS m/z(ESI): 618[M+1].

Compound 6B (t_{R} = 4.203 min) and commercially available substituted aniline bromide were used as the starting materials to obtain Example Compounds 1-79 to 1-82 in Table 2 (refer to the synthesis of Compound 1-78).

**Table 2**

| Examples | Structures | LCMS [M+H]⁺ |
|---|---|---|
| 1-79 | 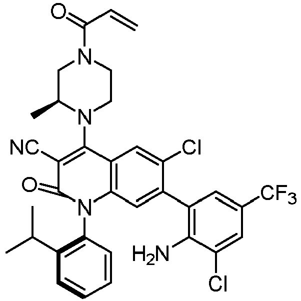 | 668.3 |
| 1-80 | 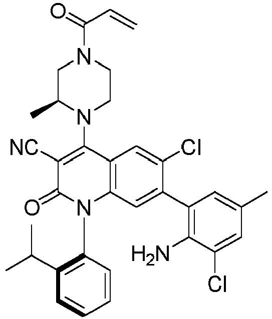 | 614.2 |
| 1-81 | 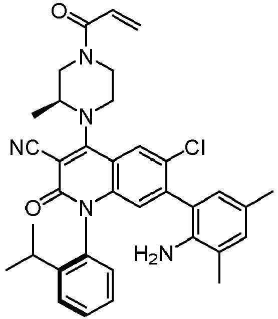 | 594.3 |
| 1-82 | 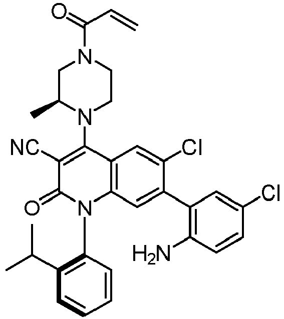 | 599.2 |

### Preparation of Compound 1-83-1 and Compound I-83-2

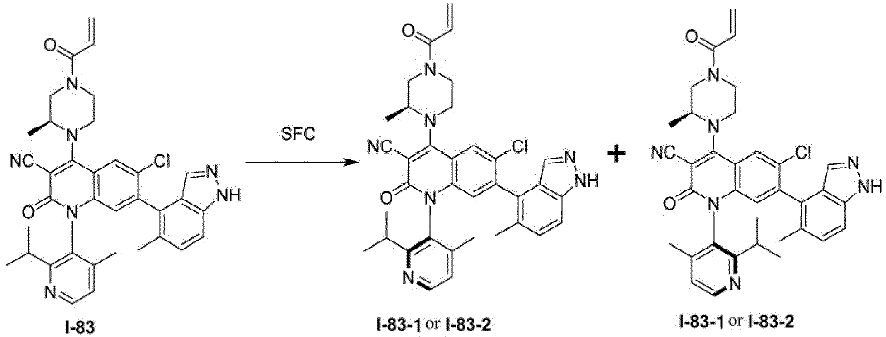

### Step 1:

Compound I-83 was synthesized by referring to the synthesis method of Compound 1-2 in Example 2.

### Step 2:

Compound I-83 was subjected to chiral resolution by SFC (column model: CHIRALPAK IC, 250 mm ^{∗} 30 mm, 5 µm; mobile phase A: n-hexane/dichloromethane (75/25, containing 10 mM methylamine); mobile phase B: methanol, detection wavelength: 254 nm) to obtain Compound 1-83-1 (t_{R} = 2.72 min) and Compound 1-83-2 (t_{R} = 3.85 min).

Compound represented by Formula I-83-1:
MS m/z(ESI):620[M+1].

¹H NMR (400 MHz, CDCl₃) 8.51 (d, J =4.8 MHz, 1H), 8.07 (s, 1H), 7.52-7.41 (m, 2H), 7.30-7.28 (m, 1H), 7.11-7.10 (m, 1H), 6.68-6.60 (m, 1H), 6.49 (s, 1H), 6.45-6.40 (m, 1H), 5.84-5.82 (m, 1H), 4.47-3.45 (m, 7H), 2.62-2.53 (m, 1H), 2.12 (s, 3H), 2.09 (s, 3H), 1.48 (d, J =6.8 MHz, 3H), 1.18 (d, J =6.8 MHz, 3H), 1.01 (d, J =6.8 MHz, 3H).

Compound represented by Formula I-83-2:
MS m/z(ESI):620[M+1].

¹H NMR (400 MHz, CDCl3) 8.59-8.57 (m, 1H), 8.08-8.06 (m, 1H), 7.52-7.30 (m, 4H), 6.67-6.62 (m, 2H), 6.45-6.40 (m, 1H), 5.85-5.82 (m, 1H), 4.65-3.46 (m, 7H), 2.82-2.75 (m, 1H), 2.24-1.97 (m, 6H), 1.44-1.43 (m, 3H), 1.26-1.21 (m, 3H), 1.16 (m, 3H).

### Preparation of Compound 1-84-1 and Compound I-84-2

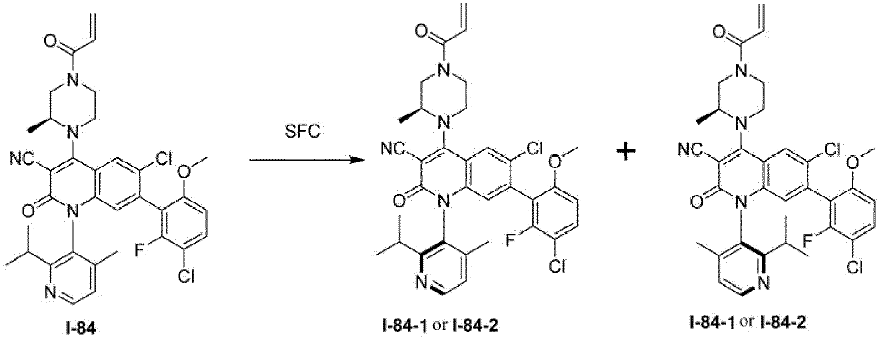

### Step 1:

Compound 1-84 was synthesized by referring to the synthesis method of Compound 1-2 in Example 2.

### Step 2:

Compound 1-84 was subjected to chiral resolution by SFC (column model: CHIRALPAK IC, 250 mm ^{∗} 30 mm, 5 µm; mobile phase A: n-hexane/dichloromethane (75/25, containing 10 mM methylamine); mobile phase B: methanol, detection wavelength: 254 nm) to obtain

Compound 1-84-1 (t_{R} = 3.32 min) and Compound 1-84-2 (t_{R} = 4.16 min).

Compound represented by Formula I-84-1:
MS m/z(ESI):648.2[M+1].

¹H NMR (400 MHz,CDCl₃) 8.59 (d, J =4.8 MHz, 1H), 8.01 (d, J =4.8 MHz, 1H), 7.39 (t, J =8.4 MHz, 1H), 7.18-7.17 (m, 1H), 6.70-6.66 (m, 2H), 6.43-6.39 (m, 2H), 5.82 (d, J =8.4 MHz, 1H), 4.37-4.32 (m, 1H), 4.04 (m, 2H), 3.69 (s, 3H), 3.68-3.57 (m, 2H), 2.74-2.69 (m, 1H), 1.96 (s, 3H), 1.39 (d, J =6.4 MHz, 3H), 1.26-1.25 (m, 1H), 1.23 (d, J =6.4 MHz, 3H), 1.10 (d, J =6.4 MHz, 3H).

Compound represented by Formula I-84-2:
MS m/z(ESI):648.2[M+1].

¹H NMR (400 MHz, CDCl₃) 8.60 (d, J =4.8 MHz, 1H), 8.00 (brs, 1H), 7.39 (t, J =8.4 MHz, 1H), 7.15-7.14 (m, 1H), 6.70-6.66 (m, 2H), 6.44-6.39 (m, 2H), 5.84-5.81 (m, 1H), 4.37-4.04 (m, 4H), 3.69 (s, 3H), 3.45-3.42 (m, 2H), 2.72-2.68 (m, 1H), 1.97 (s, 3H), 1.39 (d, J =6.4 MHz, 3H), 1.26-1.25 (m, 1H), 1.22 (d, J =6.4 MHz, 3H), 1.10 (d, J =6.4 MHz, 3H).

### Preparation of Compound 1-85-1 and Compound I-85-2

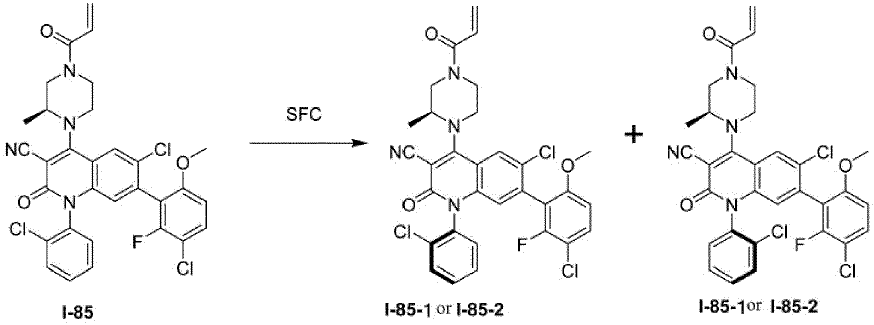

### Step 1:

Compound 1-85 was synthesized by referring to the synthesis method of Compound 1-2 in Example 2.

### Step 2:

Compound 1-85 was subjected to chiral resolution by SFC (column model: CHIRALPAK IC, 250 mm ^{∗} 30 mm, 5 µm; mobile phase A: n-hexane/dichloromethane (75/25, containing 10 mM methylamine); mobile phase B: methanol, detection wavelength: 254 nm) to obtain Compound 1-85-1 (t_{R} = 3.45 min) and Compound I-85-2 (t_{R} = 4.67 min).

Compound represented by Formula I-85-1:
MS m/z(ESI):625[M+1].

¹H NMR (400 MHz, CDCl3) 8.00 (s, 1H), 7.61-7.60 (m, 1H), 7.50-7.48 (m, 2H), 7.39-7.36 (m, 2H), 6.70-6.68 (m, 2H), 6.51-6.50 (m, 1H), 5.83-5.81 (m, 1H), 4.31-4.30 (m, 2H), 3.76-3.69 (m, 3H), 3.64 (s, 3H), 3.40 (m, 1H), 1.37-1.35 (m, 4H).

Compound represented by Formula I-85-2:
MS m/z(ESI):625[M+1].

¹H NMR (400 MHz, CDCl₃) 7.99 (s, 1H), 7.65-7.63 (m, 1H), 7.48-7.35 (m, 4H), 6.70-6.68 (m, 2H), 6.51 (s, 1H), 6.42-6.38 (m, 1H), 5.83-5.80 (m, 1H), 4.30-3.98 (m, 4H), 3.74-3.70 (m, 4H), 3.59 (m, 1H), 3.42-3.40 (m, 1H), 1.37-1.36 (d, J =6.0 MHz, 3H).

### Preparation of Compound 1-86-1 and Compound I-86-2

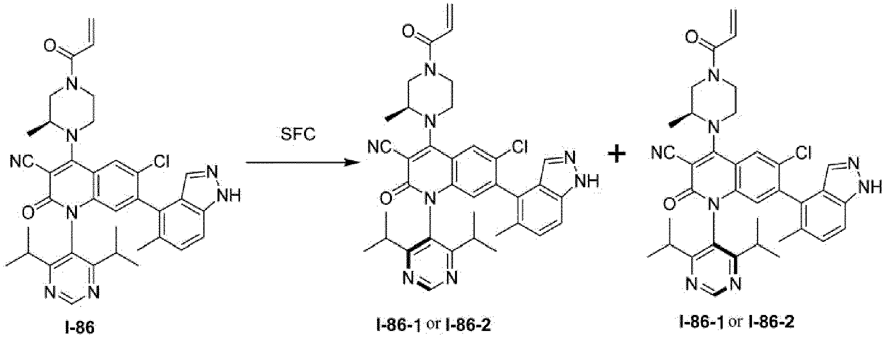

### Step 1:

Compound 1-86 was synthesized by referring to the synthesis method of Compound 1-2 in Example 2.

### Step 2:

Compound 1-86 was subjected to chiral resolution by SFC (column model: CHIRALPAK IC, 250 mm ^{∗} 30 mm, 5 µm; mobile phase A: n-hexane/dichloromethane (75/25, containing 10 mM methylamine); mobile phase B: methanol, detection wavelength: 254 nm) to obtain Compound 1-86-1 (t_{R} = 2.72 min) and Compound 1-86-2 (t_{R} = 3.65 min).

Compound represented by Formula I-86-1:
MS m/z(ESI):649.2[M+1].

¹H NMR (400 MHz, CDCl3) 8.90 (s, 1H), 7.68-7.66 (m, 1H), 7.41 (t, J =8.8 MHz, 1H), 7.00 (m, 1H), 6.73-6.62 (m, 2H), 6.44-6.40 (m, 1H), 5.85-5.82 (m, 1H), 4.39-3.86 (m, 4H), 3.76-3.72 (m, 4H), 3.53-3.46 (m, 2H), 3.08-3.01 (m, 2H), 1.44-1.42 (m, 3H), 1.22-1.20 (m, 12H).

Compound represented by Formula I-86-2:
MS m/z(ESI):649.2[M+1].

¹H NMR (400 MHz, CDCl3) 8.91 (s, 1H), 7.55 (m, 1H), 7.42 (t, J =8.8 MHz, 1H), 7.18-7.16 (m, 1H), 6.73-6.58 (m, 2H), 6.44-6.39 (m, 1H), 5.84-5.82 (m, 1H), 4.68-3.95 (m, 4H), 3.73-3.70 (m, 3H), 3.52-3.49 (m, 2H), 3.09-3.07 (m, 3H), 1.43-1.41 (m, 3H), 1.24-1.23 (m, 12H).

### Preparation of Compound 1-87-1 and Compound I-87-2

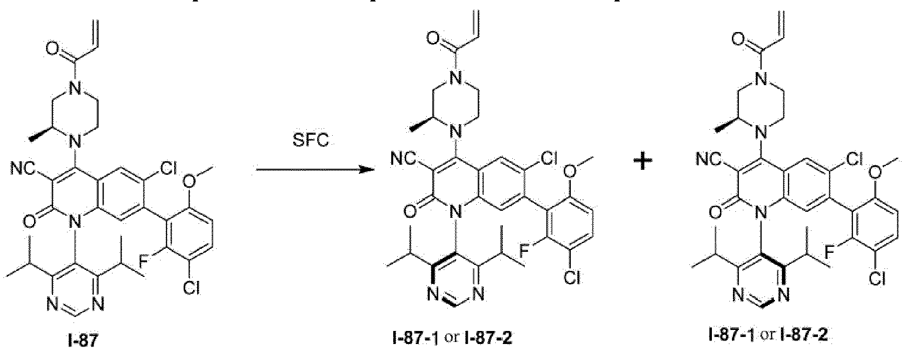

### Step 1:

Compound 1-87 was synthesized by referring to the synthesis method of Compound 1-2 in Example 2.

### Step 2:

Compound 1-87 was subjected to chiral resolution by SFC (column model: CHIRALPAK IC, 250 mm ^{∗} 30 mm, 5 µm; mobile phase A: n-hexane/dichloromethane (75/25, containing 10 mM methylamine); mobile phase B: methanol, detection wavelength: 254 nm) to obtain Compound 1-87-1 (t_{R} = 3.34 min) and Compound 1-87-2 (t_{R} = 4.09 min).

Compound represented by Formula I-87-1:
MS m/z(ESI):677.2[M+1].

¹H NMR (400 MHz, MeOD) HNMR: (400MHz, CDCl3) 8.90 (s, 1H), 7.68-7.66 (m, 1H), 7.41 (t, J =8.8 MHz, 1H), 7.00 (m, 1H), 6.73-6.62 (m, 2H), 6.44-6.40 (m, 1H), 5.85-5.82 (m, 1H), 4.39-3.86 (m, 4H), 3.76-3.72 (m, 4H), 3.53-3.46 (m, 2H), 3.08-3.01 (m, 2H), 1.44-1.42 (m, 3H), 1.22-1.20 (m, 12H).

Compound represented by Formula I-87-2:
MS m/z(ESI):677.2[M+1].

¹H NMR (400 MHz, MeOD) HNMR: (400MHz, CDCl3) 8.88 (s, 1H), 7.63-7.57 (m, 1H), 7.48-7.43 (m, 2H), 7.33-7.28 (m, 1H), 7.24-7.22 (m, 1H), 6.64-6.59 (m, 1H), 6.45-6.40 (m, 1H), 5.85-5.81 (m, 1H), 4.58-3.45 (m, 7H), 3.12-3.06 (m, 2H), 2.20 (s, 3H), 1.46-1.45 (m, 3H), 1.24 (d, J =6.8 MHz, 12H).

### Effect Example 1: Pharmacodynamic Assay 1 (Determination of the efficacy of the compounds of the present disclosure on NCI-H358 human non-small cell lung cancer cell with KRAS G12C mutation)

The following method was used to determine the effect of the compounds of the present disclosure on tumor cell proliferation.

For the KRAS G12C subtype, NCI-H358 non-small cell lung cancer cells with KRAS G12C mutation were used to determine the efficacy of the compounds in inhibiting cancer cell viability. NCI-H358 cells were cultured in a DMEM medium containing 10% fetal bovine serum, 100 U penicillin and 100 ng/mL streptomycin. The cells were cultured in an incubator with 5% CO₂ at 37°C. Cancer cell viability was evaluated by determining the content of ATP with Cell Titer-Glo^{®} kit (Luminescent Cell Viability Assay kit, please refer to the manufacturer's instructions for use) and evaluating the inhibition of cell growth.

The experimental method was proceeded in accordance with the steps in the instructions for the kit, and was described briefly as follows. The test compounds were first dissolved in DMSO to prepare stock solutions, and then the stock solutions were subjected to gradient dilution with the culture media of the corresponding cells, thereby preparing test samples. The final concentrations of the compounds were in the range of 30 µM to 0.01 nM. Tumor cells in logarithmic growth phase were seeded into a 96-well cell culture plate at an appropriate density. After the cell culture plate was left overnight in an incubator with 5% CO₂ at 37°C, the test compound samples were added thereto and then the cells were further cultured for 72 hours. After the incubation was complete, an appropriate volume of Cell Titer-Glo^{®} reagent was added into each well, and the cells were incubated at 37 °C for 1 to 4 hours. Then, the absorbance value of each sample well at 450 nM was read on a microplate reader. The percentage inhibition rates of the compounds at each concentration point were calculated by comparing the absorbance values of the sample wells with that of the control group (0.3% DMSO), and then non-linear regression analysis of the concentration of the compound vs inhibition rate was performed by using GraphPad Prism 5 software, thereby obtaining IC₅₀ values indicating the inhibitory effects of the compounds on cell proliferation, where A represented IC₅₀of less than 100 nM, B represented IC₅₀of between 100 nM and 1000 nM, and C represented IC₅₀of greater than 1000 nM. The specific experimental results were as shown in Table 3.

**Table 3: IC₅₀ data indicating the inhibitory effects of the compounds represented by Formula I of the present disclosure on NCI-H358 human non-small cell lung cancer cells**

| Compound No. | IC₅₀ (nM)/NCI-H358 |
|---|---|
| Control Compound ARS-1620 | 226 |
| I-1 | 1172 |
| 1-2-1 | 1150 |
| 1-2-2 | 50 |
| 1-3-1 | 1980 |
| 1-3-2 | 250 |
| I-4-1 | C |
| I-4-2 | B |
| I-5-1 | C |
| I-5-2 | B |
| I-6-1 | C |
| I-6-2 | A |
| I-7-1 | C |
| I-7-2 | B |
| I-8-1 | C |
| I-8-2 | B |
| I-9 | B |
| I-10 | c |
| I-11 | c |
| I-12 | B |
| I-13 | B |
| I-14 | B |
| I-15 | B |
| I-16 | B |
| I-17 | C |
| I-18 | B |
| I-19 | C |
| I-20 | B |
| I-21 | B |
| I-22 | A |
| I-23 | B |
| I-24 | A |
| I-25 | B |
| I-26 | B |
| I-27 | B |
| I-28 | C |
| I-29 | B |
| I-30 | C |
| I-31 | A |
| I-32 | B |
| I-34 | C |
| I-35 | A |
| I-36 | B |
| I-37 | B |
| I-38 | B |
| I-39 | B |
| I-40 | C |
| I-41 | B |
| 1-42 | B |
| 1-43 | A |
| 1-44 | B |
| 1-45 | B |
| I-46 | C |
| 1-47 | B |
| 1-48 | B |
| 1-49 | B |
| I-50 | B |
| I-51 | B |
| I-52 | B |
| I-53 | B |
| I-54 | B |
| I-55 | B |
| I-56 | B |
| I-57 | A |
| I-58 | B |
| I-59 | C |
| 1-60 | B |
| I-61 | A |
| 1-62 | B |
| 1-63 | B |
| 1-64 | B |
| 1-65 | B |
| 1-66 | A |
| 1-67 | B |
| 1-68 | B |
| 1-69 | A |
| 1-70 | A |
| I-71 | B |
| 1-72 | A |
| 1-73 | B |
| I-74 | C |
| 1-75 | B |
| 1-76 | A |
| 1-77 | B |
| 1-78 | A |
| 1-79 | A |
| 1-80 | A |
| I-81 | B |
| I-82 | A |
| I-83-1 | A |
| I-83-2 | B |
| I-84-1 | A |
| I-84-2 | B |
| I-85-1 | B |
| I-85-2 | B |
| I-86-1 | A |
| I-86-2 | B |
| I-87-1 | A |
| I-87-2 | B |

Conclusion: as could be seen from Table 3, the compounds of the present disclosure had very good inhibitory effects on NCI-H358 human non-small cell lung cancer cells, in which KRAS G12C was highly expressed, and the compounds of the present disclosure could be used as drugs prepared as KRAS G12C inhibitors.

### Effect Example 2: Pharmacodynamic Assay 2 (Determination of the efficacy of the compounds of the present disclosure on HCT116 human colon cancer cells with high expression of KRAS G13D and A549 human non-small cell lung cancer cells with high expression of KRAS G12S)

The following method was used to determine the effects of the compounds of the present disclosure on tumor cell proliferation.

For KRAS G13D subtype, HCT116 human colon cancer cell was used. For KRAS G12S subtype, A549 human non-small cell lung cancer cell was used. The efficacy of the compounds in inhibiting cancer cell viability was determined. HCT116 cells or A549 cells were cultured in a DMEM medium containing 10% fetal bovine serum, 100 U penicillin and 100 ng/mL streptomycin. The cells were cultured in an incubator with 5% CO₂ at 37°C. Cancer cell viability was evaluated by determining the content of ATP with Cell Titer-Glo^{®} kit (Luminescent Cell Viability Assay kit, please refer to the manufacturer's instructions for use) and evaluating the inhibition of cell growth.

The experimental method was proceeded in accordance with the steps in the instructions for the kit, and was described briefly as follows. The test compounds were first dissolved in DMSO to prepare stock solutions, and then the stock solutions were subjected to gradient dilution with the culture media of the corresponding cells, thereby preparing test samples. The final concentrations of the compounds were in the range of 30 µM to 0.01 nM. Tumor cells in logarithmic growth phase were seeded into a 96-well cell culture plate at an appropriate density. After the cell culture plate was left overnight in an incubator with 5% CO₂ at 37°C, the test compound samples were added thereto and then the cells were further cultured for 72 hours. After the incubation was complete, an appropriate volume of Cell Titer-Glo^{®} reagent was added into each well, and the cells were incubated at 37 °C for 1 to 4 hours. Then, the absorbance value of each sample well at 450 nM was read on a microplate reader. The percentage inhibition rates of the compounds at each concentration point were calculated by comparing the absorbance values of the sample wells with that of the control group (0.3% DMSO), and then non-linear regression analysis of the concentration of the compound vs inhibition rate was performed by using GraphPad Prism 5 software, thereby obtaining IC₅₀ values indicating the inhibitory effects of the compounds on cell proliferation. The experimental results were as shown in Table 4.

**Table 4: IC₅₀ data indicating the inhibitory effects of some of the compounds of the present disclosure on HCT116 human colon cancer cells and A549 human non-small cell lung cancer cells**

| Examples | IC₅₀ (nM)/HCT116 | IC₅₀(nM)/A549 |
|---|---|---|
| Control compound ARS-1620 | >3000 | >3000 |
| I-1 | >3000 | >3000 |
| 1-2-1 | >3000 | >3000 |
| 1-2-2 | >3000 | >3000 |

Conclusion: The compounds of the present disclosure had no inhibitory effect on cells without G12C mutation, such as HCT116 human colon cancer cells and A549 human non-small cell lung cancer cells, which indicated that the compounds of the present disclosure had extremely high selectivity.

### Effect Example 3: Pharmacokinetic Experiment

(1) Compound 1-2-2 of the present disclosure prepared in the above Example was used to be formulated into a clear solution (0.3 mg/mL) (2% DMSO + 30% PEG 300 + 2% Tween 80 + 66% H₂O) as a drug for oral administration; and was used to be formulated into a clear solution (0.2 mg/mL) (2% DMSO + 30% PEG 300 + 2% Tween 80 + 66% H₂O) as a drug for intravenous administration.
(2) Male CD-1 mice (three mice in each group, weighing 27 g to 28 g) were provided by Shanghai Slack Laboratory Animal Co., Ltd. The test mice were given an environmental accommodation period of 2 to 4 days before the experiment. The mice were fasted for 8 to 12 hours before administration, and they were provided access to water (2 hours after administration) and food (4 hours after administration).
(3) 12 hours after the mice were fasted but had free access to water, blank plasma at time 0 was taken.
(4) The mice in step 1) were taken; and they were orally (PO) administered 3 mg/kg of the compound to be tested, or intravenously (IV) administered 1 mg/kg of the compound to be tested.
(5) At 5 min, 15 min, 30 min, 1 h, 2 h, 4 h, 8 h, 10 h, and 24 h after oral administration, blood was successively taken from the fundus oculi venous plexus and placed in EP tubes with heparin. After centrifugation at 8000 rpm/min for 5 min, the plasma in upper layer was collected and frozen at -20°C, waiting for LC-MS/MS analysis.
(6) WinNonlin software was used to calculate the pharmacokinetic parameters based on the blood concentration-time data obtained in step 3). The specific data were as shown in Table 5.

**Table 5: Pharmacokinetic data of the compound of the Example of the present disclosure**

| Groups | Parameters | Example |
|---|---|---|
| IV (1mg/kg) | Cl (mL/kg/min) | 16.7 |
| | V_{d} (L/kg) | 1.17 |
| | AUC (ng·h/mL) | 867 |
| | T_{1/2} (h) | 0.718 |
| PO (3mg/kg) | Cₘₐₓ (ng/mL) | 220 |
| | Tₘₐₓ (h) | 1.0 |
| | AUC (ng·h/mL) | 479 |
| | F (%) | 18.9% |

Conclusion: In the pharmacokinetic evaluation experiment conducted in mice, the compound of the present disclosure showed relatively high exposure and very ideal oral bioavailability at a relatively low dose.

### Effect Example 4: In-vivo Pharmacodynamic Experiment

Purpose of the experiment: To evaluate the anti-tumor effect of the test drug in female BALB/c nude mice model subcutaneously xenografted with MIAPaCa-2 human-derived pancreatic cancer cells.

Experimental operation: BALB/c nude mice, female, 6- to 8-weeks old, weighing about 17.6 g to 21.1 g. Each mouse was subcutaneously inoculated with 5×10⁶ MIAPaCa-2 cells (with Matrigel; the volume ratio was 1:1) in the right flank. The administration was initiated when the average tumor volume reached about 100 cubic millimeters. The test compounds were orally administered daily by intragastric injection (6 mice in each group) for a total of 16 or 23 days. The doses were as shown in Table 5. Tumor volumes were measured three times a week. The volume was measured in cubic millimeters and calculated by the following formula: tumor volume (mm³) = 1/2 × (a × b²) (where a represented the long diameter and b represented the short diameter). The anti-tumor efficacy of the compound was evaluated by TGI (%).The calculation formula was as follows: TGI% = (1-T/C) × 100% (T and C were the relative tumor volumes (RTV) of a mouse in the treatment group and a mouse in the control group at a specific time point), respectively. Safety was evaluated according to the changes of the body weight of the animals and animal deaths.

The experimental results were as shown in Table 6 and Table 7.

**Table 6**

| Groups | Tumor volumes (mm³) (Day 0) | Tumor volumes (mm³) (Day 23) | TGI (%) | T/C | P Value |
|---|---|---|---|---|---|
| Solvent control group | 100.3 | 631.29 | - | - | - |
| Example 1-2-2 30 mg/kg (Day 0 to Day 9) + 15 mg/kg (Day 10 to Day 16) | 100.25 | 438.75 | 36.25 | 63.75 | 0.517 |
| Example 1-2-2 100 mg/kg (Day 0 to Day 9) + 50 mg/kg (Day 10 to Day 16) + 100 mg (Day 17 to Day 23) | 100.06 | 179.60 | 85.02 | 14.98 | 0.001 |

Experimental conclusion: The compound of the present disclosure showed good *in-vivo* efficacy in a tumor model subcutaneously xenografted with MIAPaCa-2 human pancreatic cancer cells. 23 days after the initiation of the administration, the compound of the present disclosure, as compared with the solvent control group, showed significant inhibitory effect on tumor and an obvious dose-response relationship.

**Table 7**

| Groups | Weight at the Initiation of Administration (g) | Weight on Day 23 (g) | Weight Change Rate (%) |
|---|---|---|---|
| Solvent control group | 20.1 | 21.1 | +4.64 |
| Example 1-2-2 30 mg/kg (Day 0 to Day 9) + 15 mg/kg (Day 10 to Day 16) | 19.8 | 21.3 | +7.53 |
| Example 1-2-2 100 mg/kg (Day 0 to Day 9) + 50 mg/kg (Day 10 to Day 16) + 100 mg (Day 17 to Day 23) | 20.0 | 22.4 | +12.32 |

Experimental conclusion: In the treatment groups of the compound of the present disclosure, there was no animal death, no weight loss, and no obvious drug toxicity during the administration period, and the compound of the present disclosure was well tolerated during the treatment period.

In the description of this specification, descriptions with reference to the terms "one Example", "some Examples", "examples", "a specific example", "some examples" or the like mean that the specific features, structures, materials, or characteristics described in conjunction with the Example or example are included in at least one Example or example of the present disclosure. In this specification, the exemplary description of the above-mentioned terms does not necessarily refer to the same Example or example. Those described are merely preferred Examples of the present disclosure and are not intended to limit the present disclosure. Any modifications, equivalent substitutions, improvements and the like made within the spirit and principles of the present disclosure shall be included in the protection scope of the present disclosure.

## Claims

1. A compound represented by formula I, or a pharmaceutically acceptable salt thereof, or a stereoisomer thereof, or a tautomer thereof, or a hydrate thereof, or a solvate thereof, or a metabolite thereof, or a prodrug thereof, 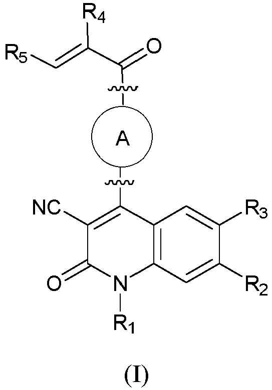 wherein,
R₁ is independently selected from an aryl optionally substituted with a plurality of R₆, or a heteroaryl optionally substituted with a plurality of R₆; and when R₁ is substituted with a plurality of R₆, each R₆ may be the same as or different from each other;
R₂ is independently selected from an aryl optionally substituted with a plurality of R₇, or a heteroaryl optionally substituted with a plurality of R₇; and when R₂ is substituted with a plurality of R₇, each R₇ may be the same as or different from each other;
R₃ is selected from H, halogen, cyano, amide group, hydroxy, amino, C₁-C₃ alkyl, C₁-C₃ heteroalkyl, C₁-C₃ haloalkyl, or C₃-C₈ heterocycloalkyl; said C₁-C₃ alkyl, C₁-C₃ heteroalkyl, C₁-C₃ haloalkyl, or C₃-C₈ heterocycloalkyl is optionally substituted with 0 to 3 R₇, and when said C₁-C₃ alkyl, C₁-C₃ heteroalkyl, C₁-C₃ haloalkyl, or C₃-C₈ heterocycloalkyl is substituted with a plurality of R₇, each R₇ may be the same as or different from each other;
R₄ and R₅ are each independently selected from H, halogen, C₁-C₈ alkyl optionally substituted with 0 to 3 R₇, or C₁-C₈ heteroalkyl optionally substituted with 0 to 3 R₇; 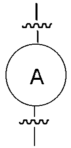 is selected from C₄-C₈ monoheterocycloalkyl optionally substituted with 0 to 3 R₈, C₆-C₁₂ bridged heterocycloalkyl optionally substituted with 0 to 3 R₈, or C₆-C₁₂ spiroheterocycloalkyl optionally substituted with 0 to 3 R₈;
R₆ is selected from halogen, OH, CN, NH₂, C₁-C₈ alkyl, C₁-C₈ heteroalkyl, C₁-C₃ haloalkyl, C₁-C₃ haloalkoxy, C₃-C₈ cycloalkyl, or C₃-C₈ heterocycloalkyl; wherein said C₁-C₈ alkyl, C₁-C₈ heteroalkyl, C₁-C₃ haloalkyl, C₁-C₃ haloalkoxy, C₃-C₈ cycloalkyl, or C₃-C₈ heterocycloalkyl may be substituted with a plurality of the following groups: F, Cl, Br, I, OH, CN, NH₂, CH₃, CH₃CH₂, CH₃O, CF₃, CHF₂, CH₂F, cyclopropyl, isopropyl, N(CH₃)₂, and NH(CH₃)₂;
R₇ is selected from halogen, OH, CONH₂, CN, NH₂, C₁-C₈ alkyl, C₁-C₈ heteroalkyl, C₁-C₃ haloalkyl, C₁-C₃ haloalkoxy, C₃-C₈ cycloalkyl, or C₃-C₈ heterocycloalkyl, wherein said C₁-C₈ alkyl, C₁-C₈ heteroalkyl, C₁-C₃ haloalkyl, C₁-C₃ haloalkoxy, C₃-C₈ cycloalkyl, or C₃-C₈ heterocycloalkyl may be substituted with a plurality of the following groups: F, Cl, Br, I, OH, CN, NH₂, CH₃, CH₃CH₂, CH₃O, CF₃, CHF₂, CH₂F, cyclopropyl, isopropyl, N(CH₃)₂, and NH(CH₃)₂; and
R₅ is selected from H, halogen, CN, OH, C₁-C₃ alkyl, halogen-substituted C₁-C₃ alkyl, or cyano-substituted C₁-C₃ alkyl.

2. The compound, or the pharmaceutically acceptable salt thereof, or the stereoisomer thereof, or the tautomer thereof, or the hydrate thereof, or the solvate thereof, or the metabolite thereof, or the prodrug thereof according to claim 1, wherein
said R₁ and R₂ are each independently selected from cyclopropyl, cyclobutyl, cyclopentyl, cyclohexyl, pyrrolyl, piperazinyl, piperidinyl, phenyl, pyridyl, pyrimidinyl, pyrazolyl, thiazolyl, oxazolyl, imidazolyl, or indazolyl;
said R₃ is selected from hydrogen, chlorine, fluorine, amino, cyano, hydroxy, isopropyl, cyclopropyl, methyl, difluoromethyl, trifluoromethyl, methoxy, trifluoromethoxy, -OCH₂CH₃, -OCH₂CHF₂, or -OCH₂CF₃;
said R₄ and R₅ are each independently selected from hydrogen, chlorine, fluorine, methyl, or -CH₂N(CH₃)₂;
said R₆ is selected from hydrogen, chlorine, fluorine, bromine, amino, cyano, hydroxy, methyl, ethylmethyl, isopropyl, cyclopropyl, difluoromethyl, trifluoromethyl, methoxy, trifluoromethoxy, -OCH₂CH₃, -OCH₂CHF₂, -OCH₂CF₃, and -CH₂N(CH₃)₂;
said R₇ is selected from hydrogen, chlorine, fluorine, bromine, amino, carboxamido, cyano, hydroxy, methyl, ethylmethyl, isopropyl, cyclopropyl, difluoromethyl, trifluoromethyl, methoxy, trifluoromethoxy, -OCH₂CH₃, -OCH₂CHF₂, and -OCH₂CF₃; and
said R₈ is selected from hydrogen, methyl, -CH₂OH or -CH₂CN.

3. The compound, or the pharmaceutically acceptable salt thereof, or the stereoisomer thereof, or the tautomer thereof, or the solvate thereof, or the metabolite thereof, or the prodrug thereof according to claim 1 or 2, wherein heteroatoms or heteroatom radicals in said C₁-C₈ heteroalkyl, C₃-C₈ heterocycloalkyl, heteroaryl, monoheterocycloalkyl, C₆-C₁₂ bridged heterocycloalkyl, and C₆-C₁₂ spiroheterocycloalkyl are each independently selected from -O-, -S-, -CN, -NH-, =O, -O-N=, -C(=O)O-, -C(=O)-, -S(=O)-, -S(=O)₂-, -C(=O)NH-, -S(=O)₂NH-, or -NHC(=O)NH-, and the number of the heteroatoms or heteroatom radicals is independently selected from 1, 2 or 3.

4. The compound, or the pharmaceutically acceptable salt thereof, or the stereoisomer thereof, or the tautomer thereof, or the hydrate thereof, or the solvate thereof, or the metabolite thereof, or the prodrug thereof according to claim 1, wherein said 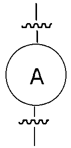 is selected from

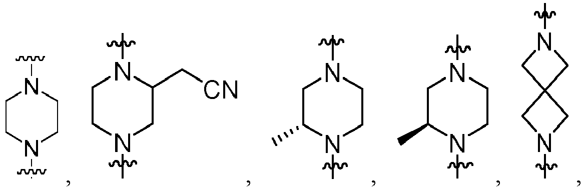

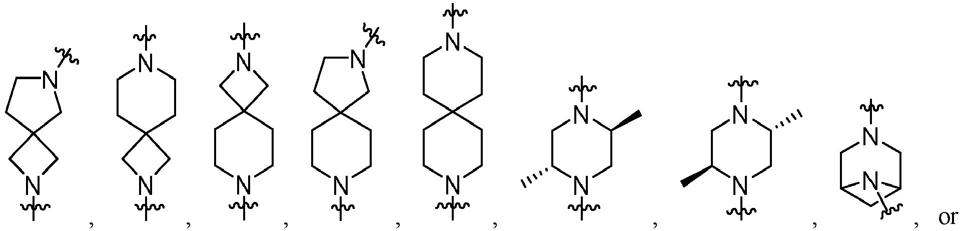

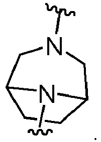

5. The compound, or the pharmaceutically acceptable salt thereof, or the stereoisomer thereof, or the tautomer thereof, or the hydrate thereof, or the solvate thereof, or the metabolite thereof, or the prodrug thereof according to claim 1, wherein the compound represented by formula I is selected from 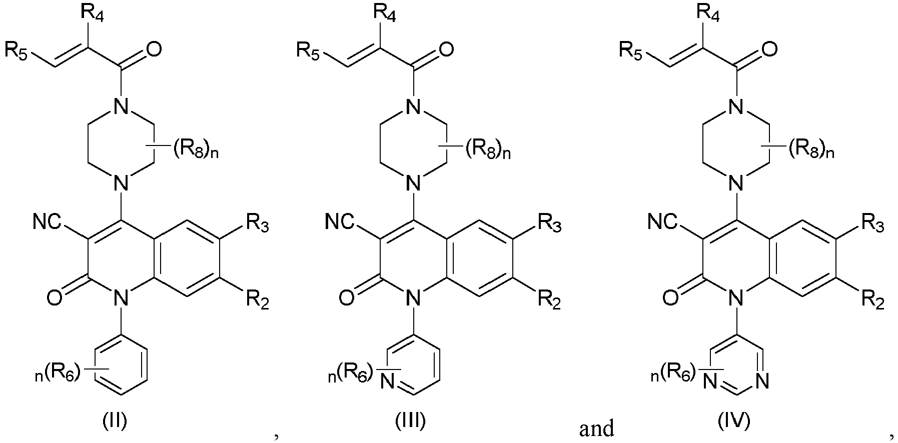 wherein, R₂, R₃, R₄, R₅, R₆, R₇ and R₈ are as defined in claim 1 or 2, and n is 0, 1, 2, 3 or 4.

6. The compound, or the pharmaceutically acceptable salt thereof, or the stereoisomer thereof, or the tautomer thereof, or the hydrate thereof, or the solvate thereof, or the metabolite thereof, or the prodrug thereof according to claim 5, wherein the compound represented by formula I is selected from 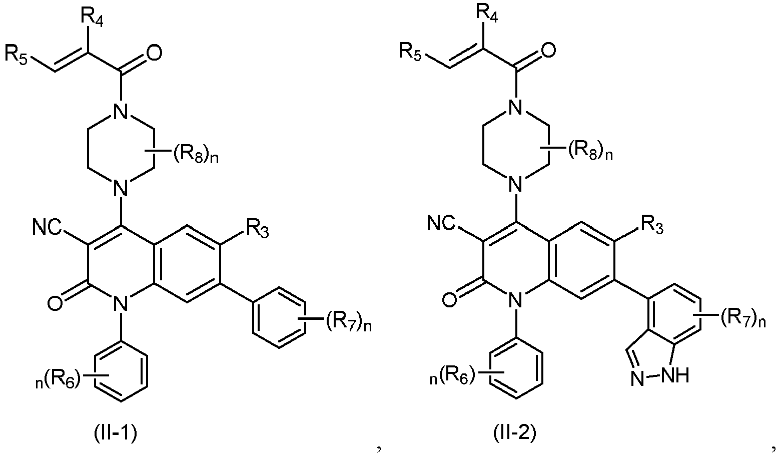 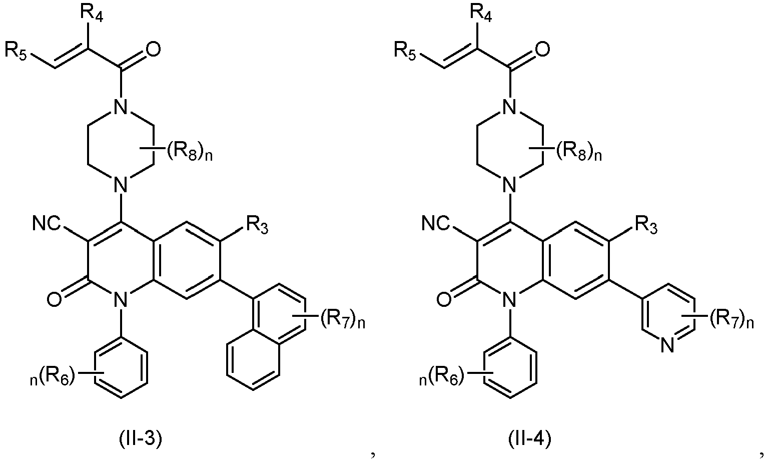 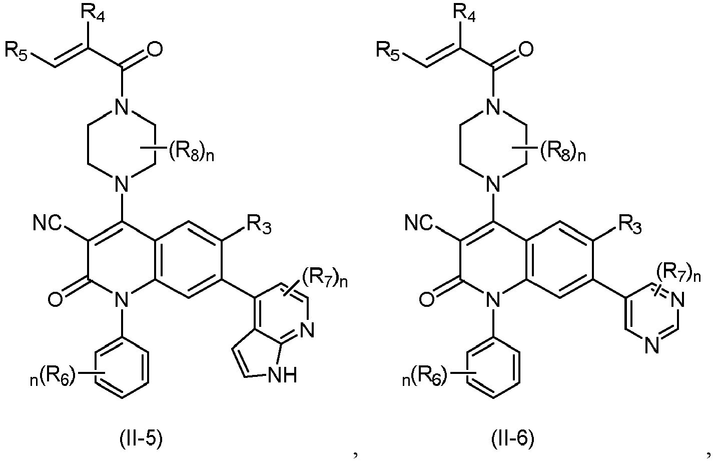 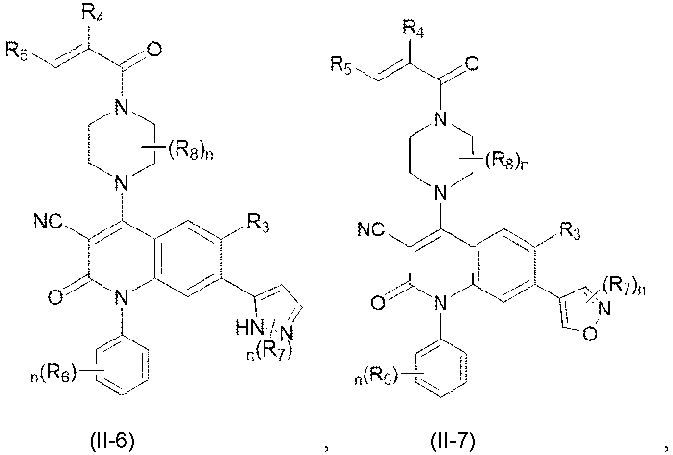 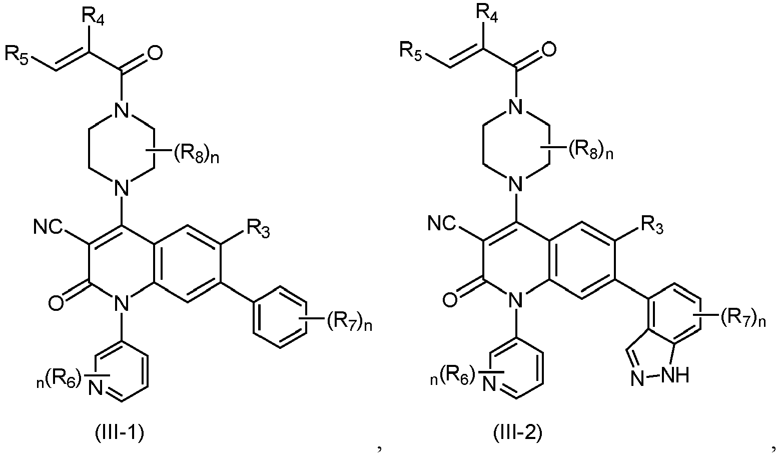 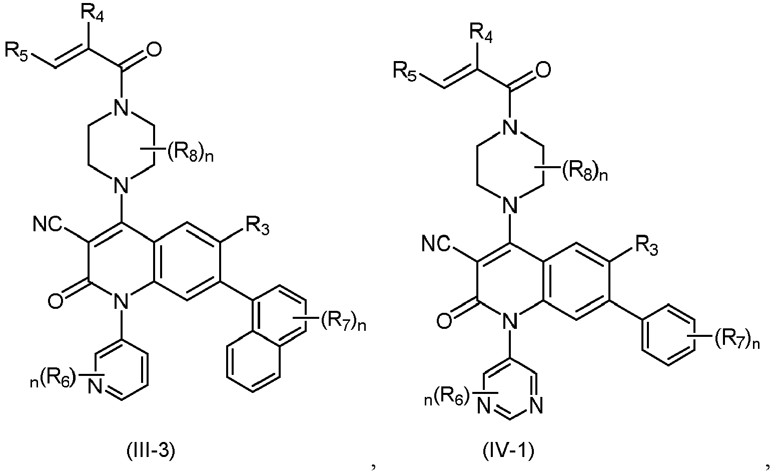 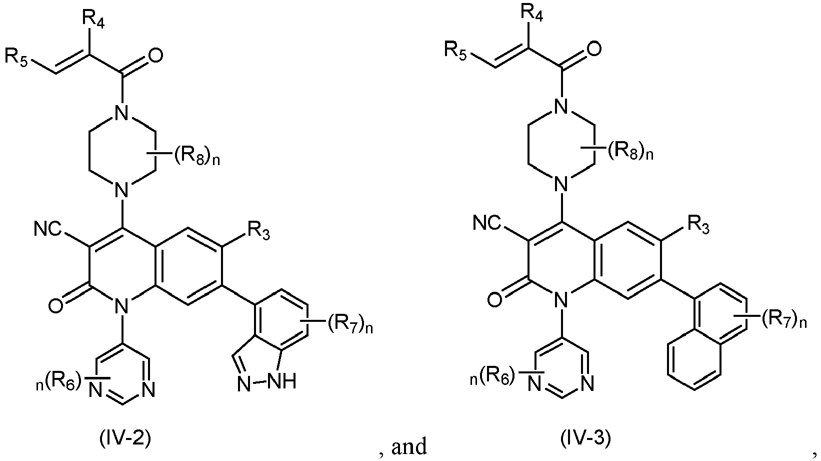 wherein,
R₃ is selected from H, F, Cl, OH, CF₃, CH₃, cyclopropyl, OCF₃, CHF₂ or OCH₃;
R₄ and R₅ are independently selected from H, F or CH₃;
R₆ is selected from H, F, Cl, Br, methyl, ethyl, isopropyl, methoxy, cyclopropyl or -CH₂N(CH₃)₂;
R₇ is selected from H, F, Cl, Br, NH2, OH, OCH₃, CN, CF₃, CONH₂, methyl, ethyl, isopropyl, cyclopropyl or CHF₂;
R₈ is selected from H or methyl; and
n is 0, 1, 2, 3 or 4.

7. A pharmaceutical composition comprising an effective dose of the compound, or the pharmaceutically acceptable salt thereof, or the stereoisomer thereof, or the tautomer thereof, or the hydrate thereof, or the solvate thereof, or the metabolite thereof, or the prodrug thereof according to any one of claims 1 to 6, and at least one pharmaceutically acceptable excipient.

8. Use of the compound, or the pharmaceutically acceptable salt thereof, or the stereoisomer thereof, or the tautomer thereof, or the hydrate thereof, or the solvate thereof, or the metabolite thereof, or the prodrug thereof according to any one of claims 1 to 6, or the pharmaceutical composition according to claim 7 in the preparation of a drug for treating diseases caused by KRAS G12C mutation.

9. Use of the compound, or the pharmaceutically acceptable salt thereof, or the stereoisomer thereof, or the tautomer thereof, or the hydrate thereof, or the solvate thereof, or the metabolite thereof, or the prodrug thereof according to any one of claims 1 to 6, or the pharmaceutical composition according to claim 7 in the preparation of a drug as a KRAS G12C inhibitor.

10. Use of the compound, or the pharmaceutically acceptable salt thereof, or the stereoisomer thereof, or the tautomer thereof, or the hydrate thereof, or the solvate thereof, or the metabolite thereof, or the prodrug thereof according to any one of claims 1 to 6, or the pharmaceutical composition according to claim 7 in the preparation of a drug for treating and/or preventing cancer.
